# EUROPÄISCHE PATENTSCHRIFT

(11) **EP 1 732 901 B1**
(45) Veröffentlichungstag und Bekanntmachung des Hinweises auf die Patenterteilung: **02.11.2011**
(21) Anmeldenummer: 05715944.4
(22) Anmeldetag: 11.03.2005
(51) Int. Cl.: C07D 233/90, C07D 401/12, C07D 403/12, A61K 31/4164, A61P 31/12

(54) **4-AMINOCARBONYLAMINO-SUBSTITUTIERTE IMIDAZOLVERBINDUNGEN MIT ANTIVIRALER WIRKUNG**
ANTIVIRAL 4-AMINOCARBONYLAMINO-SUBSTITUTED IMIDAZOLE COMPOUND
COMPOSES IMIDAZOLE A SUBSTITUTION 4-AMINOCARBONYLAMINO A ACTIVITE ANTIVIRALE

(30) Priorität: 26.03.2004 DE 102004015007
(43) Veröffentlichungstag der Anmeldung: 20.12.2006
(73) Patentinhaber: AiCuris GmbH & Co. KG, 42117 Wuppertal (DE)
(72) Erfinder: ZIMMERMANN, Holger, 42113 Wuppertal (DE); BRÜCKNER, David, 45128 Essen (DE); HEIMBACH, Dirk, 40629 Düsseldorf (DE); HENDRIX, Martin, 51519 Odenthal (DE); HENNINGER, Kerstin, 42115 Wuppertal (DE); HEWLETT, Guy, 42327 Wuppertal (DE); ROSENTRETER, Ulrich, 42349 Wuppertal (DE); KELDENICH, Jörg, 42113 Wuppertal (DE); LANG, Dieter, 42553 Velbert (DE); RADTKE, Martin, 40699 Erkrath (DE)
(74) Vertreter: Witte, Weller & Partner
(86) Internationale Anmeldenummer: PCT/EP2005/002571
(87) Internationale Veröffentlichungsnummer: WO 2005/092865

(56) Entgegenhaltungen:
- WO-A-00/34261
- WO-A-00/42043
- WO-A-99/23091

## Beschreibung

Die Erfindung betrifft substituierte Imidazole und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

WO 99/23091 beschreibt aromatische heterocyclische Verbindungen als antiinflammatorische Mittel, die unter anderem auch zur Behandlung von viralen Infektionen geeignet sein können.

Auf dem Markt sind zwar strukturell andersartige antiviral wirkende Mittel vorhanden, aber die gegenwärtig verfügbaren Therapien mit Ganciclovir, Valganciclovir, Foscarnet und Cidofovir sind mit schweren Nebenwirkungen verbunden, z. B. Nephrotoxizität, Neutropenie oder Thrombozytopenie. Zudem kann es regelmäßig zu einer Resistenzentwicklung kommen. Neue Mittel für eine wirksame Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Imidazole antiviral hochwirksam sind.

Gegenstand der vorliegenden Erfindung sind Verbindungen der Formel 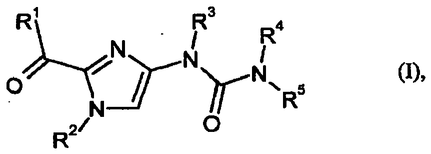 in welcher
- R¹: für -OR⁶ oder -NR⁷R⁸ steht,
- R²: für C₁-C₆-Alkyl oder C₁-C₆-Alkenyl steht,
wobei Alkyl und Alkenyl substituiert sein können mit 1 bis 3 Substituenten, wobei die Substituenten unabhängig voneinander ausgewählt werden aus der Gruppe, bestehend aus Halogen, C₁-C₆-Alkoxy, C₃-C₈-Cycloalkyl, 5- bis 10-gliedriges Heterocyclyl mit bis zu 3 Heteroatomen aus der Reihe S, O und/oder N, C₆-C₁₀-Aryl, Phenoxy und 5- bis 10-gliedriges Heteroaryl mit bis zu 5 Heteroatomen aus der Reihe S, O und/oder N,
worin Cycloalkyl, Heterocyclyl, Aryl, Phenoxy und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, C₁-C₆-Alkyl, C₁-C₆-Alkoxy, Hydroxycarbonyl, C₁-C₆-Alkoxycarbonyl Amino, C₁-C₆-Alkylamino, Aminocarbonyl, C₁-C₆₋Alkylaminocarbonyl und Phenyl,
R³ und R⁴ unabhängig voneinander für Wasserstoff oder C₁-C₆-Alkyl stehen,
- R⁵: für Phenyl steht, wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, C₁-C₆-Alkyl und C₁-C₆-Alkoxy,
- R⁶: für C₁-C₆-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, C₁-C₆-Alkoxy, Hydroxycarbonyl, C₁-C₆-Alkoxycarbonyl, Amino, C₁-C₆-Alkylamino, Aminocarbonyl, C₁-C₆-Alkylcarbonyloxy, C₁-C₆-Alkylcarbonylamino, C₁-C₆-Alkylamincarbonyl, C₁-C₆-Alkoxycarbonylamino, C₁-C₈-Cycloalkyl, C₃-C₈-Cycloalkylamino, 5- bis 10-gliedrige Heterocyclyl mit bis zu 3 Heteroatomen aus der Reihe S, O und/oder N, 5- bis 7-gliedriges Heterocyclyl-carbonyl mit bis zu 3 Heteroatomen aus der Reihe S, O und/oder N, C₆-C₁₀-Aryl und 5- bis 10-gliedriges Heteroaryl mit bis zu 5 Heteroatomen aus der Reihe S, O und/oder N,
worin Cycloalkyl, Cycloalkylamino, Heterocyclyl, Heterocyclylcarbonyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Nitro, Cyano, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₈-Cycloalkylamino und 5- bis 7-gliedriges Heterocyclyl mit bis zu 3 Heteroatomen aus der Reihe S, O und/oder N,
worin Heterocyclyl seinerseits substituiert sein kann mit 1 bis 3 Substituenten, wobei die Substituenten unabhängig voneinander ausgewählt werden aus der Gruppe, bestehend aus C₁-C₄-Alkyl und Oxo,
- R⁷: für Wasserstoff oder C₁-C₆-Alkyl steht,
und
- R⁸: für C₁-C₆-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, Trifluormethyl, C₁-C₆-Alkoxy, Hydroxycarbonyl, C₁-C₆-Alkoxycarbonyl, Amino, C₁-C₆-Alkylamino, Aminocarbonyl, C₁-C₆-Alkylcarbonyloxy, C₁-C₆-Alkylcarbonylamino, C₁-C₆-Alkylaminocarbonyl, C₁-C₆-Alkoxycarbonylamino, C₃-C₈-Cycloalkyl, C₃-C₈-Cycloalkylamino, 5- bis 10-gliedriges Heterocyclyl mit bis zu 3 Heteroatomen aus der Reihe S, O und/oder N, 5- bis 7-gliedriges Heterocyclylcarbonyl mit bis zu 3 Heteroatomen aus der Reihe S, O und/oder N, C₆-C₁₀-Aryl, C₆-C₁₀-Arylamino, 5- bis 10-gliedriges Heteroaryl mit bis zu 5 Heteroatomen aus der Reihe S, O und/oder N und 5- bis 10-gliedriges Heteroarylamino mit bis zu 5 Heteroatomen aus der Reihe S, O und/oder N,
worin Alkoxy und Alkylamino substituiert sein können mit 1 bis 3 Substituenten, wobei die Substituenten unabhängig voneinander ausgewählt werden aus der Gruppe, bestehend aus Halogen, Hydroxy und C₁-C₆-Alkoxy,
und
worin Cycloalkyl, Cycloalkylamino, Heterocyclyl, Heterocyclylcarbonyl, Aryl, Arylamino, Heteroaryl und Heteroarylamino substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, C₁-C₆-Alkyl, C₁-C₆-Alkoxy, Hydroxycarbonyl, C₁-C₆-Alkoxycarbonyl Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₈-Cycloalkylamino und 5- bis 7-gliedriges Heterocyclyl mit bis zu 3 Heteroatomen aus der Reihe S, O und/oder N,
worin Heterocyclyl seinerseits substituiert sein kann mit 1 bis 3 Substituenten, wobei die Substituenten unabhängig voneinander ausgewählt werden aus der Gruppe, bestehend aus C₁-C₄-Alkyl und Oxo,
und ihre Salze, ihre Solvate und die Solvate ihrer Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln (Ia), (Ib), (Ic) und (Id) sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkylcarbonyl, Alkylcarbonyloxy, Alkoxycarbonyl, Alkoxycarbonylamino, Alkylaminocarbonyl und Alkylcarbonylamino stehen für einen linearen oder verzweigten Alkylrest mit in der Regel 1 bis 6 ("C₁-C₆-Alkyl"), vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.
Alkoxy steht beispielhaft und vorzugsweise für Methoxy, Ethoxy, n-Propoxy, Isopropoxy, tert.-Butoxy, n-Pentoxy und n-Hexoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N,N-*Dimethylamino, *N,N-*Diethylamino, *N*-Ethyl-*N*-methylamino, N-Methyl-N-n-propylamino, *N*-Isopropyl-N-n-propylamino, N-t-Butyl-N-methylamino, N-Ethyl-N-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.
Alkylcarbonyl steht beispielhaft und vorzugsweise für Acetyl und Propanoyl.
Alkylcarbonyloxy steht beispielhaft und vorzugsweise für Methylcarbonyloxy, Ethylcarbonyloxy, n-Propylcarbonyloxy, Isopropylcarbonyloxy, tert.-Butylcarbonyloxy, n-Pentylcarbonyloxy und n-Hexylcarbonyloxy.
Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.
Alkoxycarbonylamino steht beispielhaft und vorzugsweise für Methoxycarbonylamino, Ethoxycarbonylamino, n-Propoxycarbonylamino, Isopropoxycarbonylamino, tert.-Butoxycarbonylamino, n-Pentoxycarbonylamino und n-Hexoxycarbonylamino.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, tert.-Butylaminocarbonyl, n-Pentylaminocarbonyl, n-Hexylaminocarbonyl, *N,N*-Dimethylaminocarbonyl, *N,N-*Diethylaminocarbonyl, N-Ethyl-N-methylaminocarbonyl, *N*-Methyl-*N*-n-propylaminocarbonyl, *N-*Isopropyl-N-n-propylaminocarbonyl, *N*-tert.-Butyl-*N*-methylaminocarbonyl, *N*-Ethyl-*N*-n-pentylaminocarbonyl und *N*-n-Hexyl-N-methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.
Alkylcarbonylamino steht beispielhaft und vorzugsweise für Methylcarbonylamino, Ethylcarbonylamino, n-Propylcarbonylamino, Isopropylcarbonylamino, tert.-Butylcarbonylamino, n-Pentylcarbonylamino und n-Hexylcarbonylamino.
Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.
Arylamino steht für einen mono- bis tricyclischen aromatischen, carbocyclischen Rest mit in der Regel 6 bis 14 Kohlenstoffatomen, der über eine Amino-Gruppe gebunden ist. Der zweite Substituent an der Aminogruppe steht für Wasserstoff oder C₁-C₆-Alkyl. Beispielsweise und vorzugsweise seien genannt: Phenylamino, Phenylmethylamino, Naphthylamino und Phenanthrenylamino.
5- bis 10-gliedriges Heteroaryl steht im Rahmen der Erfindung im Allgemeinen für einen aromatischen, mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff oder Heteroatom gebunden sein. Beispielsweise und vorzugsweise seien genannt: Thienyl, Furyl, Pyrrolyl, Thiazolyl, Oxazolyl, Pyrazolyl, Imidazolyl, Pyridyl, Pyrimidyl, Pyridazinyl, Indolyl, Indazolyl, Benzofuranyl, Benzothiophenyl, Chinolinyl und Isochinolinyl.
5- bis 10-gliedriges Heteroarylamino steht im Rahmen der Erfindung im Allgemeinen für einen aromatischen, mono- oder bicyclischen Rest mit 5 bis 10 Ringatomen und bis zu 5 Heteroatomen aus der Reihe S, O und/oder N, der über eine Amino-Gruppe gebunden ist. Bevorzugt sind 5- bis 6-gliedrige Heteroaryle mit bis zu 4 Heteroatomen. Der Heteroarylrest ist über ein Kohlenstoffatom an das Amin gebunden. Der zweite Substituent an der Aminogruppe steht für Wasserstoff oder C₁-C₆-Alkyl. Beispielsweise und vorzugsweise seien genannt: Thienylamino, Furylamino, Pyrrolylamino, Thiazolylamino, Oxazolylamino, Pyrazolylamino, Imidazolylamino, Pyridylamino, Pyrimidylamino, Pyridazinylamino, Indolylamino, Indazolylamino, Benzofuranyl-amino, Benzothiophenylamino, Chinolinylamino und Isochinolinylamino.
Cycloalkyl steht für eine Cycloalkylgruppe mit in der Regel 3 bis 8, bevorzugt 3 bis 6 Kohlenstoffatomen, beispielhaft und vorzugsweise für Cyclopropyl, Cyclobutyl, Cyclopentyl, Cyclohexyl und Cycloheptyl.
Cycloalkylamino steht für eine Cycloalkylaminogruppe mit in der Regel 3 bis 8, bevorzugt 3 bis 6 Kohlenstoffatomen, beispielhaft und vorzugsweise für Cyclopropylamino, Cyclobutylamino, Cyclopentylamino, Cyclohexylamino und Cycloheptylamino.
5- bis 10-gliedrige Heterocyclyl steht im Rahmen der Erfindung für einen mono- oder bicyclischen, gesättigten oder partiell ungesättigten Heterocyclus mit bis zu drei Heteroatomen aus der Reihe N, O und/oder S, der über ein Ringkohlenstoffatom oder ein Stickstoffatom des Heterocyclus verknüpft ist. Beispielhaft und vorzugsweise seien genannt: Tetrahydrofuryl, Dihydrofuryl, Imidazolidinyl, Thiolanyl, Dioxolanyl, Pyrrolidinyl, Pyrrolinyl, Tetrahydropyranyl, Dihydropyranyl, Piperidinyl, Piperazinyl, Morpholinyl, Thiomorpholinyl, 7-Oxabicyclo[2.2.1]heptanyl und 7-Oxabicyclo[2.2.1]hept-5-enyl.
5- bis 10-gliedriges Heterocyclylcarbonyl steht im Rahmen der Erfindung für einen mono- oder bicyclischen, gesättigten oder partiell ungesättigten Heterocyclus, der über eine Carbonylgruppe gebunden ist, mit bis zu drei Heteroatomen aus der Reihe N, O und/oder S, der über ein Ringkohlenstoffatom oder ein Stickstoffatom des Heterocyclus an die Carbonylgruppe verknüpft ist. Beispielhaft und vorzugsweise seien genannt: Tetrahydrofurylcarbonyl, Dihydrofurylcarbonyl, Imidazolidinylcarbonyl, Thiolanylcarbonyl, Dioxolanylcarbonyl, Pyrrolidinylcarbonyl, Pyrrolinylcarbonyl, Tetrahydropyranylcarbonyl, Dihydropyranylcarbonyl, Piperidinylcarbonyl, Piperazinylcarbonyl, Morpholinylcarbonyl, Thiomorpholinylcarbonyl, 7-Oxabicydo[2.2.1]heptanylcarbonyl und 7-Oxabicyclo[2.2.1]hept-5-enylcarbonyl.
Ein 4- bis 8-gliedrige Heterocyclus mit mindestens einem Ringstickstoffatom steht im Rahmen der Erfindung für einen gesättigten oder partiell ungesättigten, monocyclischen Heterocyclus, der bis zu zwei weitere Heteroatome aus der Reihe N, O und/oder S enthalten kann und über ein Ringstickstoffatom des Heterocyclus verknüpft ist. Bevorzugt ist ein 5- bis 7-gliedriger, gesättigter, monocyclischer N-Heterocyclus, der ein zweites Stickstoffatom oder ein Sauerstoffatom als weiteres Heteroatom enthalten kann. Beispielhaft und vorzugsweise seien genannt: Pyrrolidinyl, Pyrrolinyl, Oxazolidinyl, Thiazolidinyl, Piperidinyl, Piperazinyl, Morpholinyl, Thiomorpholinyl, Hexahydroazepinyl, Hexahydro-1,4-diazepinyl, Octahydroazocinyl.
Halogen steht für Fluor, Chlor, Brom und Jod.

Ein Symbol * an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung eine Enantiomerenüberschuss (enantiomeric excess) von mehr als 90 % verstanden wird (> 90 %ee).

Bevorzugt im Rahmen der vorliegenden Erfindung sind Verbindungen der Formel (I),
in welcher
- R¹: fiur -OR⁶ oder -NR⁷R⁸ steht,
- R²: für C₁-C₆-Alkyl oder C₁-C₆-Alkenyl steht,
wobei Alkyl und Alkenyl substituiert sein können mit 1 bis 3 Substituenten, wobei die Substituenten unabhängig voneinander ausgewählt werden aus der Gruppe, bestehend aus Halogen, C₁-C₆-Alkoxy, C₃-C₆-Cycloalkyl, 5- bis 10-gliedriges Heterocyclyl mit bis zu 3 Heteroatomen aus der Reihe S, O und/oder N, C₆-C₁₀-Aryl, Phenoxy und 5- bis 10-gliedriges Heteroaryl mit bis zu 5 Heteroatomen aus der Reihe S, O und/oder N,
worin Cycloalkyl, Heterocyclyl, Aryl, Phenoxy und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Trifluormethoxy, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl, C₁-C₆-Alkylaminocarbonyl und Phenyl,
R³ und R⁴ unabhängig voneinander für Wasserstoff oder C₁-C₆-Alkyl stehen,
- R⁵: für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Trifluormethoxy, C₁-C₆-Alkyl und C₁-C₆-Alkoxy,
- R⁶: für C₁-C₆-Akyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, C₁-C₆-Alkoxy, Hydroxycarbonyl, C₁-C₆-Alkoxycarbonyl, Amin, C₁-C₆-Alkylamino, Aminocarbonyl, C₁-C₆-Alkylcarbonyloxy, C₁-C₆-Alkylcarbonylamino, C₁-C₆-Alkylaminocarbonyl, C₁-C₆-Alkoxycarbonylamino, C₃-C₈-Cycloalkyl, C₃-C₈-Cycloalkylamino, 5- bis 10-gliedriges Heterocyclyl mit bis zu 3 Heteroatomen aus der Reihe S, O und/oder N, 5- bis 7-gliedriges Heterocyclyl-carbonyl mit bis zu 3 Heteroatomen aus der Reihe S, O und/oder N, C₆-C₁₀-Aryl und 5- bis 10-gliedriges Heteroaryl mit bis zu 5 Heteroatomen aus der Reihe S, O und/oder N,
worin Cycloalkyl, Cycloalkylamino, Heterocyclyl, Heterocyclylcarbonyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Nitro, Cyano, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₈-Cycloalkylamino und 5- bis 7-gliedriges Heterocyclyl mit bis zu 3 Heteroatomen aus der Reihe S, O und/oder N,
worin Heterocyclyl seinerseits substituiert sein kann mit 1 bis 3 Substituenten, wobei die Substituenten unabhängig voneinander ausgewählt werden aus der Gruppe, bestehend aus C₁-C₄-Alkyl und Oxo,
- R⁷: für Wasserstoff oder C₁-C₆-Alkyl steht,
und
- R⁸: für C₁-C₆-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, Trifluormethyl, C₁-C₆-Alkoxy, Hydroxycarbonyl, C₁-C₆-Alkoxycarbonyl, Amino, C₁-C₆-Alkylamino, Aminocarbonyl, C₁-C₆-Alkylcarbonyloxy, C₁-C₆-Alkylcarbonylamino, C₁-C₆-Alkylaminocarbonyl, C₁-C₆-Alkoxycarbonylamino, C₃-C₈-Cycloalkyl, C₃-C₈-Cycloalkylamino, 5- bis 10-gliedriges Heterocyclyl mit bis zu 3 Heteroatomen aus der Reihe S, O und/oder N, 5- bis 7-gliedriges Heterocyclylcarbonyl mit bis zu 3 Heteroatomen aus der Reihe S, O und/oder N, C₆-C₁₀-Aryl und 5- bis 10-gliedriges Heteroaryl mit bis zu 5 Heteroatomen aus der Reihe S, O und/oder N,
worin Alkoxy und Alkylamino substituiert sein können mit 1 bis 3 Substituenten, wobei die Substituenten unabhängig voneinander ausgewählt werden aus der Gruppe, bestehend aus Halogen, Hydroxy und C₁-C₆-Alkoxy,
und
worin Cycloalkyl, Cycloalkylamino, Heterocyclyl, Heterocyclylcarbonyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Trifluormethoxy, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₈-Cycloalkylamino und 5- bis 7-gliedriges Heterocyclyl mit bis zu 3 Heteroatomen aus der Reihe S, O und/oder N,
worin Heterocyclyl seinerseits substituiert sein kann mit 1 bis 3 Substituenten, wobei die Substituenten unabhängig voneinander ausgewählt werden aus der Gruppe, bestehend aus C₁-C₄-Alkyl und Oxo,
und ihre Salze, ihre Solvate und die Solvate ihrer Salze.

Bevorzugt im Rahmen der vorliegenden Erfindung sind auch Verbindungen der Formel (I),
in welcher
- R¹: für -OR⁶ oder -NR⁷R⁸ steht,
- R²: für C₁-C₄-Alkyl oder C₁-C₅-Alkenyl steht,
wobei Alkyl und Alkenyl substituiert sein können mit 1 bis 2 Substituenten, wobei die Substituenten unabhängig voneinander ausgewählt werden aus der Gruppe, bestehend aus Halogen, C₁-C₄-Alkoxy, C₃-C₆-Cycloalkyl, Phenyl und Phenoxy,
worin Cycloalkyl, Phenyl und Phenoxy substituiert sein können mit 1 bis 3 Substituenten, wobei die Substituenten unabhängig voneinander ausgewählt werden aus der Gruppe, bestehend aus Halogen, Hydroxy, Cyano, Trifluormethyl, Trifluormethoxy, C₁-C₄-Alkyl, C₁-C₄-Alkoxy, Hydroxycarbonyl, C₁-C₄-Alkoxycarboxyl, Amino, C₁-C₆-Alkylamino, Aminocarbonyl, C₁-C₆-Alkylaminocarbonyl und Phenyl,
R³ und R⁴ für Wasserstoff stehen,
- R⁵: für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Trifluormethoxy, C₁-C₄-Alkyl und C₁-C₄-Alkoxy,
- R⁶: für C₁-C₅-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, C₁-C₄-Alkoxy, Hydroxycarbonyl, Amino, C₁-C₆-Alkylamino, Aminocarbonyl, C₁-C₄-Alkylcarbonyloxy, C₁-C₄-Alkylcarbonylamino, C₁-C₆-Alkylaminocarbonyl, C₁-C₄-Alkoxycarbonylamino, C₃-C₇-Cycloalkyl, C₃-C₇-Cycloalkylamino, 5- bis 7-gliedriges Heterocyclyl mit bis zu 3 Heteroatomen aus der Reihe S, O und/oder N, 5- bis 7-gliedriges Heterocyclylcarbonyl mit bis zu 3 Heteroatomen aus der Reihe S, O und/oder N, C₆-C₁₀-Aryl und 5- bis 10-gliedriges Heteroaryl mit bis zu 5 Heteroatomen aus der Reihe S, O und/oder N,
worin Cycloalkyl, Cycloalkylamino, Heterocyclyl, Heterocyclylcarbonyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Cyano, C₁-C₄-Alkyl, C₁-C₄-Alkoxy, Hydroxycarbonyl, C₁-C₄-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe,
bestehend aus Hydroxycarbonyl, Amino, C₁-C₆-Alkylamino, C₃-C₇-Cycloalkylamino und 5- bis 7-gliedriges Heterocyclyl mit bis zu 3 Heteroatomen aus der Reihe S, O und/oder N,
- R⁷: für Wasserstoff steht,
und
- R⁸: für C₁-C₅-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, Trifluormethyl, C₁-C₆-Alkoxy, Hydroxycarbonyl, Amino, C₁-C₆-Alkylamino, Aminocarbonyl, C₁-C₄-Alkylcarbonyloxy, C₁-C₄-Alkylcarbonylamino, C₁-C₆-Alkylaminocarbonyl, C₁-C₄-Alkoxycarbonylamino, C₃-C₇-Cycloalkyl, C₃-C₇-Cycloalkylamino, 5- bis 7-gliedriges Heterocyclyl mit bis zu 3 Heteroatomen aus der Reihe S, O und/oder N, 5- bis 7-gliedriges Heterocyclyl-carbonyl mit bis zu 3 Heteroatomen aus der Reihe S, O und/oder N, C₆-C₁₀-Aryl und 5- bis 10-gliedriges Heteroaryl mit bis zu 5 Heteroatomen aus der Reihe S, O und/oder N,
worin Alkoxy und Alkylamino substituiert sein können mit 1 bis 3 Substituenten, wobei die Substituenten unabhängig voneinander ausgewählt werden aus der Gruppe, bestehend aus Halogen, Hydroxy und C₁-C₄-Alkoxy,
und
worin Cycloalkyl, Cycloalkylamino, Heterocyclyl, Heterocyclylcarbonyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Cyano, Trifluormethyl, C₁-C₄-Alkyl, C₁-C₄-Alkoxy, Hydroxycarbonyl, C₁-C₄-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₇-Cycloalkylamino und 5- bis 7-gliedriges Heterocyclyl mit bis zu 3 Heteroatomen aus der Reihe S, O und/oder N,
worin Heterocyclyl seinerseits substituiert sein kann mit 1 bis 3 Substituenten, wobei die Substituenten unabhängig voneinander ausgewählt werden aus der Gruppe, bestehend aus C₁-C₄-Alkyl und Oxo, und ihre Salze, ihre Solvate und die Solvate ihrer Salze.

Bevorzugt im Rahmen der vorliegenden Erfindung sind auch Verbindungen der Formel (I),
in welcher
- R¹: für -OR⁶ oder -NR⁷R⁸ steht,
- R²: für Methyl, Ethyl, n-Butyl, Prop-2-en-1-yl oder 3-Methyl-but-2-en-1-yl steht,
wobei Methyl, Ethyl, n-Butyl und Propyl-2-en-1-yl substituiert sein können mit 1 bis 2 Substituenten, wobei die Substituenten unabhängig voneinander ausgewählt werden aus der Gruppe, bestehend aus Chlor, Methoxy, Cyclopropyl, Phenyl und Phenoxy,
worin Phenyl substituiert sein kann mit einem Substituenten Trifluormethyl,
R³ und R⁴ für Wasserstoff stehen,
- R⁵: für Phenyl steht,
wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy und Methyl,
- R⁶: für C₁-C₃-Alkyl steht,
wobei Alkyl substituiert sein kann mit 1 bis 2 Substituenten, wobei die Substituenten unabhängig voneinander ausgewählt werden aus der Gruppe, bestehend aus Halogen, Cyano, Hydroxy und Methylcarbonyloxy,
worin Methylcarbonyloxy substituiert sein kann mit 1 bis 3 Substituenten, wobei die Substituenten unabhängig voneinander ausgewählt werden aus der Gruppe, bestehend aus Isobutylamino, Dimethylamino, Diethylamino, Cyclopropylamino, Pyrrolidinyl und Morpholinyl,
- R⁷: für Wasserstoff steht,
und
- R⁸: für C₁-C₃-Alkyl steht,
wobei Alkyl substituiert sein kann mit 1 bis 2 Substituenten, wobei die Substituenten unabhängig voneinander ausgewählt werden aus der Gruppe, bestehend aus Halogen, Cyano, Hydroxy, Trifluormethyl, Ethoxy, Isobutylamino, Dimethylamino, Diethylamino, Methylethylamino, Aminocarbonyl, Methylcarbonyloxy, Propylcarbonyloxy, Dimethylaminocarbonyl, Diethylaminocarbonyl, Ethoxycarbonylamino, Cyclopropylamino, Pyrrolidinyl, Piperidinyl, Morpholinyl, Phenyl, Thienyl, Pyrazolyl, Imidazolyl, Triazolyl, Pyridyl und Benzimidazolyl,
worin Ethoxy und Methylethylamino substituiert sein können mit einem Substituenten, wobei der Substituent ausgewählt wird aus der Gruppe, bestehend aus Hydroxy und Methoxy,
und
worin Phenyl, Pyrazolyl, Imidazolyl, Pyridyl und Benzimidazolyl substituiert sein können mit 1 bis 3 Substituenten, wobei die Substituenten unabhängig voneinander ausgewählt werden aus der Gruppe, bestehend aus Methyl und Methoxy,
und
worin Methylcarbonyloxy und Propylcarbonyloxy substituiert sein können mit 1 bis 3 Substituenten, wobei die Substituenten unabhängig voneinander ausgewählt werden aus der Gruppe, bestehend aus Hydroxycarbonyl, Amino, Isobutylamino, Dimethylamino, Diethylamino, Cyclopropylamino, Pyrrolidinyl und Morpholinyl,
und ihre Salze, ihre Solvate und die Solvate ihrer Salze.

Bevorzugt im Rahmen der vorliegenden Erfindung sind auch Verbindungen der Formel (I), in welcher Für -NR⁷R⁸ steht.

Bevorzugt im Rahmen der vorliegenden Erfindung sind auch Verbindungen der Formel (I), in welcher R¹ für -OR⁶ steht.

Bevorzugt im Rahmen der vorliegenden Erfindung sind auch Verbindungen der Formel (I), in welcher R³ und R⁴ für Wasserstoff stehen.

Bevorzugt im Rahmen der vorliegenden Erfindung sind auch Verbindungen der Formel (I), in welcher R⁵ für Phenyl steht, wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy und Methyl.

Bevorzugt im Rahmen der vorliegenden Erfindung sind auch Verbindungen der Formel (I), in welcher R⁷ für Wasserstoff steht.

Gegenstand der Erfindung ist weiterhin ein Verfahren zur Herstellung der Verbindungen der Formel (I), wobei
nach Verfahren [A]
Verbindungen der Formel 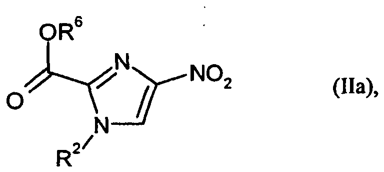 in welcher
R⁶ die oben angegebene Bedeutung hat, und
R² die oben angegebene Bedeutung hat,
in der ersten Stufe mit einem Reduktionsmittel,
in der zweiten Stufe gegebenenfalls mit Verbindungen der Formel 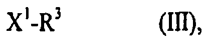 in welcher
R³ die oben angegebene Bedeutung hat, und
X¹ für Halogen, bevorzugt Brom oder Chlor, steht
und in der dritten Stufe in Gegenwart eines Kohlensäurederivates mit Verbindungen der Formel 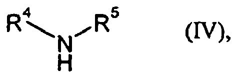 in welcher
R⁴ und R⁵ die oben angegebene Bedeutung haben,
zu Verbindungen der Formel 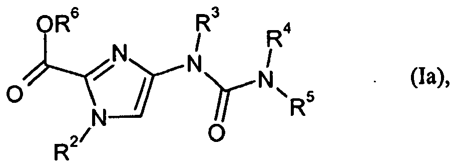 in welcher
R⁶ die gleiche Bedeutung wie in Formel (IIa) hat, und
R², R³, R⁴ und R⁵ die oben angegebene Bedeutung haben,
umgesetzt werden,
oder
nach Verfahren [B]
Verbindungen der Formel (Ia),
in welcher
R⁸ für Methyl oder Ethyl steht,
in Gegenwart von Basen zu Verbindungen der Formel 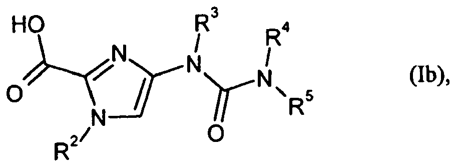 in welcher
R², R³, R⁴ und R⁵ die oben angegebene Bedeutung haben,
umgesetzt werden,
oder
nach Verfahren [C]
Verbindungen der Formel (Ib) mit Verbindungen der Formel 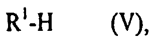 in welcher
R¹ die oben angegebene Bedeutung hat,
in Gegenwart von Dehydratisierungsreagenzien zu Verbindungen der Formel (I) umgesetzt werden,
oder
nach Verfahren [D]
Verbindungen der Formel 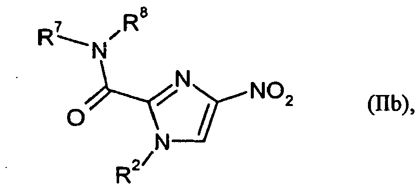 in welcher
R², R⁷ und R⁸ die oben angegebene Bedeutung haben,
in der ersten Stufe mit einem Reduktionsmittel,
in der zweiten Stufe gegebenenfalls mit Verbindungen der Formel (III)
und in der dritten Stufe in Gegenwart eines Kohlensäurederivates mit Verbindungen der Formel (IV)
zu Verbindungen der Formel 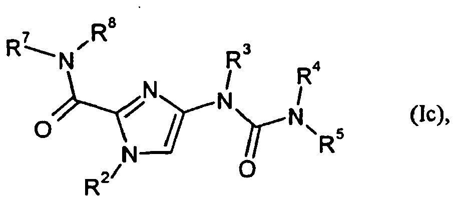 in welcher
R², R³, R⁴, R⁵, R⁷ und R⁸ die oben angegebene Bedeutung haben,
umgesetzt werden,
oder
nach Verfahren [E]
Verbindungen der Formel (IIa) oder (IIb)
in der ersten Stufe mit einem Reduktionsmittel,
in der zweiten Stufe gegebenenfalls mit Verbindungen der Formel (III)
und in der dritten Stufe mit Verbindungen der Formel 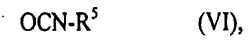 in welcher
R⁵ die oben angegebene Bedeutung hat,
zu Verbindungen der Formel 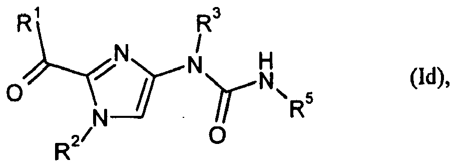 in welcher
R¹, R², R³ und R⁵ die oben angegebene Bedeutung haben,
umgesetzt werden.

Formel (I) umfasst die Verbindungen der Formeln (Ia), (Ib), (Ic) und (Id).

Formel (II) umfasst die Verbindungen der Formeln (IIa) und (IIb).

Die Verbindungen der Formel (III), (IV), (V) und (VIII) sind bekannt oder lassen sich nach bekannten Verfahren aus den entsprechenden Edukten synthetisieren.

Für Verfahren [A], [D] und [E] gilt:

### 1. Stufe:

Die Umsetzung erfolgt im Allgemeinen in inerten Lösungsmitteln, bevorzugt in einem Temperaturbereich von 0°C bis zum Rückfluss der Lösungsmittel bei Normaldruck bis 3 bar.

Reduktionsmittel sind beispielsweise Palladium auf Aktivkohle und Wasserstoff, Ameisensäure/Triethylamin/Palladium auf Aktivkohle, Zink, Zink/Salzsäure, Eisen, Eisen/Salzsäure, Eisen(II)sulfat/Salzsäure, Natriumsulfid, Natriumdisulfid Natriumdithionit, Ammoniumpolysulfid, Natriumborhydrid/Nickelchlorid, Zinndichlorid, Titantrichlorid oder Raney-Nickel und wässrige Hydrazin-Lösung, bevorzugt ist Raney-Nickel und wässrige Hydrazin-Lösung.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, im Falle von wassermischbaren Lösungsmitteln auch Gemische derselben mit Wasser, als Lösungsmittel ist bevorzugt Methanol, Ethanol, iso-Propanol oder im Falle von Raney-Nickel und wässrige Hydrazin-Lösung Tetrahydrofuran.

### 2. Stufe:

Die 2. Stufe wird bei der Herstellung von erfindungsgemäßen Verbindungen, bei denen R³ für C₁-C₆-Alkyl steht, durchgeführt und entfällt bei der Herstellung von erfindungsgemäßen Verbindungen, bei denen R³ für Wasserstoff steht.

Die Umsetzung erfolgt im Allgemeinen in inerten Lösungsmitteln, gegebenenfalls in Gegenwart einer Base, bevorzugt in einem Temperaturbereich von -20°C bis 40°C bei Normaldruck.

Basen sind beispielsweise Amide wie Natriumamid, Lithiumhexamethyldisilazid, Kaliumhexamethyldisilazid, Lithiumdiisopropylamid, oder andere Basen wie Natriumhydrid, DBU oder Diisopropylethylamin, bevorzugt Natriumamid, Lithiumhexamethyldisilazid, Kaliumhexamethyldisilazid oder Lithiumdiisopropylamid.

Inerte Lösungsmittel sind beispielsweise Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Ethylbenzol, Xylol, Toluol, bevorzugt Tetrahydrofuran oder Toluol.

### 3. Stufe Verfahren [A] und [D]:

Die Umsetzung erfolgt im Allgemeinen in inerten Lösungsmitteln, bevorzugt in einem Temperaturbereich von Raumtemperatur bis 40°C bei Normaldruck.

Kohlensäurederivate sind beispielsweise N,N-Carbonyldiimidazol, Phosgen, Diphosgen, Triphosgen, Chlorameisensäurephenylester oder Chlorameisensäure-4-nitrophenylester, bevorzugt ist N,N-Carbonyldiimidazol.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, im Falle von wassermischbaren Lösungsmitteln auch Gemische derselben mit Wasser, bevorzugt ist Dimethylsulfoxid.

### 3. Stufe Verfahren [E]:

Die Umsetzung erfolgt im Allgemeinen in inerten Lösungsmitteln, gegebenenfalls in Gegenwart einer Base, bevorzugt in einem Temperaturbereich von Raumtemperatur bis zum Rückfluß der Lösungsmittel bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, bevorzugt sind Tetrahydrofuran oder Methylenchlorid.

Basen sind beispielsweise Alkalicarbonate wie Cäsiumcarbonat, Natrium- oder Kaliumcarbonat, oder Kalium-tert.-butylat, oder andere Basen wie Natriumhydrid, DBU, Triethylamin oder Diisopropylethylamin, bevorzugt Triethylamin.

Für Verfahren [B] gilt:

Die Umsetzung erfolgt im Allgemeinen in inerten Lösungsmitteln, bevorzugt in einem Temperaturbereich von 0°C bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt Natriumhydroxid.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Gemische von Lösungsmitteln mit Wasser, als Lösungsmittel ist bevorzugt ein Gemisch aus Ethanol und Wasser.

Für Verfahren [C] gilt:

Die Umsetzung erfolgt im Allgemeinen in inerten Lösungsmitteln, gegebenenfalls in Gegenwart einer Base, bevorzugt in einem Temperaturbereich von -70°C bis 40°C bei Normaldruck.

Als Dehydratisierungsreagenzien eignen sich hierbei beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder O-(Benzotriazol-1-yl)-N,N,N',N'-tetra-methyluroniumhexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP), oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin, oder DBU, DBN, Pyridin, bevorzugt ist Triethylamin.

Vorzugsweise wird die Kondensation mit Carbonyldiimidazol durchgeführt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, im Falle von wassermischbaren Lösungsmitteln auch Gemische derselben mit Wasser, bevorzugt ist Dimethylformamid.

Die Verbindungen der Formel (II) sind bekannt oder können hergestellt werden, indem Verbindungen der Formel 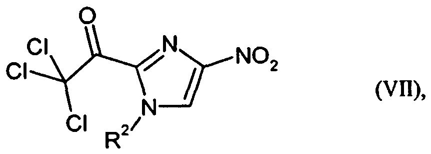 in welcher
R² die oben angegebene Bedeutung hat,
mit Verbindungen der Formel (V) umgesetzt werden.

Die Umsetzung erfolgt im Allgemeinen in inerten Lösungsmitteln, gegebenenfalls in Gegenwart einer Base, bevorzugt in einem Temperaturbereich von Raumtemperatur bis 40°C bei Normaldruck.

Basen sind beispielsweise Alkalicarbonate wie Cäsiumcarbonat, Natrium- oder Kaliumcarbonat, oder Kalium-tert.-butylat, oder andere Basen wie Natriumhydrid, DBU, Triethylamin oder Diisopropylethylamin, bevorzugt sind Diisopropylethylamin und Triethylamin.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylacetamid, 2-Butanon, Dimethylsulfoxid, Acetonitril oder Pyridin, bevorzugt ist Ethanol und Tetrahydrofuran.

Die Verbindungen der Formel (VII) sind bekannt oder können hergestellt werden, indem Verbindungen der Formel 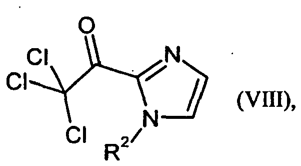 in welcher
R² die oben angegebene Bedeutung hat,
mit rauchender Salpetersäure, konzentrierter Salpetersäure, Nitriersäure oder anderen Mischungsverhältnissen von Schwefelsäure und Salpetersäure, gegebenenfalls in Acetanhydrid als Lösungsmittel, bevorzugt in einem Temperaturbereich von -60°C bis 0°C bei Normaldruck, umgesetzt werden.

Die Verbindungen der Formel (VIII) sind bekannt oder lassen sich nach bekannten Verfahren aus den entsprechenden Edukten synthetisieren.

In einem alternativen Verfahren können die Verbindungen der Formel (IIa), in welcher R⁶ für Methyl oder Ethyl steht, und R² die oben angegebene Bedeutung hat,
hergestellt werden, indem Verbindungen der Formel 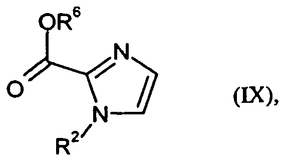 in welcher
R⁶ für Methyl oder Ethyl steht, und
R² die oben angegebene Bedeutung hat,
mit rauchender Salpetersäure, konzentrierter Salpetersäure, Nitriersäure oder anderen Mischungsverhältnissen von Schwefelsäure und Salpetersäure, gegebenenfalls in Acetanhydrid als Lösungsmittel, bevorzugt in einem Temperaturbereich von Raumtemperatur bis 60°C bei Normaldruck, umgesetzt werden.

Die Verbindungen der Formel (IX) sind bekannt oder lassen sich nach bekannten Verfahren aus den entsprechenden Edukten synthetisieren.

Die Beinführung des Substituenten R² durch dem Fachmann bekannte Alkylierungsmethoden kann je nach Substitutionsmuster des Imidazols an verschiedenen Stellen der Syntheseroute erfolgen.

### Syntheseschema:

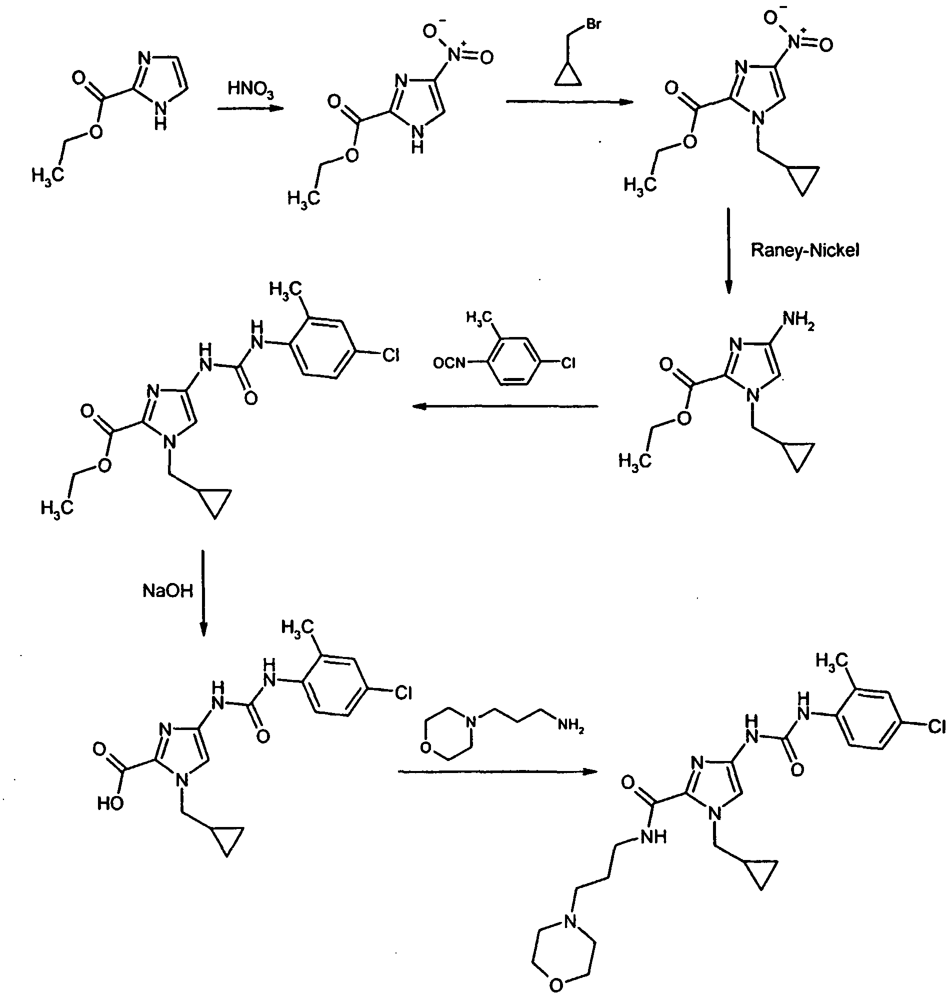

Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV). Sie sind somit geeignet zur Behandlung und Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Die Verbindungen der allgemeinen Formel (I) können aufgrund ihrer besonderen Eigenschaften zur Herstellung von Arzneimitteln, die zur Prophylaxe und/oder Behandlung von Krankheiten, insbesondere Virusinfektionen, geeignet sind, verwendet werden.

Als Indikationsgebiete können beispielsweise genannt werden:
- 1): Behandlung und Prophylaxe von HCMV-Infektionen bei AIDS-Patienten (Retinitis, Pneumonitis, gastrointestinale Infektionen).
- 2): Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
- 3): Behandlung und Prophylaxe von HCMV-Infektionen bei Neugeborenen und Kleinkindern.
- 4): Behandlung einer akuten HCMV-Infektion bei Schwangeren.
- 5): Behandlung der HCMV-Infektion bei immunsupprimierten Patienten bei Krebs und Krebs-Therapie.
- 6): Behandlung von HCMV-positiven Krebspatienten mit dem Ziel, HCMV-vermittelte Tumorprogression zu verringern (vgl. J. Cinatl , et al., FEMS Microbiology Reviews 2004, 28, 59-77).

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Die erfindungsgemäßen Verbindungen können aufgrund ihrer pharmakologischen Eigenschaften allein und bei Bedarf auch in Kombination mit anderen Wirkstoffen, insbesondere antiviralen Wirkstoffen wie beispielsweise Gancyclovir oder Acyclovir, zur Behandlung und/oder Prävention von Virusinfektionen, insbesondere von HCMV-Infektionen, eingesetzt werden.

Weiterer Gegenstand der vorliegenden Erfindung ist der Einsatz der erfindungsgemäßen Verbindungen zur Behandlung und/oder Prophylaxe von Erkrankungen, vorzugsweise von Virusinfektionen, insbesondere von Infektionen mit dem humanen Cytomegalovirus (HCMV) oder einem anderen Vertreter der Gruppe der Herpes viridae.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung einer antiviral wirksamen Menge der erfindungsgemäßen Verbindungen in einem Verfahren zur Behandlung und/oder Prophylaxe von Erkrankungen, insbesondere der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- Bsp.: Beispiel
- CD₃CN: Deuteroacetonitril
- DC: Dünnschichtchromatographie
- DCI: direkte chemische Ionisation (bei MS)
- DCM: Dichlormethan
- DIEA: *N,N*-Diisopropylethylamin (Hünig Base)
- DMAP: 4-*N,N-*Dimethylaminopyridin
- DMF: *N,N-*Dimethylformamid
- DMSO: Dimethylsulfoxid
- d. Th.: der Theorie
- EDCI x HCl: N'-(3-Dimethylaminopropyl)-N-ethylcarbodiimid Hydrochlorid
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- H: Stunde
- HATU: *O*-(7-Azabenzotriazol-1-yl)-*N,N,N',N*'-tetramethyluronium-hexafluorophosphat
- HBTU: *O*-(Benzotriazol-1-yl)-*N,N,N',N*'-tetramethyluronium-hexafluorophosphat
- HPLC: Hochdruck-, Hochleistungsflüssigchromatographie
- i. V.: im Vakuum
- konz.: konzentriert
- LC-MS: Flüssigchromatographie-gekoppelte Massenspektroskopie
- LDA: Lithiumdiisopropylamid
- Lit.: Literatur(stelle)
- Lsg.: Lösung
- MS: Massenspektroskopie
- NMR: Kernresonanzspektroskopie
- proz.: prozentig
- RP-HPLC: Reverse Phase HPLC
- RT: Raumtemperatur
- Rₜ: Retentionszeit (bei HPLC)
- Schmp.: Schmelzpunkt
- TBTU: O-(Benzotriazol-1-yl)-N,N,N',N'-tetramethyluronium-tetrafluoroborat
- THF: Tetrahydrofuran
- verd.: verdünnt
- wässr.: wässrig

### HPLC- und LC-MS-Methoden:

**Methode 1 (LC-MS):** Instrument: Micromass Platform LCZ mit HPLC Agilent Serie 1100; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 210 nm.
**Methode 2 (LC-MS):** Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.
**Methode 3 (LC-MS):** Instrument: Micromass Platform LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.
**Methode 4 (LC-MS):** Gerätetyp MS: Micromass ZQ; Gerätetyp HPLC: Waters Alliance 2795; Säule: Phenomenex Synergi 2µ Hydro-RP Mercury 20 mm x 4mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.
**Methode 5 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: **0.0** min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.
**Methode 6 (LC-MS):** Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50%ige Ameisensäure /1, Eluent B: Acetonitril + 500 µl 50%ige Ameisensäure 1; Gradient: 0.0 min 0%B → 2.9 min 70%B → 3.1 min 90%B → 4.5 min 90%B; Ofen: 50 °C; Fluss: 0.8 ml/min; UV-Detektion: 210 nm.
**Methode 7 (LC-MS):** Gerätetyp MS: Micromass ZQ; Gerätetyp HPLC: Waters Alliance 2795; Säule: Merck Chromolith SpeedROD RP-18e 50 mm x 4.6mm; Eluent A: Wasser + 500 µl 50%ige Ameisensäure / 1; Eluent B: Acetonitril + 500 µl 50%ige Ameisensäure / 1; Gradient: 0.0 min 10%B→ 3.0 min 95%B→ 4.0 min 95%B; Ofen: 35°C; Fluss: 0.0 min 1.0 ml/min→ 3.0 min 3.0 ml/min→ 4.0 min 3.0 ml/min; UV-Detektion: 210 nm.
**Methode 8 (LC-MS):** Instrument: Micromass Quattro LCZ, mit HPLC Agilent Serie 1100; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.
**Methode 9 (präparative HPLC):** Säule: Machery Nagel VP50/21 Nucleosil 100-5 C18 Nautilus, 5 µm, 21 mm x 50 mm; Eluent A: Wasser + 0.1 % Ameisensäure, Eluent B: Acetonitril, Gradient: 0 min 10%B, 2 min 10%B, 6 min 90%B, 7 min 90%B, 7.1 min 10%B, 8 min 10%B; Fluss: 25 ml/min, UV-Detektion: 220 nm.
**Methode 10 (präparative HPLC):** Säule: Machery Nagel VP50/21 Nucleodur C18 Gravity, 5 µm, 21 mm x 50 mm; Eluent A: Wasser + 0.1 % Ammoniak , Eluent B: Acetonitril, Gradient: 0 min 10%B, 2 min 10%B, 6 min 90%B, 7 min 90%B, 7.1 min 10%B, 8 min 10%B; Fluss: 25 ml/min, UV-Detektion: 220 nm.
**Methode 11 (analytische HPLC):** Säule: Kromasil 100 RP-18, 60 mm x 2.1 mm, 3.5 µm; Eluent A: Wasser + 0.5% Perchlorsäure (70 %ig), Eluent B: Acetonitril; Gradient: 0 min 2%B, 0.5 min 2%B, 4.5 min 90%B, 9 min 90%B, 9.2 min 2%B, 10 min 2%B; Fluss: 0.75 ml/min; Säulentemperatur: 30°C; Detektion: UV 210 nm.

### Aussanssverbindunsen

### Beispiel 1A

1-Benzyl-1H-imidazol-2-carbonsäureethylester 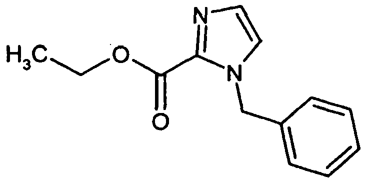 148 g (936 mmol) 1-Benzyl-1H-imidazol werden in 480 ml Acetonitril suspendiert und bei -20°C mit 120 ml (87.1 g; 860 mmol) Triethylamin versetzt. Innerhalb von 15 Minuten werden dann 211.2 ml (239 g; 2208 mmol) Chlorameisensäureethylester zugetropft. Die Reaktionsmischung wird 10 Minuten bei -20°C gerührt. Nach Erwärmen auf 15 bis 20°C wird die Reaktionsmischung 18 h gerührt und dann im Vakuum eingeengt. Der Rückstand wird mit Wasser, gesättigter Natriumchloridlösung und gesättigter Natriumhydrogencarbonatlösung versetzt und dreimal mit Ethylacetat extrahiert. Die vereinigten organischen Phasen werden mit gesättigter Natriumchloridlösung gewaschen und nach Trocknen mit Magnesiumsulfat im Vakuum eingedampft. Der Rückstand wird im Hochvakuum fraktioniert destilliert (Siedepunkt = 173 bis 181°C, Druck = 1.7 bis 1.2 mbar).
Ausbeute: 122.6 g (46 % d. Th.)
LC-MS (Methode 4): Rₜ = 1.71 min.
MS (ESI⁺): m/z = 231 [M+H]⁺
¹H-NMR (300MHz, DMSO-d₆): δ = 7.6 (s, 1H), 7.4 - 7.1 (m, 6H), 5.2 (s, 2H), 4.25 (q, 2H), 1.25 (tr, 3H) ppm.

### Beispiel 2A

Imidazol-2-carbonsäureethylester 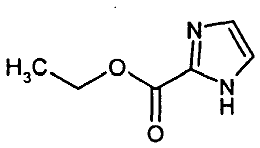 34.7 g (150.9 mmol) 1-Benzyl-1H-imidazol-2-carbonsäureethylester werden in 1005 ml Ethanol gelöst und mit 34 g Ammoniumformiat versetzt. Die Reaktionsmischung wird ca. 6 h zum Rückfluss erhitzt. Dabei werden in kleinen Portionen insgesamt 8 g 10% Palladium auf Aktivkohle und 18 g Ammoniumformiat zugegeben. Nach Abkühlen wird der Katalysator abfiltriert und das Filtrat im Vakuum eingeengt. Das dabei auskristallisierende Produkt wird in 80 ml Eiswasser verrührt und abgesaugt.
Ausbeute: 15.9 g (75 % d. Th.)
MS (ESI⁺): m/z = 141 [M+H]⁺
¹H-NMR (200MHz, DMSO-d₆): δ = 13.3 (s breit, 1H), 7.4 (s, 1H), 7.15 (s, 1H), 4.3 (q, 2H), 1.3 (tr, 3H) ppm.

### Beispiel 3A

4-Nitro-1H-imidazol-2-carbonsäureethylester 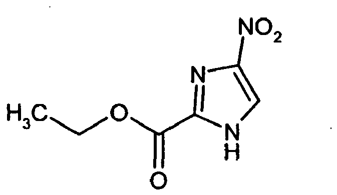 16.08 g (114.7 mmol) Imidazol-2-carbonsäureethylester werden unter Eiskühlung in 71.7 ml konzentrierter Schwefelsäure gelöst. Dann werden 71.7 ml 100%ige rauchende Salpetersäure zugetropft. Die Reaktionslösung wird 3 h bei 50 bis 60°C gerührt und nach Abkühlen auf 800 ml Eis/Wasser-Gemisch gegossen. Die ausfallenden Kristalle werden abgesaugt und mit 1500 ml Eiswasser gewaschen.
Ausbeute: 15 g (70 % d. Th.)
MS (ESI⁺): m/z = 186 [M+H]⁺
¹H-NMR (300MHz, DMSO-d₆): δ = 14.5 (s breit, 1H), 8.5 (s, 1H), 4.4 (q, 2H), 1.35 (tr, 3H), ppm.

### Beispiel 4A

4-({[(4-Chlor-2-methylphenyl)amino]carbonyl}amino)-1-(cyclopropylmethyl)-1H-imidazol-2-carbonsäure

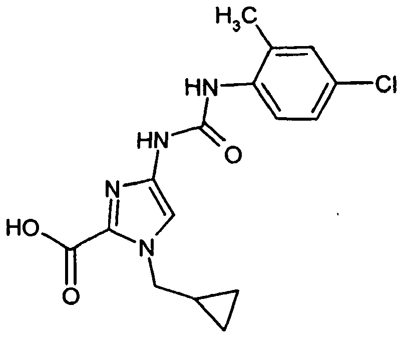

### Stufe 1

1-(Cyclopropylmethyl)-4-nitro-1H-imidazol-2-carbonsäureethylester 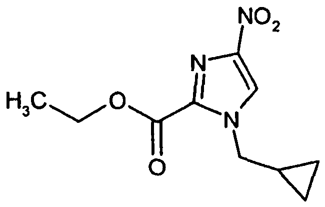 15 g (81 mmol) 4-Nitro-1H-imidazol-2-carbonsäureethylester werden unter Argon zusammen mit 13.13 g (97.2 mmol) Cyclopropylmethylbromid und 22.4 g (162 mmol) Kaliumcarbonat in 165 ml DMF 1 h bei 80°C gerührt. Nach Abkühlen wird die Reaktionsmischung mit Wasser verdünnt und viermal mit Ethylacetat extrahiert. Die vereinigten organischen Phasen werden einmal mit Wasser und dreimal mit gesättigter Natriumchloridlösung gewaschen, mit Magnesiumsulfat getrocknet und im Vakuum eingedampft. Der kristalline Rückstand wird für die nächste Reaktion direkt weiterverwendet.
Ausbeute: 17.59 g (70 % d. Th.)
LC-MS (Methode 2): Rₜ = 2.02 min.
MS (ESI⁺): m/z = 240 [M+H]⁺
¹H-NMR (300MHz, DMSO-d₆): δ = 8.2 (s, 1H), 4.4 (q, 2H), 4.3 (d, 2H), 1.4 (m, 4H), 0.55 (q, 2H), 0.45 (q, 2H) ppm.

### Stufe 2

4-Amino-1-(cyclopropylmethyl)-1H-imidazol-2-carbonsäureethylester 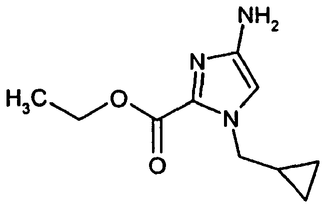 3.89 g (16.26 mmol) 1-(Cyclopropylmethyl)-4-nitro-1H-imidazol-2-carbonsäureethylester werden in 50 ml THF gelöst und mit einer Spatelspitze Raney-Nickel versetzt. Die Reaktionsmischung wird in einer Hydrierungsapparatur bei Raumtemperatur mit Wasserstoff hydriert. Der Katalysator wird abfiltriert und das Filtrat im Vakuum eingedampft. Der Eindampfrückstand wird für die nächste Reaktion direkt weiterverwendet.
Ausbeute: 3.46 g (100 % d. Th.)
LC-MS (Methode 3): Rₜ = 1.21 min.
MS (ESI⁺): m/z = 210 [M+H]⁺
¹H-NMR (300MHz, DMSO-d₆): δ = 6.55 (s, 1H), 4.55 (s, 2H), 4.2 (q, 2H), 4.1 (d, 2H), 1.25 (tr, 3H), 1.2 (m, 1H), 0.5 (q, 2H), 0.3 (q, 2H) ppm.

### Stufe 3

4-({[(4-Chlor-2-methylphenyl)amino]carbonyl}amino)-1-(cyclopropylmethyl)-1H-imidazol-2-carbonsäureethylester 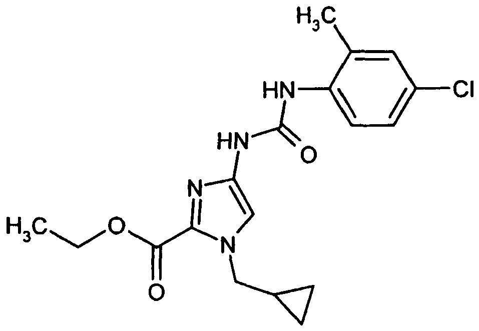 7.49 g (35.8 mmol) 4-Amino-1-(cyclopropylmethyl)-1H-imidazol-2-carbonsäureethylester werden in 18 ml THF unter Argon mit 6 g (35.8 mmol) 3-Chlor-4-phenylisocyanat versetzt und 4 h bei Raumtemperatur gerührt. Die Reaktionsmischung wird im Vakuum eingeengt und das dabei auskristallisierende Produkt in 40 ml Ethylacetat verrührt und abgesaugt.
Ausbeute: 11.1 g (82 % d. Th.)
LC-MS (Methode 2): Rₜ = 2.66 min.
MS (ESI⁺): m/z = 376 [M+H]⁺
¹H-NMR (300MHz, DMSO-d₆): δ = 9.45 (s, 1H), 8.0 (d, 1H), 7.35 (s, 1H), 7.3 (d, 1H), 7.2 (dd, 1H), 4.3 (q, 2H), 4.25 (d, 2H), 2.25 (s, 3H), 1.3 (tr, 3H), 1.25 (m, 1H), 0.55 (q, 2H), 0.35 (q, 2H) ppm.

### Stufe 4

4-({[(4-Chlor-2-methylphenyl)amino]carbonyl}amino)-1-(cyclopropylmethyl)-1H-imidazol-2-carbonsäure 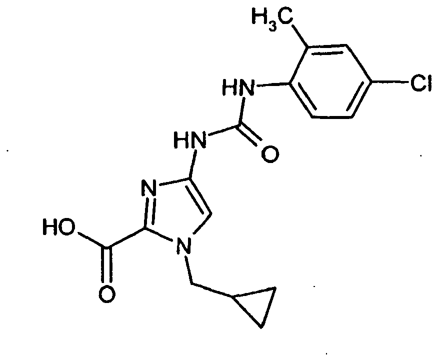 10.6 g (28.1 mmol) 4-({[(4-Chlor-2-methylphenyl)amino]carbonyl}amino)-1-(cyclopropylmethyl)-1H-imidazol-2-carbonsäureethylester werden in 158 ml Ethanol suspendiert. Unter Eiskühlung werden 16.4 ml Wasser und 6 ml (112 mmol) 50%-ige wässrige Natronlauge-Lösung zugegeben. Die Reaktionsmischung wird 1 h bei Raumtemperatur gerührt und dann im Vakuum eingeengt. Der Rückstand wird in 100 ml Isopropanol aufgenommen und unter Eiskühlung mit 100 ml 1N Salzsäure versetzt. Die Kristalle werden abgesaugt und im Vakuum bei 40°C getrocknet.
Ausbeute: 9.85 g (100 % d. Th.)
LC-MS (Methode 4): Rₜ = 1.74 min.
MS (ESI⁺): m/z = 349 [M+H]⁺
¹H-NMR (400MHz, DMSO-d₆): δ = 9.4 (s, 1H), 8.0 (d, 1H), 7.3 (s, 1H), 7.25 (d, 1H), 7.2 (dd, 1H), 4.25 (d, 2H), 2.25 (s, 3H), 1.25 (m, 1H), 0.55 (q, 2H), 0.35 (q, 2H) ppm.

### Beispiel 5A

1-(Cyclopropylmethyl)-4-[({[4-(trifluormethoxy)phenyl]amino}carbonyl)amino]-1H-imidazol-2-carbonsäure 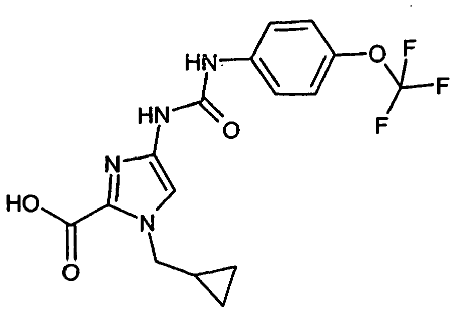 Die Herstellung erfolgt analog zu Beispiel 4A.
Ausbeute: 10.2 g (93 % d. Th.)
LC-MS (Methode 4): Rₜ = 1.87 min.
MS (ESI⁺): m/z = 385 [M+H]⁺
¹H-NMR (400MHz, DMSO-d₆): δ = 8.6 (s, 1H), 8.4 (s, 1H), 7.55 (d, 2H), 7.4 (s, 1H), 7.25 (d, 2H), 4.25 (d, 2H), 1.25 (m, 1H), 0.55 (q, 2H), 0.35 (q, 2H) ppm.

### Beispiel 6A

1-Butyl-4-({[(4-chlor-2-methylphenyl)amino]carbonyl}amino)-1H-imidazol-2-carbonsäure 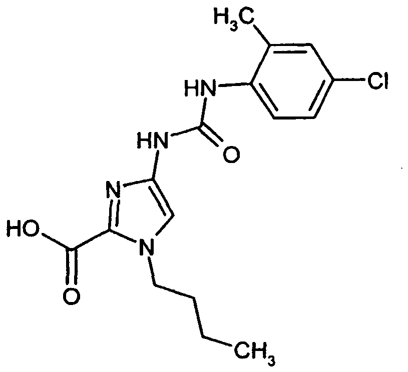 Die Herstellung erfolgt analog zu Beispiel 4A.
Ausbeute: 2.2 g (93 % d. Th.)
LC-MS (Methode 4): Rₜ = 1.83 min.
MS (ESI⁺): m/z = 351 [M+H]⁺
¹H-NMR (300MHz, DMSO-d₆): δ = 9.35 (s, 1H), 8.0 (d, 1H), 7.3 (s, 1H), 7.25 (d, 1H), 7.2 (dd, 1H), 4.35 (tr, 2H), 2.25 (s, 3H), 1.7 (quintett, 2H), 1.25 (sextett, 2H), 0.9 (tr, 3H) ppm.

### Beispiel 7A

1-Butyl-4-[({[4-(trifluormethoxy)phenyl]amino}carbonyl)amino]-1H-imidazol-2-carbonsäure 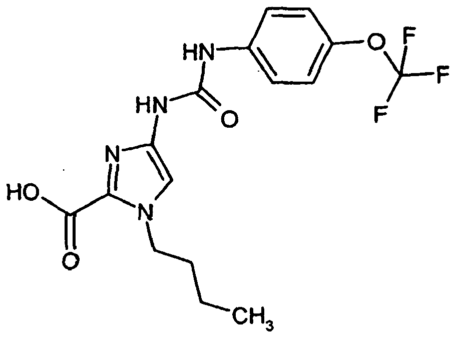 Die Herstellung erfolgt analog zu Beispiel 4A.
Ausbeute: 2.05 g (96 % d. Th.)
LC-MS (Methode 4): Rₜ = 1.96 min.
MS (ESI⁺): m/z = 387 [M+H]⁺
¹H-NMR (300MHz, DMSO-d₆): δ = 9.0 (s, 1H), 8.9 (s, 1H), 7.55 (d, 2H), 7.3 (s, 1H), 7.25 (d, 1H), 4.35 (tr, 2H), 1.7 (quintett, 2H), 1.25 (sextett, 2H), 0.9 (tr, 3H) ppm.

### Beispiel 8A

4-({[(4-Chlor-2-methylphenyl)amino]carbonyl}amino)-1-(2-methoxyethyl)-1H-imidazol-2-carbonsäure 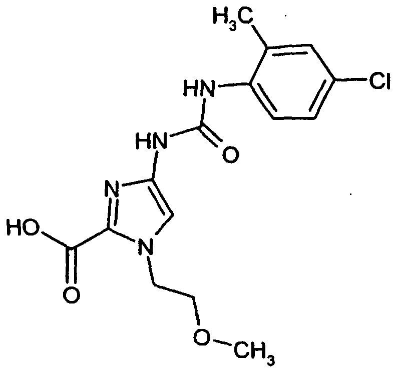 Die Herstellung erfolgt analog zu Beispiel 4A.
Ausbeute: 1.36 g (91 % d. Th.)
LC-MS (Methode 4): Rₜ = 1.56 min.
MS (ESI⁺): m/z = 353 [M+H]⁺
¹H-NMR (300MHz, DMSO-d₆): δ = 9.3 (s, 1H), 8.0 (d, 1H), 7.25 (d, 1H), 7.2 (m, 2H), 4.55 (tr, 2H), 3.65 (tr, 2H), 2.25 (s, 3H) ppm.

### Beispiel 9A

1-(2-Methoxyethyl)-4-[({[4-(trifluormethoxy)phenyl]amino}carbonyl)amino]-1H-imidazol-2-carbonsäure 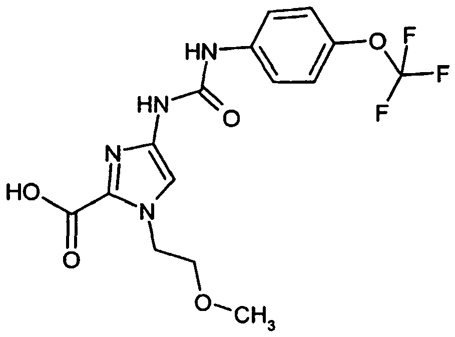 Die Herstellung erfolgt analog zu Beispiel 4A.
Ausbeute: 1 g (49 % d. Th.)
LC-MS (Methode 4): Rₜ = 1.70 min.
MS (ESI⁺): m/z = 389 [M+H]⁺
¹H-NMR (300MHz, DMSO-d₆): δ = 9.15 (s, 1H), 8.45 (s, 1H), 7.55 (d, 2H), 7.3 (s, 1H), 7.25 (d, 2H), 4.55 (tr, 2H), 3.65 (tr, 2H) ppm.

### Beispiel 10A

1-Benzyl-4-({[(4-chlor-2-methylphenyl)amino]carbonyl}amino)-1H-imidazol-2-carbonsäure 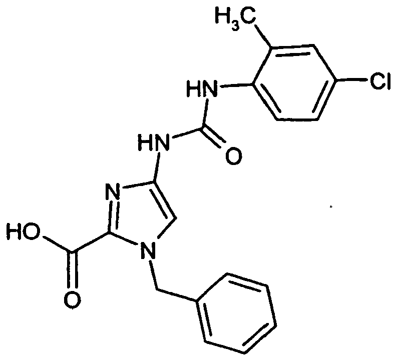 Die Herstellung erfolgt analog zu Beispiel 4A.
Ausbeute: 2.34 g (93 % d. Th.)
LC-MS (Methode 2): Rₜ = 2.12 min.
MS (ESI⁺): m/z = 385 [M+H]⁺
¹H-NMR (300MHz, DMSO-d₆): δ = 9.45 (s, 1H), 7.9 (d, 1H), 7.4-7.1 (m, 8H), 5.65 (s, 2H), 2.25 (s, 3H) ppm.

### Beispiel 11A

1-Benzyl-4-[({[4-(trifluormethoxy)phenyl]amino}carbonyl)amino]-1H-imidazol-2-carbonsäure 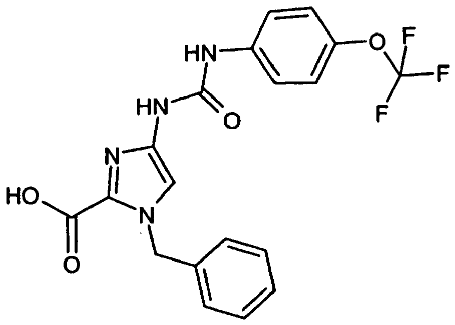 Die Herstellung erfolgt analog zu Beispiel 4A.
Ausbeute: 2.37 g (96 % d. Th.)
LC-MS (Methode 5): Rₜ = 2.29 min.
MS (ESI⁺): m/z = 421 [M+H]⁺
¹H-NMR (300MHz, DMSO-d₆): δ = 9.05 (s, 1H), 8.9 (s, 1H), 7.55 (d, 2H), 7.4-7.2 (m, 8H), 5.6 (s, 2H) ppm.

### Beispiel 12A

4-({[(4-Chlor-2-methylphenyl)amino]carbonyl}amino)-1-(3-methylbut-2-en-1-yl)-1H-imidazol-2-carbonsäure 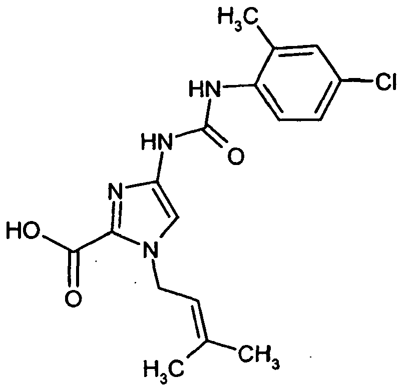 Die Herstellung erfolgt analog zu Beispiel 4A.
Ausbeute: 2.53 g (95 % d. Th.)
LC-MS (Methode 4): Rₜ = 1.91 min.
MS (ESI⁺): m/z = 363 [M+H]⁺
¹H-NMR (300MHz, DMSO-d₆): δ = 9.3 (s, 1H), 8.0 (d, 1H), 7.25 (s, 1H), 7.2 (m, 2H), 5.35 (tr, 1H), 5.0 (d, 2H), 2.25 (s, 3H), 1.8 (s, 3H), 1.75 (s, 3H) ppm.

### Beispiel 13A

1-(3-Methylbut-2-en-1-yl)-4-[({[4-(trifluormethoxy)phenyl]amino}carbonyl)amino]-1H-imidazol-2-carbonsäure 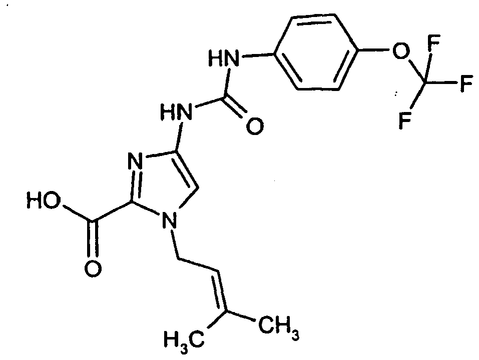 Die Herstellung erfolgt analog zu Beispiel 4A.
Ausbeute: 2.67 g (91 % d. Th.)
LC-MS (Methode 4): Rₜ = 2.03 min.
MS (ESI⁺): m/z = 399 [M+H]⁺
¹H-NMR (300MHz, DMSO-d₆): δ = 9.05 (s, 1H), 8.9 (s, 1H), 7.55 (d, 2H), 7.3 (m, 3H), 5.35 (tr, 1H), 5.0 (d, 2H), 1.75 (s, 3H), 1.7 (s, 3H) ppm.

### Beispiele 14A

4-({[(4-Chlor-2-methylphenyl)amino]carbonyl}amino)-1-(2-phenoxyethyl)-1H-imidazol-2-carbonsäure 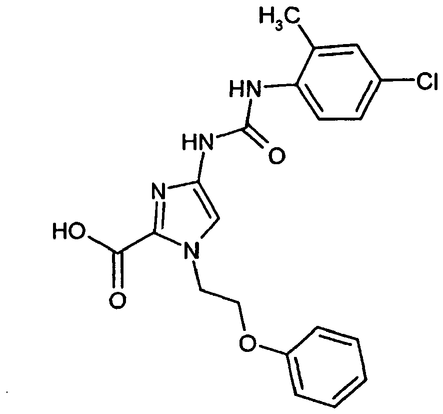 Die Herstellung erfolgt analog zu Beispiel 4A.
Ausbeute: 2.97 g (99 % d. Th.)
LC-MS (Methode 3): Rₜ = 2.21 min.
MS (ESI⁺): m/z = 415 [M+H]⁺
¹H-NMR (300MHz, DMSO-d₆): δ = 9.6 (s, 1H), 8.0 (d, 1H), 7.3-7.1 (m, 5H), 6.9 (m, 3H), 4.8 (tr, 2H), 4.35 (d, 2H), 2.25 (s, 3H) ppm.

### Beispiel 15A

1-(2-Phenoxyethyl)-4-[({(4-(trifluormethoxy)phenyl]amino}carbonyl)amino]-1H-imidazol-2-carbonsäure 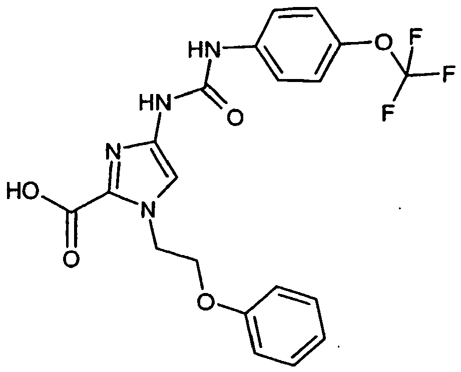 Die Herstellung erfolgt analog zu Beispiel 4A.
Ausbeute: 2.9 g (98 % d. Th.)
LC-MS (Methode 3): Rₜ = 2.31 min.
MS (ESI⁺): m/z = 45 [M+H]⁺
¹H-NMR (300MHz, DMSO-d₆): δ = 8.9 (s, 1H), 7.55 (d, 2H), 7.4 (s, 1H), 7.25 (m, 4H), 6.9 (m, 3H), 4.8 (tr, 2H), 4.35 (d, 2H) ppm.

### Beispiel 16A

4-({[(4-Chlor-2-methylphenyl)amino]carbonyl}amino)-1-(3,3-dichlorprop-2-en-1-yl)-1H-imidazol-2-carbonsäure 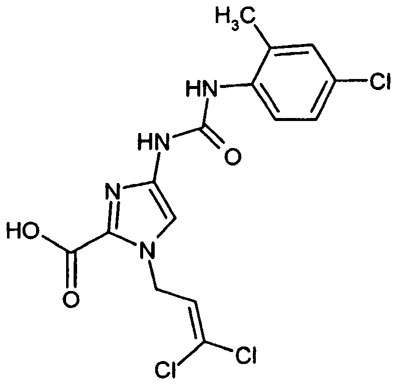 Die Herstellung erfolgt analog zu Beispiel 4A.
Ausbeute: 1.46 g (94 % d. Th.)
LC-MS (Methode 3): Rₜ = 2.21 min.
MS (ESI⁺): m/z = 403 [M+H]⁺
¹H-NMR (300MHz, DMSO-d₆): δ = 9.4 (s, 1H), 7.95 (d, 1H), 7.25 (m, 2H), 7.2 (dd, 2H), 6.4 (tr, 1H), 5.1 (d, 2H), 2.25 (s, 3H) ppm.

### Beispiel 17A

1-(3,3-Dichlorprop-2-en-1-yl)-4-[({[4-(trifluormethoxy)phenyl]amino}carbonyl)amino]-1H-imidazol-2-carbonsäure 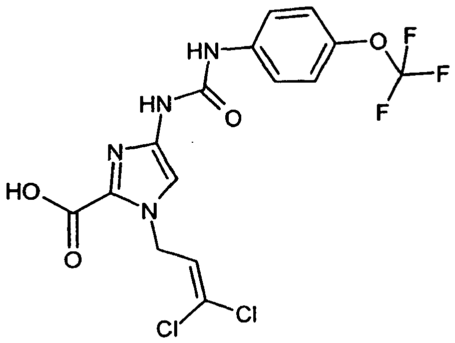 Die Herstellung erfolgt analog zu Beispiel 4A.
Ausbeute: 1.93 g (82 % d. Th.)
LC-MS (Methode 5): Rₜ = 2.35 min.
MS (ESI⁺): m/z = 439 [M+H]⁺
¹H-NMR (400MHz, DMSO-d₆): δ = 9.0 (m, 2H), 7.55 (d, 2H), 7.35 (s, 1H), 7.2 (d, 2H), 6.4 (tr, 1H), 5.15 (d, 2H) ppm.

### Beispiel 18A

4-({[(4-Methyl-3-fluorphenyl)amino]carbonyl}amino)-1-methyl-1H-imidazol-2-carbonsäure

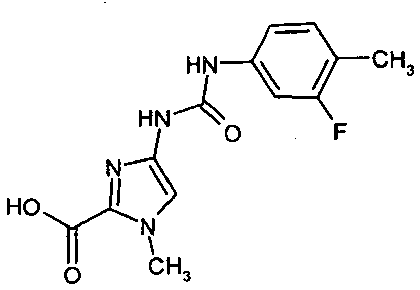

### Stufe 1

4-({[(3-Fluor-4-methylphenyl)amino]carbonyl}amino)-1-methyl-1H-imidazol-2-carbonsäureethylester 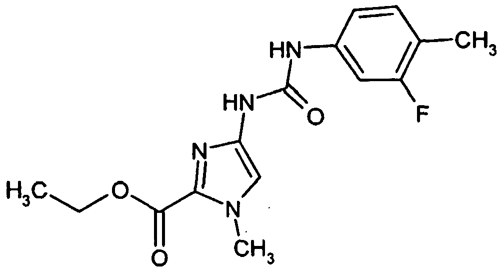 1.30 g (3.84 mmol) 4-Amino-1-methyl-1H-imidazol-2-carbonsäureethylester (Synthese analog Beispiel 4A Stufe 3 oder auch gemäß Tetrahedron Lett. 2003, 44, 1607 und dort zitierter Literatur) werden in 50 ml THF unter Argon mit 1.16 g (7.68 mmol) 3-Fluor-4-methylphenylisocyanat versetzt und über Nacht bei Raumtemperatur gerührt. Die Reaktionsmischung wird filtriert, das Filtrat im Vakuum eingeengt und durch präparative HPLC gereinigt. Der Filtrationsrückstand ergibt zusammen mit dem nach der HPLC-Reinigung erhaltenen Produkt 715 mg Produkt.
Ausbeute: 715 mg (58 % d. Th.)
LC-MS (Methode 6): Rₜ = 2.9 min.
MS (ESI⁺): m/z = 321 [M+H]⁺

### Stufe 2

4-({[(4-Methyl-3-fluorphenyl)amino]carbonyl}amino)-1-methyl-1H-imidazol-2-carbonsäure 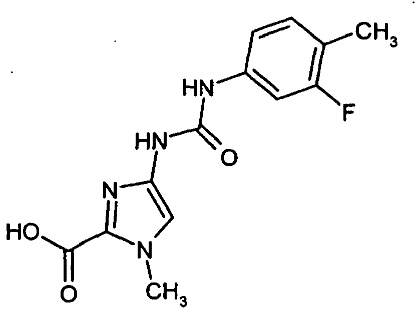 690 mg (2.15 mmol) 4-({[(3-Fluor-4-methylphenyl)amino]carbonyl}amino)-1-methyl-1H-imidazol-2-carbonsäureethylester werden in 5 ml Ethanol und 12 ml Tetrahydrofuran suspendiert. Unter Eiskühlung werden 2 ml (25 mmol) 50%-ige wässrige Natronlauge-Lösung zugegeben. Die Reaktionsmischung wird über Nacht bei Raumtemperatur gerührt und dann unter Eiskühlung mit 1N Salzsäure sauer gestellt. Die Lösung wird mit Dichlormethan extrahiert. Die organisch Phase wird im Vakuum eingeengt.
Ausbeute: quantitativ
LC-MS (Methode 7): Rₜ = 1.65 min.
MS (ESI⁺): m/z = 293 [M+H]⁺

### Beispiel 19A

4-[({[4-(Trifluormethoxy)phenyl]amino}carbonyl)amino]-1-[4-(trifluormethyl)benzyl]-1H-imidazol-2-carbonsäure 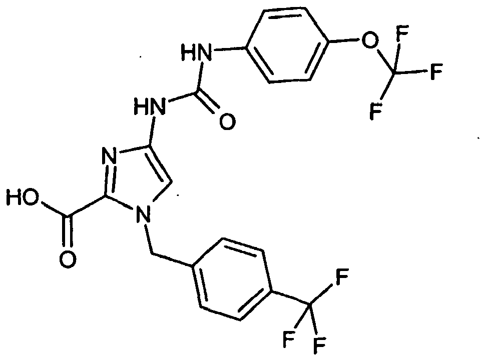 Herstellung analog zu Beispiel 4A.
Ausbeute: 15.2 g (100 % d. Th.)
LC-MS (Methode 2): Rₜ = 2.46 min.
MS (ESI⁺): m/z = 489 [M+H]⁺
¹H-NMR (300MHz, DMSO-d₆): δ = 9.15 (s, 1H), 9.05 (s, 1H), 7.75 (d, 2H), 7.55 (d, 2H), 7.45 (s, 1H), 7.35 (d, 2H), 7.25 (d, 2H), 5.7 (s, 2H) ppm.

### Beispiel 20A

4-({[(4-Chlor-2-methylphenyl)amino]carbonyl}amino)-1-[4-(trifluormethyl)benzyl]-1H-imidazol-2-carbonsäure 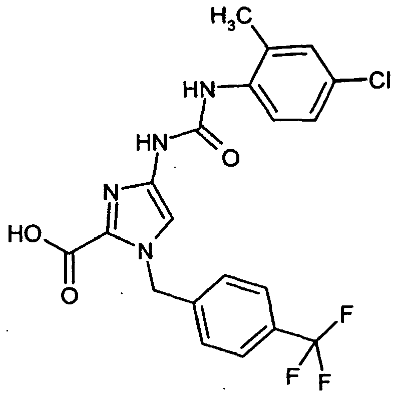 Herstellung analog zu Beispiel 4A.
Ausbeute: 15.6 g (100 % d. Th.)
LC-MS (Methode 4): Rₜ = 2.23 min.
MS (ESI⁺): m/z = 453 [M+H]⁺
¹H-NMR (300MHz, DMSO-d₆): δ = 9.5 (s, 1H), 7.95 (d, 1H), 7.75 (d, 2H); 7.4 (d, 2H), 7.35 (s, 1H), 7.25 (s, 1H), 7.15 (d, 1H), 5.7 (s, 2H), 2.25 (s, 3H) ppm.

### Beispiel 21A

N-(2-Chlorethyl)-4-({[(4-chlor-2-methylphenyl)amino]carbonyl}amino)-1-[4-(trifluormethyl)-benzyl]-1H-imidazol-2-carboxamid 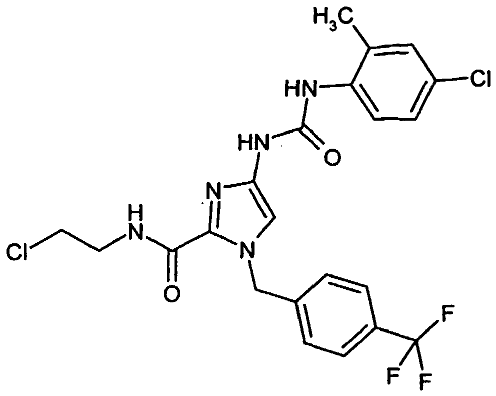 90.6 mg (0.2 mmol) 4-({[(4-Chlor-2-methylphenyl)amino]carbonyl}amino)-1-[4-(trifluormethyl)-benzyl]-1H-imidazol-2-carbonsäure (Beispiel 20A) werden in 500µl DMF gelöst und mit 36.65 mg (0.3 mmol) DMAP und 114 mg (0.3 mmol) HATU versetzt. Nach 5 Minuten Rühren bei RT wird eine Lösung von 46.4 mg (0.4 mmol) 1-Chlor-2-aminoethan und 25.8 mg (0.2 mmol) N,N-Diisopropylethylamin in 500 µl DMF zugetropft. Die Reaktionsmischung wird 6 h bei RT gerührt, danach filtriert und durch präparative HPLC gereinigt (Methode 9). Die Produkt enthaltenden Fraktionen werden im Vakuum eingedampft.
Ausbeute: 61.1 mg (59 % d. Th.)
LC-MS (Methode 4): Rₜ = 2.67 min
MS (ESI⁺): m/z = 514 [M+H]⁺
¹H-NMR (300MHz, DMSO-d₆): δ = 9.25 (s, 1H), 8.35 (s breit, 2H), 7.9 (d, 1H), 7.7 (d, 2H), 7.45 (d, 2H), 7.3 (s, 1H), 7.25 (s, 1H), 7.15 (d, 1H), 5.75 (s, 2H), 3.7 (tr, 2H), 3.55 (q, 2H), 2.25 (s, 3H) ppm.

### Beispiel 22A

N-(2-Chlorethyl)-4-[({[4-(trifluormethoxy)phenyl]amino}carbonyl)amino]-1-[4-(trifluormethyl)-benzyl]-1H-imidazol-2-carboxamid 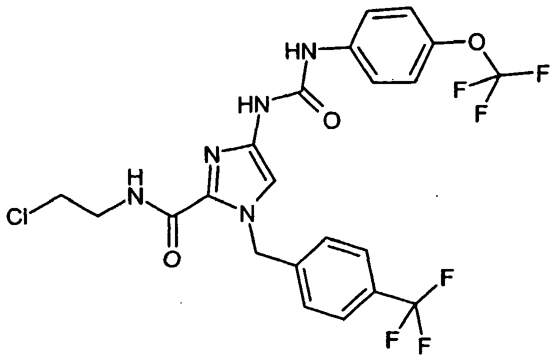 Die Herstellung erfolgt analog zu Beispiel 21A aus 4-[({[4-(Trifluormethoxy)phenyl]-amino}carbonyl)amino]-1-[4-(trifluormethyl)benzyl]-1H-imidazol-2-carbonsäure (Beispiel 19A).
Ausbeute: 635 mg (58 % d. Th.)
LC-MS (Methode 4): Rₜ = 2.73 min.
MS (ESI⁺): m/z = 550 [M+H]⁺
¹H-NMR (300MHz, DMSO-d₆): δ = 9.5 (s, 1H), 8.8 (s, 1H), 8.4 (tr, 1H), 7.75 (d, 2H), 7.55 (d, 2H), 7.45 (s, 2H), 7.35 (d, 1H), 7.25 (d, 2H), 5.75 (s, 2H), 3.7 (tr, 2H), 3.55 (q, 2H) ppm.

### Beispiel 23A

N-(2-Chlorethyl)-1-(cyclopropylmethyl)-4-[({[4-(trifluormethoxy)phenyl]amino}carbonyl)amino]-1H-imidazol-2-carboxamid 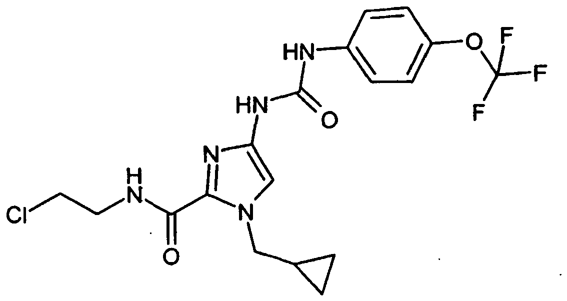 Die Herstellung erfolgt analog zu Beispiel 21A aus 1-(Cyclopropylmethyl)-4-[({[4-(trifluormethoxy)phenyl]amino}carbonyl)amino]-1H-imidazol-2-carbonsäure (Beispiel 5A).
Ausbeute: 668 mg (75 % d. Th.)
LC-MS (Methode 4): Rₜ = 2.45 min.
MS (ESI⁺): m/z = 446 [M+H]⁺
¹H-NMR (300MHz, DMSO-d₆): δ = 9.05 (s, 1H), 8.7 (s, 1H), 8:3 (tr, 1H), 7.55 (d, 2H), 7.3 (d, 1H), 7.25 (d, 2H), 4.3 (d, 2H), 3.7 (tr, 2H), 3.55 (q, 2H), 1.35 (m, 1H), 0.5 (m, 2H), 0.35 (m, 2H) ppm.

### Beispiel 24A

2-[(2-Bromacetyl)oxy]ethyl-4-({[(4-chlor-2-methylphenyl)amino]carbonyl}amino)-1-[4-(trifluormethyl)benzyl]-1H-imidazol-2-carboxylat 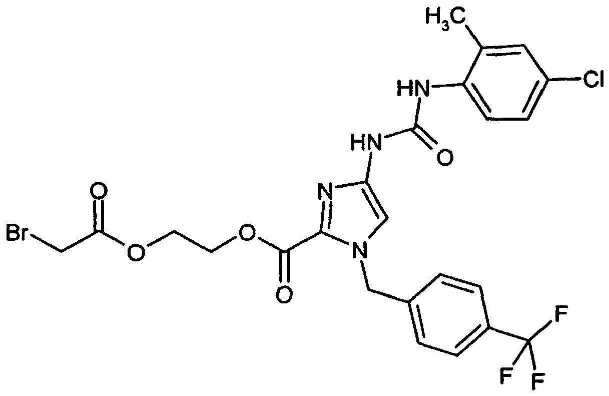 1 g (2 mmol) 2-Hydroxyethyl-4-({[(4-chlor-2-methylphenyl)amino]carbonyl}amino)-1-[4-(trifluormethyl)benzyl]-1H-imidazol-2-carboxylat (Beispiel 125) werden in einer Mischung aus 25 ml Dichlormethan und 12 ml DMF gelöst und mit 1570 µl (1.14 g, 11.2 mmol) Triethylamin versetzt. Unter Eiskühlung werden 804 µl (1.52 g, 9.6 mmol) Bromacetylchlorid und eine katalytische Menge DMAP zugegeben. Nach 16 h Rühren bei RT werden erneut 0.57 g (5.6 mmol) Triethylamin, 0.76 g (4.8 mmol) Bromacetylchlorid und wenige Kristalle DMAP unter Eiskühlung zugegeben. Die Reaktionsmischung wird 2.5 h bei RT gerührt, in gesättigte Natriumhydrogencarbonatlösung gegossen und zweimal mit Ethylacetat extrahiert. Die vereinten Extrakte werden zweimal mit Wasser und einmal mit gesättigter Natriumchloridlösung gewaschen, mit Magnesiumsulfat getrocknet und im Vakuum eingedampft. Der Eindampfrückstand wird durch Chromatographie an Kieselgel mit Dichlormethan/Methanol als Elutionsmittel gereinigt.
Ausbeute: 733 mg (59 % d. Th.)
LC-MS (Methode 4): Rₜ = 2.69 min.
MS (ESI⁺): m/z = 617 [M+H]⁺
¹H-NMR (300MHz, DMSO-d₆): δ = 9.55 (s, 1H), 8.5 (s breit, 1H), 7.95 (d, 1H), 7.7 (d, 2H), 7.45 (s, 1H), 7.4 (d, 2H), 7.25 (d, 1H), 7.15 (dd, 1H), 5.7 (s, 2H), 4.4 (m, 4H), 4.35 (s, 2H), 2.25 (s, 3H) ppm.

### Beispiel 25A

2-[({4-[({[2-Methyl-4-(trifluormethoxy)phenyl]amino}carbonyl)amino]-1-[4-(trifluormethyl)-benzyl]-1H-imidazol-2-yl}carbonyl)amino]ethylchloracetat 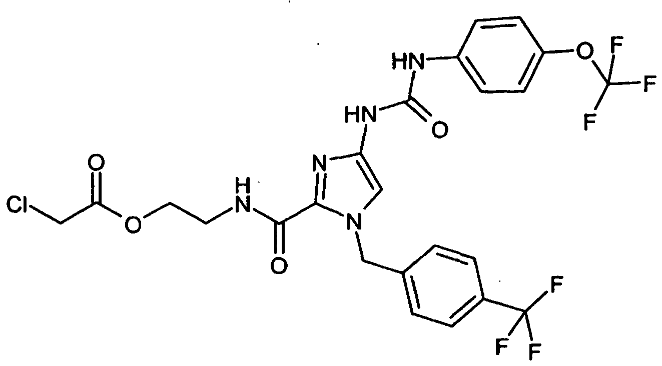 850 mg (1.6 mmol) N-(2-Hydroxyethyl)-4-[({[4-(trifluormethoxy)phenyl]amino}carbonyl)amino]-1-[4-(trifluormethyl)benzyl]-1H-imidazol-2-carboxamid (Beispiel 124) werden unter Argon in 3.2 ml absolutem DMF gelöst und mit wenigen Kristallen DMAP und 647 mg (892 µl, 6.4 mmol) Triethylamin versetzt. Unter Eiskühlung werden langsam 755 mg (400 µl, 4.8 mmol) Bromacetylchlorid zugetropft. Die Reaktionsmischung wird 1.5 h bei RT gerührt und dann in kalte gesättigte Natriumhydrogencarbonatlsösung gegossen und zweimal mit Ethylacetat extrahiert. Die vereinten Extrakte werden einmal mit Wasser und dreimal mit gesättigter Natriumchloridlösung gewaschen, mit Magnesiumsulfat getrocknet und im Vakuum eingedampft.
Ausbeute: 1 g (100 % d. Th.)
LC-MS (Methode 5): Rₜ = 2.86 min.
MS (ESI⁺): m/z = 608 [M+H]⁺
¹H-NMR (300MHz, DMSO-d₆): δ = 9.15 (s, 1H), 8.75 (s, 1H), 8.35 (tr, 1H), 7.7 (d, 2H), 7.55 (d, 2H), 7.45 (s, 2H), 7.35 (d, 1H), 7.25 (d, 2H), 5.75 (s, 2H), 4.3 (s, 2H), 4.35 (tr, 2H), 3.5 (q, 2H) ppm.

### Beispiele 26A

2-[({4-({[(4-Chlor-2-methylphenyl)amino]carbonyl}amino)-1-[4-(trifluormethyl)benzyl]-1H-imidazol-2-yl}carbonyl)amino]ethylchloracetat 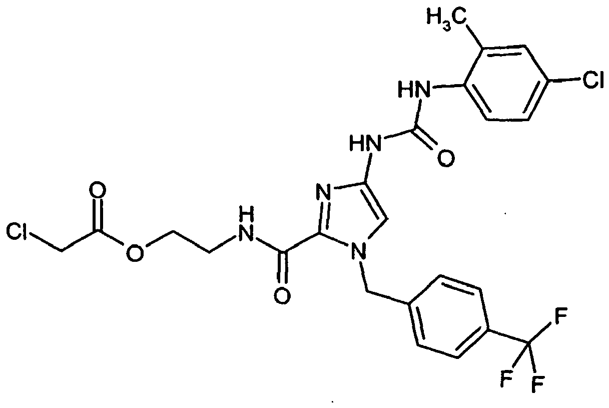 Die Herstellung erfolgt analog zu Beispiel 25A aus 4-({[(4-Chlor-2-methylphenyl)-amino]carbonyl}amino)-N-(2-hydroxyethyl)-1-[4-(trifluormethyl)benzyl]-1H-imidazol-2-carboxamid (Beispiel 123). Ausbeute: 937 mg (100 % d. Th.)
LC-MS (Methode 4): Rₜ = 2.80 min.
MS (ESI⁺): m/z = 572 [M+H]⁺
¹H-NMR (300MHz, DMSO-d₆): δ = 9.25 (s, 1H), 8.35 (s breit, 2H), 7.9 (d, 1H), 7.7 (d, 2H), 7.4 (d, 2H), 7.3 (s, 1H), 7.25 (d, 1H), 7.15 (dd, 1H), 5.75 (s, 2H), 4.3 (s, 2H), 4.25 (tr, 2H), 3.5 (q, 2H), 2.25 (s, 3H) ppm.

### Beispiel 27A

2-[(2-Bromacetyl)oxy]ethyl-4-[({[4-(trifluormethoxy)phenyl]amino}carbonyl)amino]-1-[4-(trifluormethyl)benzyl]-1H-imidazol-2-carboxylat 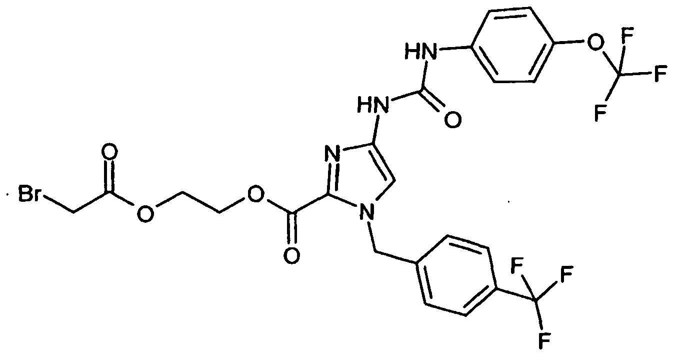 Die Herstellung erfolgt analog zu Beispiel 24A aus 2-Hydroxyethyl-4-[({[4-(trifluormethoxy)-phenyl]amino}carbonyl)amino]-1-[4-(trifluormethyl)benzyl]-1H-imidazol-2-carboxylat (Beispiel 126). Ausbeute: 834 mg (85 % d. Th.)
LC-MS (Methode 4): Rₜ = 2.69 min.
MS (ESI⁺): m/z = 653 [M+H]⁺
¹H-NMR (300MHz, DMSO-d₆): δ = 9.15 (s, 1H), 8.95 (s, 1H), 7.7 (d, 2H), 7.55 (s, 1H), 7.55 (d, 2H), 7.4 (d, 2H), 7.25 (d, 2H), 5.7 (s, 2H), 4.4 (m, 4H), 4.35 (s, 2H) ppm.

### Beispiel 28A

1-Methyl-4-[({[4-(trifluormethoxy)phenyl]amino}carbonyl)amino]-1*H*-imidazol-2-carbonsäure-ethylester 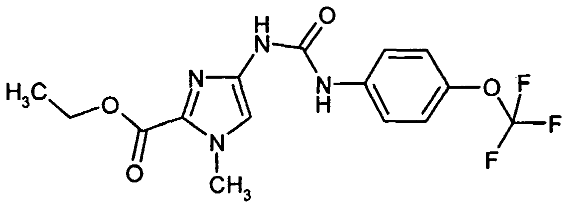 1.22 g (3.61 mmol) 4-Amino-1-methyl-1H-imidazol-2-carbonsäureethylester (Synthese analog Beispiel 4A Stufe 3 oder auch gemäß Tetrahedron Lett. **2003**, *44*, 1607 und dort zitierter Literatur) werden in 50 ml THF unter Argon mit 1.46 g (7.21 mmol) 4-(Trifluormethoxy)phenylisocyanat versetzt und über Nacht bei Raumtemperatur gerührt. Die Reaktionsmischung wird filtriert, das Filtrat im Vakuum eingeengt und chromatographisch gereinigt.
Ausbeute: 860 mg (62 % d. Th.)
LC-MS (Methode 5): Rₜ = 2.41 min.
MS (ESI⁺): m/z = 373 [M+H]⁺

### Beispiel 29A

1-Methyl-4-[({[4-(trifluormethoxy)phenyl]amino}carbonyl)amino]-1*H*-imidazol-2-carbonsäure 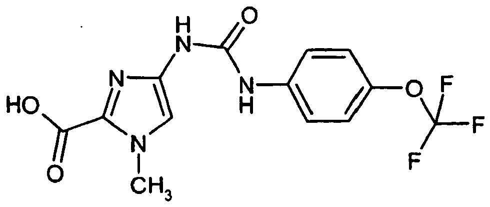 835 mg (2.13 mmol) 1-Methyl-4-[({[4-(trifluormethoxy)phenyl]amino}carbonyl)amino]-1H-imidazol-2-carbonsäureethylester (Beispiel 28A) werden in 5 ml Ethanol und 12 ml Tetrahydrofuran suspendiert. Unter Eiskühlung werden 2 ml (25 mmol) 50%ige wässrige Natronlauge-Lösung zugegeben. Die Reaktionsmischung wird über Nacht bei Raumtemperatur gerührt und dann unter Eiskühlung mit 1N Salzsäure sauer gestellt. Die Lösung wird mit Dichlormethan extrahiert. Die organische Phase wird im Vakuum eingeengt. Der Rückstand wird durch präparative HPLC gereinigt.
Ausbeute: 346 mg (44 % d. Th.).
LC-MS (Methode 4): Rₜ = 1.62 min.
MS (ESI⁺): m/z = 345 [M+H]⁺

### Beispiel 30A

1-Butyl-4-[({[4-(trifluormethyl)phenyl]amino}carbonyl)amino]-1H-imidazol-2-carbonsäure 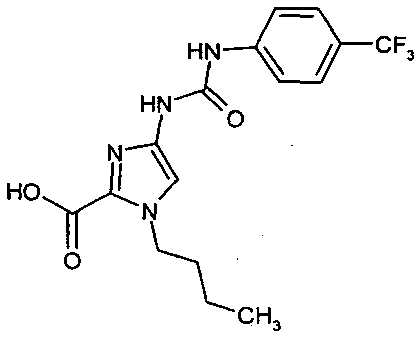 Die Herstellung erfolgt analog zu Beispiel 4A.
Ausbeute: 1.71 g (98 % d. Th.)
LC-MS (Methode 2): 1;4 = 2.13 min.
MS (ESI⁺): m/z = 371 [M+H]⁺
¹H-NMR (300MHz, DMSO-d₆): δ = 9.30 (bs, 1H), 9.03 (bs, 1H), 7.64 (m, 4H), 7.36 (s, 1H), 4.35 (t, 2H), 1.68 (quint, 2H), 1.26 (sext, 2H), 0.89 (t, 3H).

### Beispiel 31A

1-Ethyl-4-[({[4-(trifluormethoxy)phenyl]amino}carbonyl)amino]-1H-imidazol-2-carbonsäure 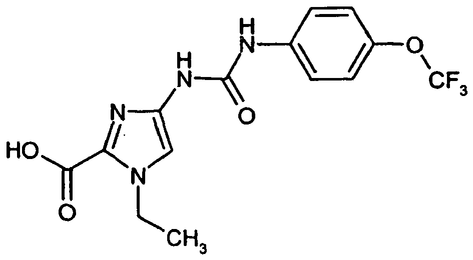 Die Herstellung erfolgte analog zu Beispiel 4A.
LC-MS (Methode 5): Rₜ = 1.94 min.
MS (ESI+): m/z = 359 [M+H]⁺
¹H-NMR (300MHz, DMSO-d₆): δ = 10.3 (bs, 1H), 7.67 (m, 2H), 7.24 (s, 1H), 7.20 (m, 2H), 4.45 (q, 2H), 1.33 (t, 3H).

### Beispiel 32A

1-(Ethyl)-4-[({[4-(trifluormethoxy)phenyl]amino}carbonyl)amino]-1H-imidazol-2-carbonsäure 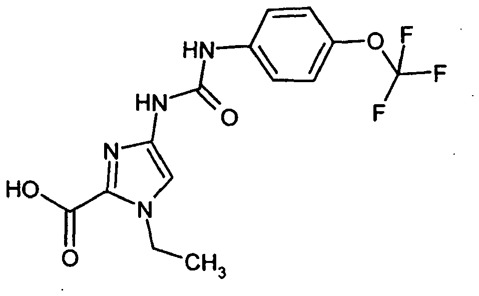 Die Herstellung erfolgt analog zu Beispiel 4A.
Ausbeute: 2 g (91 % d. Th.)
HPLC (Methode 11): Rₜ = 4.00 min.
MS (ESI⁺): m/z= 359 [M+H]⁺
¹H-NMR (300MHz, DMSO-d₆): δ = 10.5 (s breit, 1H), 7.65 (d, 2H), 7.2 (s, 1H), 7.1 (d, 2H), 4.45 (q, 2H), 1.35 (t, 3H) ppm.

### Beispiel 33A

1-(Butyl)-4-[({[4-(difluormethoxy)phenyl]amino}carbonyl)amino]-1H-imidazol-2-carbonsäure 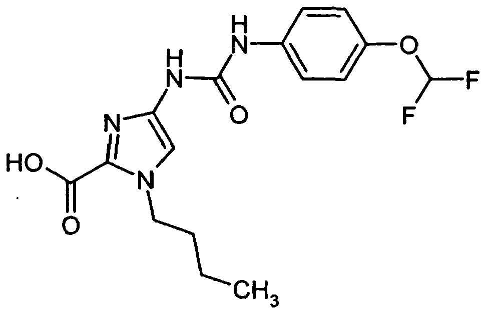 Die Herstellung erfolgt analog zu Beispiel 4A.
Ausbeute: 1.06 g (71 % d. Th.)
HPLC (Methode 11): Rₜ = 4.05 min.
MS (ESI⁺): m/z = 369 [M+H]⁺
¹H-NMR (300MHz, DMSO-d₆): δ = 11. (s breit, 1H), 7.7 (d, 2H), 7.1 (t, 1H, J_{HF} = 75 Hz), 7.05 (m, 3H), 4.5 (t, 2H), 1.7 (m, 2H), 1.3 (m, 2H), 0.9 (t, 3H) ppm.

### Ausführungsbeispiele

### Beispiel 1

4-({[(3-Fluor-4-methylphenyl)amino]carbonyl}amino)-1-methyl-N-(pyridin-3-ylmethyl)-1H-imidazol-2-carboxamid 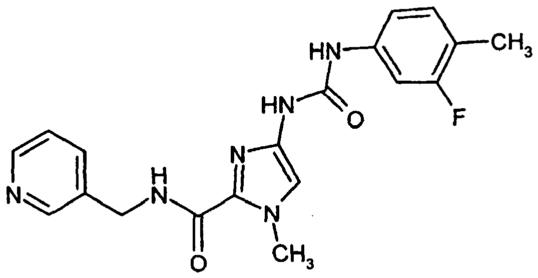 60 mg (0.2 mmol) 4-({[(4-Methyl-3-fluorphenyl)amino]carbonyl}amino)-1-methyl-1H-imidazol-2-carbonsäure (Beispiel 18A) werden in 4 ml DMF gelöst, mit 150 mg (0.39 mmol) HATU und 36 mg (0.3 mmol) DMAP versetzt und 15 Minuten bei Raumtemperatur gerührt. Zu dieser Lösung werden 43 mg (0.39 mmol) (Pyridin-3-ylmethyl)amin getropft und über Nacht gerührt. Die Reaktionsmischung wird durch präparative HPLC gereinigt.
Ausbeute: 61 mg (81 % d. Th.)
LC-MS (Methode 8): Rₜ = 2.04 min.
MS (ESI⁺): m/z= 383 [M+H]⁺
¹H-NMR (300MHz, DMSO-d₆): δ = 9.82 (s, 1H), 9.00 (t, 1H), 8.87 (s, 1H), 8.81 (d, 1H), 8.51 (d, 1H), 8.01 (dd, 1H), 7.45 (dd, 1H), 7.27 (s, 1H), 7.14 (t, 1H), 7.02 (dd, 1H), 4.60 (d, 2H), 3.91 (s, 3H), 2.15 (s, 3H) ppm.

### Beispiel 2

4-({[(3-Fluor-4-methylphenyl)amino]carbonyl}amino)-N-[1-(6-methoxypyridin-3-yl)ethyl]-1-methyl-1H-imidazol-2-carboxamid 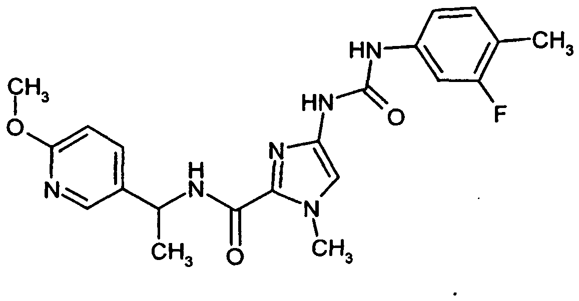 60 mg (0.2 mmol) 4-({[(4-Methyl-3-fluorphenyl)amino]carbonyl}amino)-1-methyl-1H-imidazol-2-carbonsäure (Beispiel 18A) werden in 4 ml DMF gelöst, mit 150 mg (0.39 mmol) HATU und 36 mg (0.3 mmol) DMAP versetzt und 15 Minuten bei Raumtemperatur gerührt. Zu dieser Lösung werden 60 mg (0.39 mmol) [1-(6-Methoxypyridin-3-yl)ethyl]amin getropft und über Nacht gerührt. Die Reaktionsmischung wird durch präparative HPLC gereinigt. Die richtigen Fraktionen werden im Vakuum eingedampft.
Ausbeute: 71 mg (85 % d. Th.)
LC-MS (Methode 8): Rₜ = 2.88 min.
MS (ESI⁺): m/z = 427 [M+H]⁺
¹H-NMR (300MHz, DMSO-d₆): δ = 9.20 (s, 1H), 8.70 (s, 1H), 8.48 (d, 1H), 8.17 (d, 1H), 7.84 (dd, 1H), 7.44 (dd, 1H), 7.20 (s, 1H), 7.16 (dd, 1H), 7.01 (dd, 1H), 6.79 (d, 1H), 5.07 (m, 1H), 3.88 (s, 3H), 3.83 (s, 3H), 2.16 (s, 3H), 1.49 (d, 3H) ppm.

### Beispiel 3

4-({[(4-Chlor-2-methylphenyl)amino]carbonyl}amino)-1-(cyclopropylmethyl)-N-(3-morpholin-4-ylpropyl)-1H-imidazol-2-carboxamid 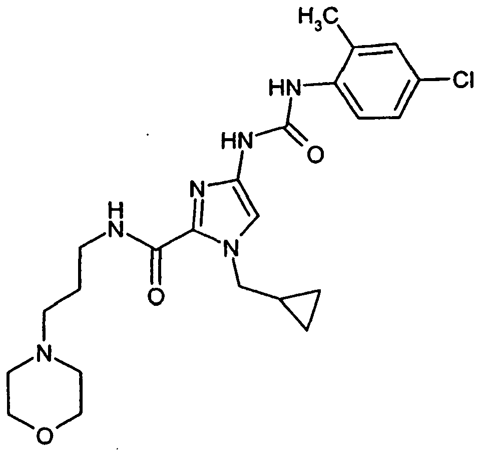 34.8 mg (0.1 mmol) 4-({[(4-Chlor-2-methylphenyl)amino)carbonyl}amino)-1-(cyclopropylmethyl)-1H-imidazol-2-carbonsäure (Beispiel 4A) werden in 0.3 ml DMF gelöst, mit 39.7 mg (0.1 1 mmol) HBTU und 10.12 mg (14 µl; 0.1 mmol) Triethylamin versetzt und 30 Minuten bei Raumtemperatur gerührt. Diese Lösung wird zu einer Lösung von 28.8 mg (0.2 mmol) (3-Morpholin-4-ylpropyl)amin in 0.1 ml DMF gegeben und über Nacht geschüttelt. Nach Filtration wird die Reaktionsmischung durch präparative HPLC gereinigt (Säule: Macherey-Nagel VP 50/21 1 Nucleodur C18 Gravity, 5 µm; Flussrate: 25 ml/min; Eluent A: Acetonitril, Eluent B: Wasser + 0.1% konz. wässrige Ammoniaklösung, Gradient: 0 min 10%A, 2.00 min 10%A, 6.00 min 90%A, 7.00 min 90%A, 7.10 min 10%A, 8 min 10%A; Laufzeit: ca. 10 min pro Trennung; Wellenlänge: 220 nm). Die richtigen Fraktionen werden im Vakuum eingedampft.
Ausbeute: 32 mg (67 % d. Th.)
LC-MS (Methode 2): Rₜ = 1.66 min.
MS (ESI⁺): m/z = 475 [M+H]⁺
¹H-NMR (400MHz, DMSO-d₆): δ = 9.1 (s, 1H), 8.7 - 8.0 (s sehr breit, 1H), 8.2 (s, 1H), 7.9 (d, 1H), 7.25 (s, 1H), 7.2 (m, 2H), 4.25 (d, 2H), 3.6 (tr, 4H), 3.25 (q, 2H), 2.3 (m, 6H), 2.25 (s, 3H), 1.65 (quintett, 2H), 1.25 (m, 1H), 0.5 (q, 2H), 0.35 (q, 2H) ppm.

### Beispiel 4

1-(2-Methoxyethyl)-N-(pyridin-3-ylmethyl)-4-[({[4(trifluormethoxy)phenyl]amino}carbonyl)-amino]-1H-imidazol-2-carboxamid 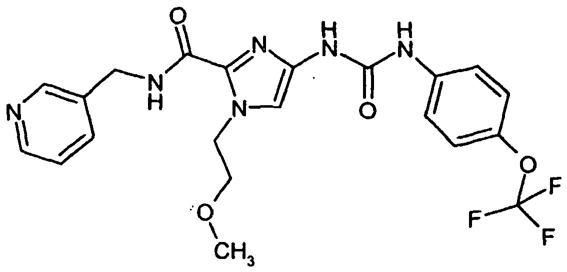 38.8 mg (0.1 mmol) 1-(2-Methoxyethyl)-4-[({[4-(trifluormethoxy)phenyl]amino}carbonyl)amino]-1H-imidazol-2-carbonsäure (Beispiel 9A) werden in 0.4 ml DMF gelöst, mit 81 mg (0.5 mmol) N,N-Carbonyldiimidazol versetzt und 50 Minuten bei Raumtemperatur gerührt. Dann werden 18 µl (18 mg; 1 mmol) Wasser zugegeben und 30 Minuten bei Raumtemperatur gerührt. Nach Zugabe von 21.6 mg (0.2 mmol) 3-Picolylamin wird 90 Minuten bei Raumtemperatur gerührt. Nach Filtration wird die Reaktionsmischung durch präparative HPLC gereinigt (Säule: Macherey-Nagel VP 50/21 Nucleodur C18 Gravity, 5 µm; Flussrate: 25 ml/min; Eluent A: Acetonitril, Eluent B: Wasser + 0.1% konz. wässrige Ammoniaklösung, Gradient: 0 min 10%A, 2.00 min 10%A, 6.00 min 90%A, 7.00 min 90%A, 7.10 min 10%A, 8 min 10%A; Laufzeit: ca. 10 min. pro Trennung; Wellenlänge: 220 nm). Die richtigen Fraktionen werden im Vakuum eingedampft.
Ausbeute: 15.8 mg (33 % d. Th.)
LC-MS (Methode 5): Rₜ = 1.87 min.
MS (ESI⁺): m/z = 479 [M+H]⁺
¹H-NMR (400MHz, DMSO-d₆): δ = 9.1 (s, 1H), 8.8 (tr, 1H), 8.7 (s, 1H), 8.55 (s, 1H), 8.45 (d, 1H), 7.7(d, 1H), 7.55 (d, 2H), 7.35 (dd, 1H), 7.3 (m, 3H), 4.55 (tr, 2H), 4.4 (d, 2H), 3.6 (tr, 2H), 3.25 (s, 3H) ppm.

Analog zu den Beispielen 3 und 4 werden die in der Tabelle 1 aufgeführten Beispiel 5 bis 121 hergestellt.

| **Tabelle 1** | | | | | | | |
|---|---|---|---|---|---|---|---|
| **Beispiel-Nr.** | **Struktur** | **Molmasse** | **MS (EI) [M+H]⁺** | **LC-MS Retentionszeit [min] (Methode)** | **Ausgangsverbindung** | **Herstellung analog zu Beispiel Nr.** | **Ausbeute [mg] (% d. Th.)** |
| **5** | 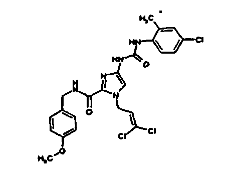 | 522,82 | 522 | 2,85 (2) | Beispiel 16A | 3 | 26 (50) |
| **6** | 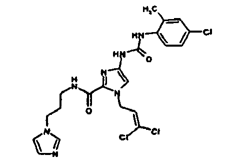 | 510,81 | 510 | 1,82 (2) | Beispiel 16A | 3 | 22 (43) |
| **7** | 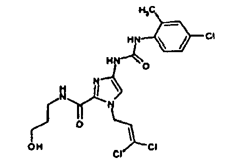 | 460,75 | 460 | 2,29 (2) | Beispiel 16A | 3 | 20 (43) |
| **8** | 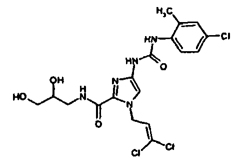 | 476,75 | 476 | 2,13 (2) | Beispiel 16A | 3 | 2,7 (6) |
| **9** | 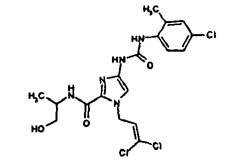 | 460,75 | 460 | 2,35 (2) | Beispiel 16A | 3 | 18 (39) |
| **10** | 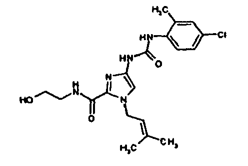 | 405,88 | 406 | 2,22 (2) | Beispiel 12A | 3 | 33 (81) |
| **11** | 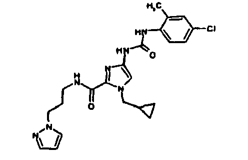 | 455,95 | 456 | 2,35 (2) | Beispiel 4A | 3 | 21 (46) |
| **12** | 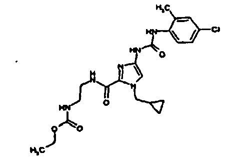 | 462,94 | 463 | 2,31 (2) | Beispiel 4A | 3 | 21 (45) |
| **13** | 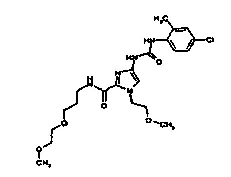 | 467,95 | 468 | 2,12 (2) | Beispiel 8A | 3 | 29 (62) |
| **14** | 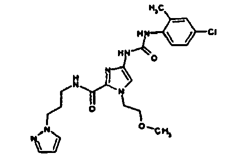 | 459,94 | 460 | 2,13 (2) | Beispiel 8A | 3 | 22 (48) |
| **15** | 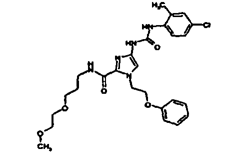 | 530,02 | 531 | 2,54 (2) | Beispiel 14A | 3 | 19 (36) |
| **16** | 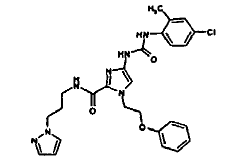 | 522,01 | 523 | 2,53 (2) | Beispiel 14A | 3 | 17 (33) |
| **17** | 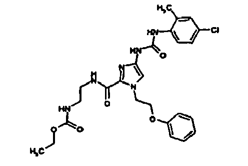 | 528,99 | 529 | 2,49 (2) | Beispiel 14A | 3 | 16 (30) |
| **18** | 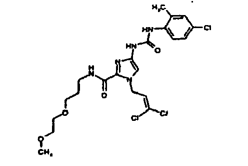 | 518,83 | 518 | 2,57 (2) | Beispiel 16A | 3 | 8 (15) |
| **19** | 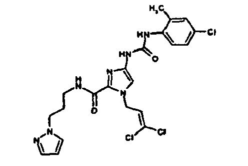 | 510,81 | 510 | 2.55 (2) | Beispiel 16A | 3 | 28 (55) |
| **20** | 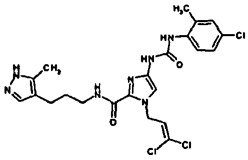 | 524,84 | 524 | 1,84 (2) | Beispiel 16A | 3 | 25 (48) |
| **21** | 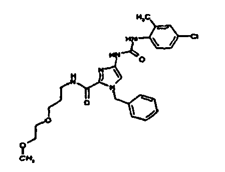 | 500,00 | 500 | 2,49 (2) | Beispiel 10A | 3 | 21 (42) |
| **22** | 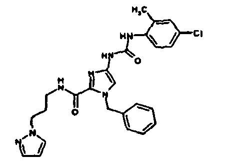 | 491,98 | 492 | 2,48 (2) | Beispiel 10A | 3 | 15 (30) |
| **23** | 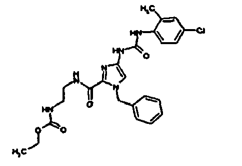 | 498,97 | 499 | 2,45 (2) | Beispiel 10A | 3 | 3,3 (7) |
| **24** | 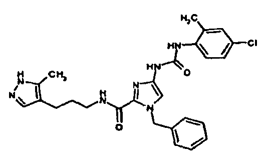 | 506,01 | 507 | 2,33 (2) | Beispiel 10A | 3 | 26 (51) |
| **25** | 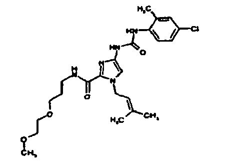 | 477,99 | 478 | 2,52 (2) | Beispiel 12A | 3 | 21 (44) |
| **26** | 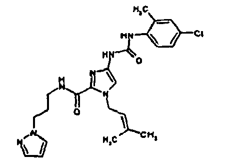 | 469,97 | 470 | 2,52 (2) | Beispiel 12A | 3 | 24 (51) |
| **27** | 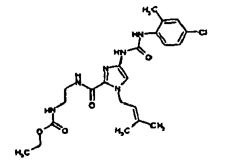 | 476,96 | 477 | 2,47 (2) | Beispiel 12A | 3 | 22 (46) |
| **28** | 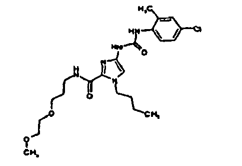 | 465,98 | 466 | 2,47 (2) | Beispiel 6A | 3 | 26 (56) |
| **29** | 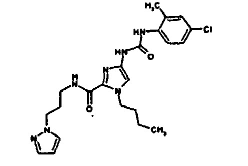 | 457,96 | 458 | 2,47 (2) | Beispiel 6A | 3 | 29 (63) |
| **30** | 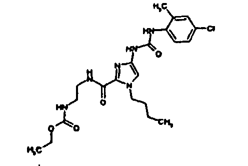 | 464,95 | 465 | 2,42 (2) | Beispiel 6A | 3 | 13 (28) |
| **31** | 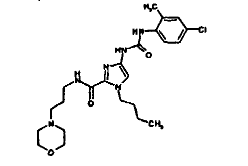 | 477,01 | 478 | 1,78 (2) | Beispiel 6A | 3 | 34 (71) |
| **32** | 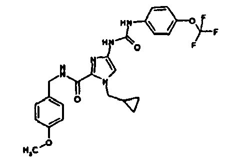 | 503,48 | 504 | 2,75 (2) | Beispiel 5A | 3 | 11 (22) |
| **33** | 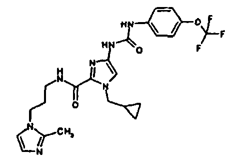 | 505,50 | 506 | 1,93 (2) | Beispiel 5A | 3 | 12 (24) |
| **34** | 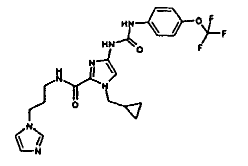 | 491,47 | 492 | 1,90 (2) | Beispiel 5A | 3 | 11 (22) |
| **35** | 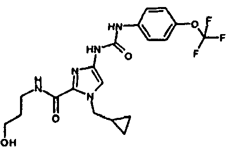 | 441,41 | 442 | 2,22 (2) | Beispiel 5A | 3 | 12 (27) |
| **36** | 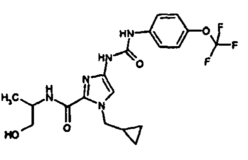 | 441,41 | 442 | 2,28 (2) | Beispiel 5A | 3 | 12 (27) |
| **37** | 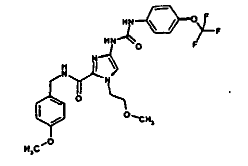 | 507,47 | 508 | 2,54 (2) | Beispiel 9A | 3 | 28 (55) |
| **38** | 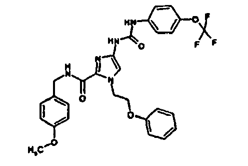 | 569,54 | 570 | 2,90 (1) | Beispiel 15A | 3 | 28 (49) |
| **39** | 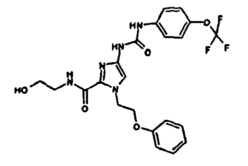 | 493,44 | 494 | 2,38 (2) | Beispiel 15A | 3 | 23 (47) |
| **40** | 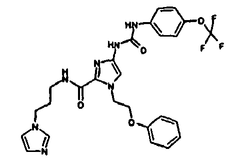 | 557,53 | 558 | 2,07 (2) | Beispiel 15A | 3 | 25 (45) |
| **41** | 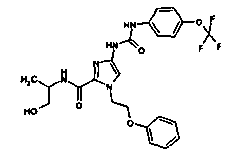 | 507,47 | 508 | 2,45 (2) | Beispiel 15A | 3 | 26 (51) |
| **42** | 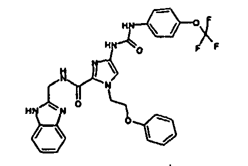 | 579,54 | 580 | 2,22 (1) | Beispiel 15A | 3 | 33 (57) |
| **43** | 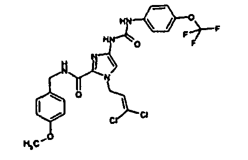 | 558,34 | 558 | 2,97 (1) | Beispiel 17A | 3 | 12 (21) |
| **44** | 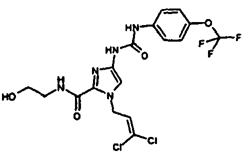 | 482,24 | 482 | 2,38 (2) | Beispiel 17A | 3 | 22 (46) |
| **45** | 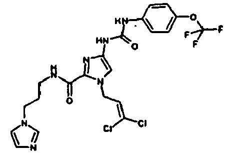 | 546,34 | 546 | 2,09 (2) | Beispiel 17A | 3 | 14 (26) |
| **46** | 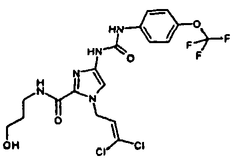 | 496,27 | 496 | 2.41 (2) | Beispiel 17A | 3 | 11 (22) |
| **47** | 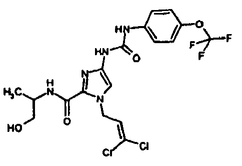 | 496,27 | 496 | 2,46 (2) | Beispiel 17A | 3 | 13 (26) |
| **48** | 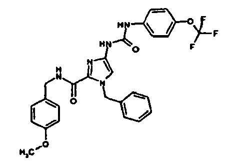 | 539,51 | 540 | 2,87 (1) | Beispiel 11A | 3 | 21 (39) |
| **49** | 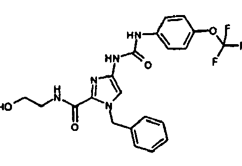 | 463,41 | 464 | 2,32 (2) | Beispiel 11A | 3 | 21 (45) |
| **50** | 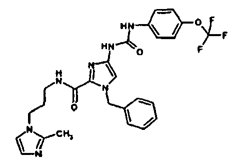 | 541,53 | 542 | 2,10 (2) | Beispiel 11A | 3 | 14 (26) |
| **51** | 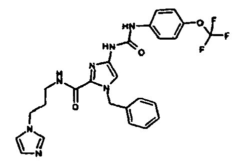 | 527,51 | 528 | 2,07 (2) | Beispiel 11A | 3 | 20 (38) |
| **52** | 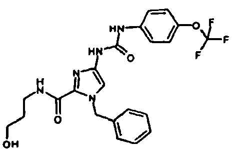 | 477,44 | 478 | 2,35 (2) | Beispiel 11A | 3 | 21 (44) |
| **53** | 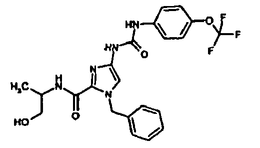 | 477,44 | 478 | 2,40 (2) | Beispiel 11A | 3 | 25 (52) |
| **54** | 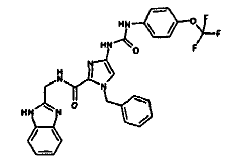 | 549,51 | 550 | 2,25 (2) | Beispiel 11A | 3 | 34 (62) |
| **55** | 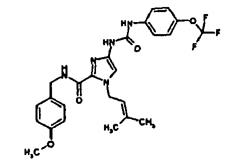 | 517,51 | 518 | 2,93 (1) | Beispiel 13A | 3 | 27 (52) |
| **56** | 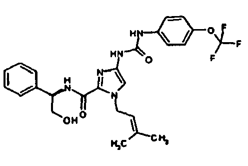 | 517,51 | 518 | 2,68 (2) | Beispiel 13A | 3 | 30 (58) |
| **57** | 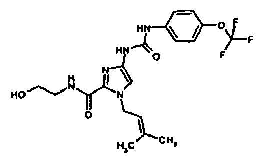 | 441,41 | 442 | 2.34 (2) | Beispiel 13A | 3 | 12 (27) |
| **58** | 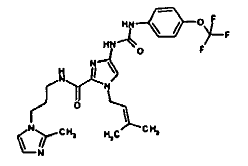 | 519,53 | 520 | 2,09 (2) | Beispiel 13A | 3 | 22 (42) |
| **59** | 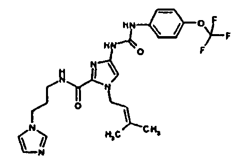 | 505,50 | 506 | 1,99 (2) | Beispiel 13A | 3 | 34 (67) |
| **60** | 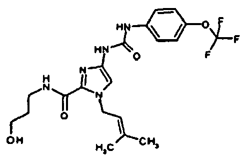 | 455,44 | 456 | 2,40 (1) | Beispiel 13A | 3 | 38 (83) |
| **61** | 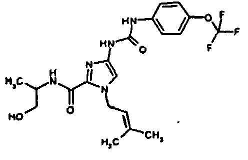 | 455,44 | 456 | 2,46 (1) | Beispiel 13A | 3 | 32 (70) |
| **62** | 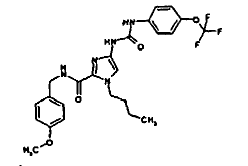 | 505,49 | 506 | 2,88 (1) | Beispiel 7A | 3 | 23 (46) |
| **63** | 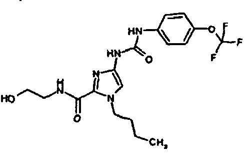 | 429,40 | 430 | 2,32 (1) | Beispiel 7A | 3 | 24 (56) |
| **64** | 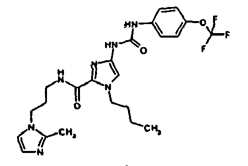 | 507,51 | 508 | 1,95 (1) | Beispiel 7A | 3 | 23 (45) |
| **65** | 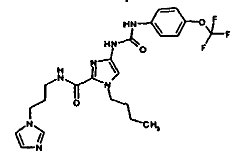 | 493,49 | 494 | 1,91 (1) | Beispiel 7A | 3 | 27 (55) |
| **66** | 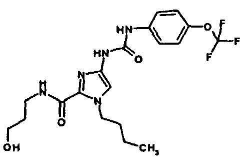 | 443,42 | 444 | 2,35 (1) | Beispiel 7A | 3 | 20 (45) |
| **67** | 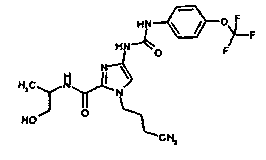 | 443,42 | 444 | 2,41 (1) | Beispiel 7A | 3 | 28 (63) |
| **68** | 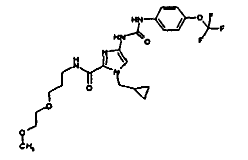 | 499,49 | 500 | 2,49 (1) | Beispiel 5A | 3 | 13 (26) |
| **69** | 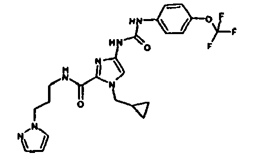 | 491,47 | 492 | 2,49 (1) | Beispiel 5A | 3 | 14 (28) |
| **70** | 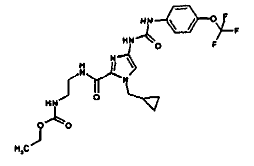 | 498,46 | 499 | 2,46 (1) | Beispiel 5A | 3 | 11 (22) |
| **71** | 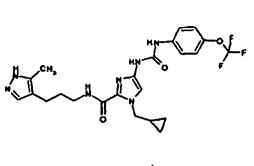 | 505,50 | 506 | 2,34 | Beispiel 5A | 3 | 16 (32) |
| **72** | 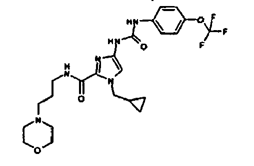 | 510,51 | 511 | 1,84 (1) | Beispiel 5A | 3 | 13 (25) |
| **73** | 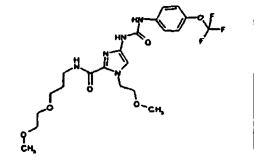 | 503,48 | 504 | 2,26 (1) | Beispiel 9A | 3 | 31 (62) |
| **74** | 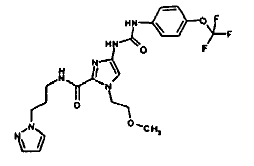 | 495,46 | 496 | 2,28 (1) | Beispiel 9A | 3 | 30 (61) |
| **75** | 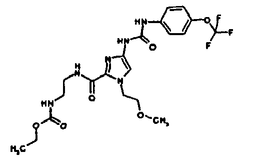 | 502,45 | 503 | 2,25 (1) | Beispiel 9A | 3 | 22 (44) |
| **76** | 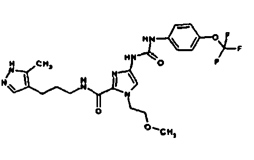 | 509,49 | 510 | 2,12 (1) | Beispiel 9A | 3 | 27 (53) |
| **77** | 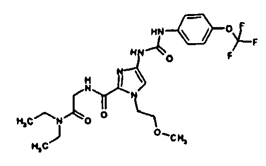 | 500,48 | 501 | 2,38 (1) | Beispiel 9A | 3 | 21 (42) |
| **78** | 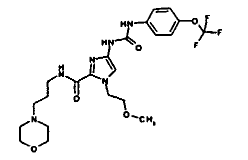 | 514,50 | 515 | 1,67 (1) | Beispiel 9A | 3 | 34 (66) |
| **79** | 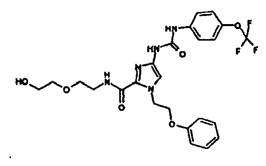 | 537,49 | 538 | 2,42 (1) | Beispiel 15A | 3 | 30 (56) |
| **80** | 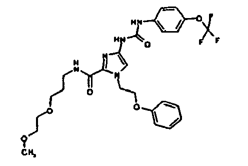 | 565,55 | 566 | 2,65 (1) | Beispiel 15A | 3 | 11 (19) |
| **81** | 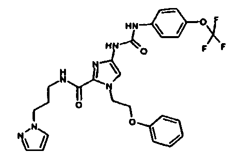 | 557,53 | 558 | 2,64 (1) | Beispiel 15A | 3 | 3,5 (6) |
| **82** | 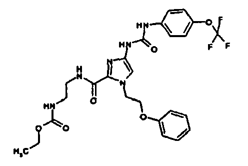 | 564,52 | 565 | 2,62 (1) | Beispiel 15A | 3 | 5,2 (9) |
| **83** | 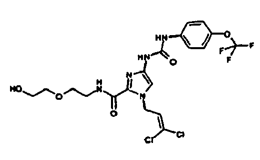 | 526,30 | 526 | 2,43 (1) | Beispiel 17A | 3 | 23 (44) |
| **84** | 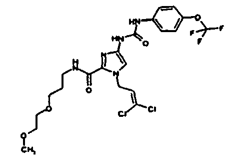 | 554,35 | 554 | 2,69 (1) | Beispiel 17A | 3 | 22 (40) |
| **85** | 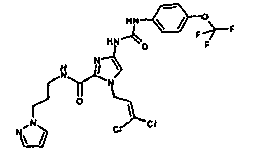 | 546,34 | 546 | 2,67 (1) | Beispiel 17A | 3 | 18 (33) |
| **86** | 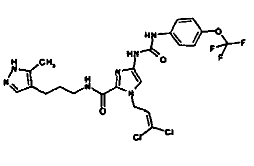 | 560,36 | 560 | 2,53 | Beispiel 17A | 3 | 29 (52) |
| **87** | 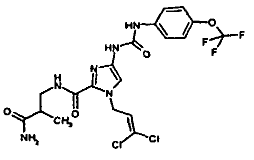 | 523,30 | 523 | 2,40 (1) | Beispiel 17A | 3 | 14 (27) |
| **88** | 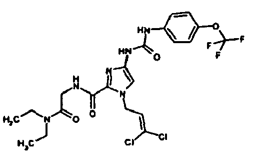 | 551,35 | 551 | 2,79 (1) | Beispiel 17A | 3 | 26 (47) |
| **89** | 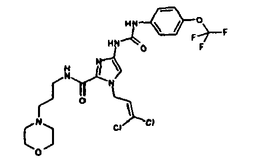 | 565,38 | 565 | 1.96 (1) | Beispiel 17A | 3 | 22 (39) |
| **90** | 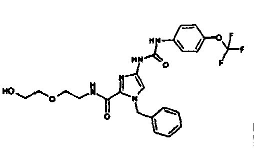 | 507,47 | 508 | 2,37 (1) | Beispiel 11A | 3 | 19 (37) |
| **91** | 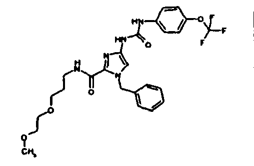 | 535,52 | 536 | 2,61 (1) | Beispiel 11A | 3 | 22 (41) |
| **92** | 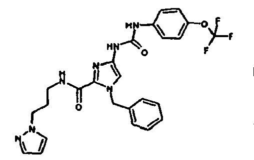 | 527,51 | 528 | 2,60 (1) | Beispiel 11A | 3 | 10 (19) |
| **93** | 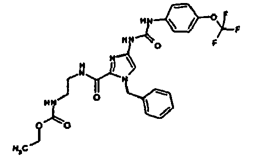 | 534,49 | 535 | 2,57 (1) | Beispiel 11A | 3 | 15 (28) |
| **94** | 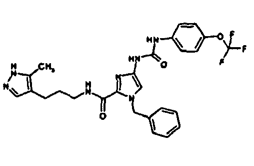 | 541,53 | 542 | 2,46 (1) | Beispiel 11A | 3 | 23 (42) |
| **95** | 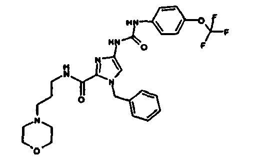 | 546,55 | 547 | 1,94 (1) | Beispiel 11A | 3 | 14 (26) |
| **96** | 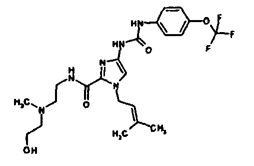 | 498,50 | 499 | 1,90 | Beispiel 13A | 3 | 19 (38) |
| **97** | 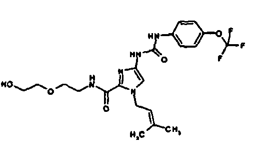 | 485,46 | 486 | 2,39 (1) | Beispiel 13A | 3 | 14 (29) |
| **98** | 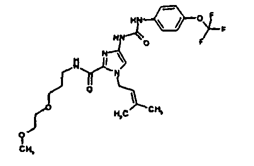 | 513,51 | 514 | 2,64 (1) | Beispiel 13A | 3 | 31 (60) |
| **99** | 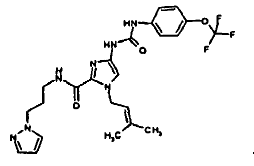 | 505,50 | 506 | 2,64 (1) | Beispiel 13A | 3 | 27 (53) |
| **100** | 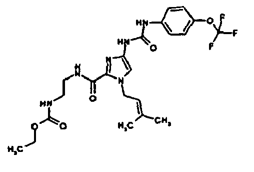 | 512,49 | 513 | 2,60 (1) | Beispiel 13A | 3 | 10 (20) |
| **101** | 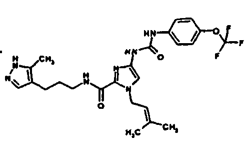 | 519,53 | 520 | 2,49 (1) | Beispiel 13A | 3 | 14 (27) |
| **102** | 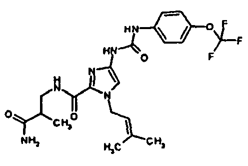 | 482,46 | 483 | 2,37 (1) | Beispiel 13A | 3 | 5 (10) |
| **103** | 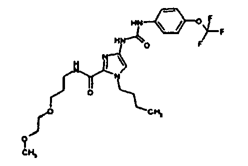 | 501,50 | 502 | 2,59 (1) | Beispiel 7A | 3 | 32 (64) |
| **104** | 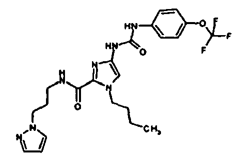 | 493,49 | 494 | 2,59 (1) | Beispiel 7A | 3 | 27 (55) |
| **105** | 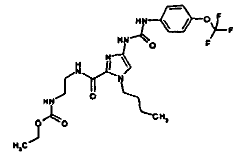 | 500,48 | 501 | 2,55 (1) | Beispiel 7A | 3 | 15 (30) |
| **106** | 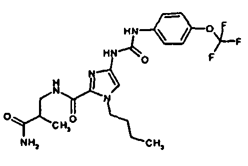 | 470,45 | 471 | 2,32 (1) | Beispiel 7A | 3 | 19 (40) |
| **107** | 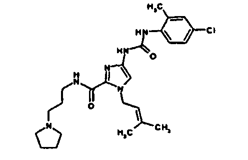 | 473,02 | 473 | 2,00 (5) | Beispiel 12A | 3 | 10 (21) |
| **108** | 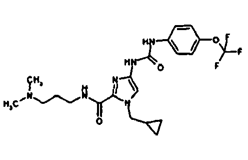 | 468,48 | 469 | 1,95 (5) | Beispiel 5A | 3 | 4,1 (9) |
| **109** | 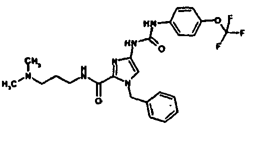 | 504,51 | 505 | 2,03 (5) | Beispiel 11A | 3 | 16 (32) |
| **110** | 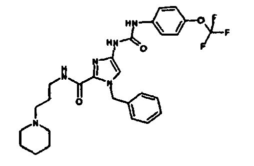 | 544,58 | 545 | 2,09 (5) | Beispiel 11A | 3 | 17 (31) |
| **111** | 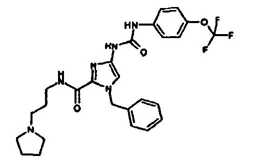 | 530,55 | 531 | 2,11 (5) | Beispiel 11A | 3 | 8 (15) |
| **112** | 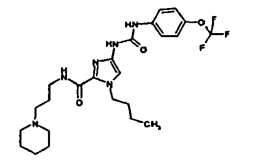 | 510,56 | 511 | 2,05 (5) | Beispiel 7A | 3 | 18 (35) |
| **113** | 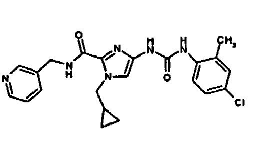 | 438,92 | 439 | 1,97 (5) | Beispiel 4A | 3 | 17,1 (85) |
| **114** | 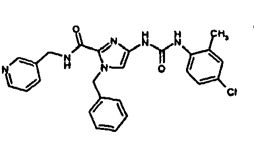 | 474,95 | 475 | 2,09 (1) | Beispiel 10A | 3 | 19,5 (41) |
| **115** | 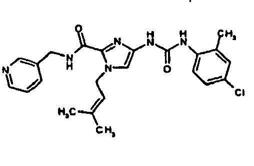 | 452,94 | 453 | 2,15 (5) | Beispiel 12A | 3 | 23,6 (52) |
| **116** | 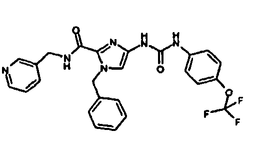 | 510,47 | 511 | 2,02 (4) | Beispiel 11A | 3 | 32,1 (63) |
| **117** | 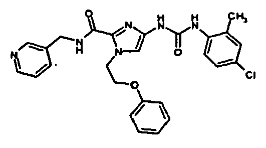 | 504,98 | 505 | 2,18 (5) | Beispiel 14A | 3 | 24 (48) |
| **118** | 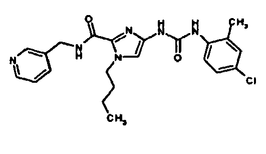 | 440,93 | 441 | 1,87 (4) | Beispiel 6A | 3 | 26,5 (60) |
| **119** | 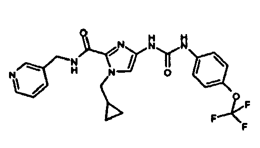 | 474,44 | 475 | 2,03 (2) | Beispiel 5A | 3 | 11 (20) |
| **120** | 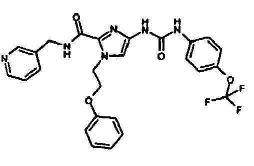 | 540,50 | 541 | 2,28 (5) | Beispiel 15A | 3 | 4,2 (8) |
| **121** | 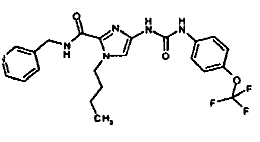 | 476,46 | 477 | 2,15 (5) | Beispiel 7A | 3 | 10,3 (22) |

### Beispiel 122

4-[({[4-(Trifluormethoxy)phenyl]amino}carbonyl)amino]-1-[4-(trifluormethyl)benzyl]-1H-imidazol-2-carbonsäureethylester 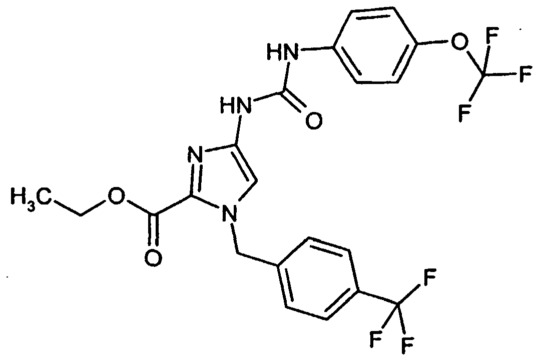 Herstellung analog zu Beispiel 4A Stufe 3.
Ausbeute: 19.8 mg (38 % d. Th.)
LC-MS (Methode 2): Rₜ = 2.89 min.
MS (ESI⁺): m/z = 517 [M+H]⁺
¹H-NMR (300MHz, DMSO-d₆): δ = 9.1 (s, 1H), 8.95 (s, 1H), 7.75 (d, 2H), 7.55 (d, 2H), 7.5 (s, 1H), 7.4 (d, 2H), 7.25 (d, 2H), 5.7 (s, 2H), 4.25 (q, 2H), 1.35 (tr, 3H) ppm.

### Beispiel 123

4-({[(4-Chlor-2-methylphenyl)amino]carbonyl}amino)-N-(2-hydroxyethyl)-1-[4-(trifluormethyl)-benzyl]-1H-imidazol-2-carboxamid 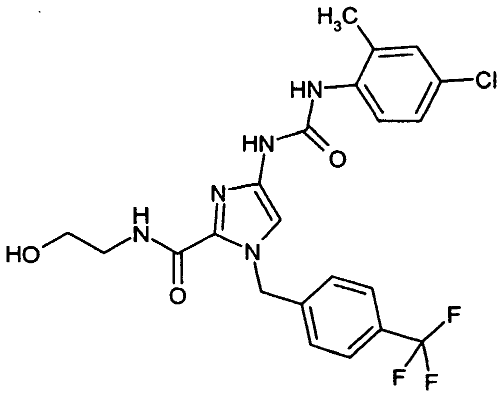 1.81 g (4 mmol) 4-({[(4-Chlor-2-methylphenyl)amino]carbonyl}amino)-1-[4-(trifluormethyl)-benzyl]-1H-imidazol-2-carbonsäure (Beispiel 20A) werden in 16 ml absolutem DMF gelöst und mit 1.94 g (12 mmol) N,N-Carbonyldiimidazol versetzt. Die Reaktionsmischung wird 1.5 h bei RT gerührt und dann mit 144 µl Wasser versetzt und 10 min bei RT gerührt. Dann werden 366 mg (6 mmol) 2-Aminoethanol zugegeben und 1 h bei RT gerührt. Die Reaktionsmischung wird mit gesättigter Natriumchloridlösung versetzt und zweimal mit Ethylacetat extrahiert. Die vereinten Extrakte werden je einmal mit 10%iger Zitronensäure, gesättigter Natriumhydrogencarbonatlösung, Wasser und gesättigter Natriumchloridlösung gewaschen, mit Magnesiumsulfat getrocknet und im Vakuum eingeengt. Der Eindampfrückstand wird durch Chromatographie an Kieselgel mit Dichlormethan/Methanol als Elutionsmittel gereinigt.
Ausbeute: 0.9 g (45.8 % d. Th.)
LC-MS (Methode 2): Rₜ = 2.48 min.
MS (ESI⁺): m/z = 496 [M+H]⁺
¹H-NMR (300MHz, DMSO-d₆): δ = 9.25 (s, 1H), 8.3 (s breit, 1H), 8.0 (tr, 1H), 7.9 (d, 1H), 7.7 (d, 2H), 7.45 (d, 2H), 7.3 (s, 1H), 7.25 (d, 1H), 7.15 (dd, 1H), 5.75 (s, 2H), 4.75 (tr, 1H), 3.5 (q, 2H), 3.3 (q, 2H), 2.25 (s, 3H) ppm.

### Beispiel 124

N-(2-Hydroxyethyl)-4-[({[4-(trifluormethoxy)phenyl]amino}carbonyl)amino]-1-[4-(trifluormethyl)benzyl]-1H-imidazol-2-carboxamid 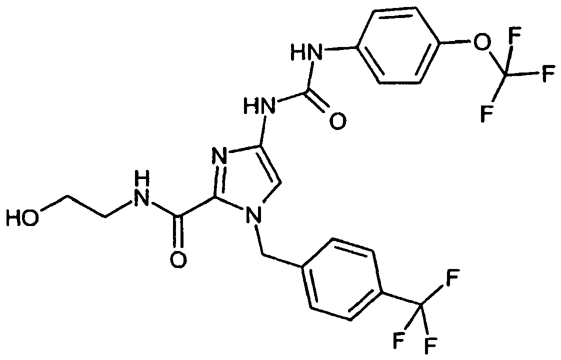 Die Herstellung erfolgt analog zu Beispiel 123 aus 4-[({[4-(Trifluormethoxy)phenyl]-amino}carbonyl)amino]-1-[4-(trifluormethyl)benzyl]-1H-imidazol-2-carbonsäure (Beispiel 19A), der Eindampfrückstand wurde durch Umkristallisation aus Dichlormethan gereinigt.
Ausbeute: 1.66 g (78 % d. Th.)
LC-MS (Methode 2): Rₜ = 2.57 min.
MS (ESI⁺): m/z = 532 [M+H]⁺
¹H-NMR (300MHz, DMSO-d₆): δ = 9.05 (s, 1H), 8.8 (s, 1H), 8.05 (tr, 1H), 7.7 (d, 2H), 7.55 (d, 2H), 7.45 (s, 2H), 7.35 (d, 1H), 7.25 (d, 2H), 5.75 (s, 2H), 4.75 (tr, 1H), 3.5 (q, 2H), 3.3 (q, 2H) ppm.

### Beispiel 125

2-Hydroxyethyl-4-({[(4-chlor-2-methylphenyl)amino]carbonyl}amino)-1-[4-(trifluormethyl)-benzyl]-1H-imidazol-2-carboxylat 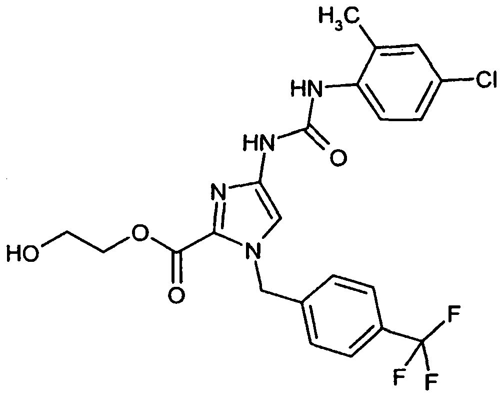 1.36 g (3 mmol) 4-({[(4-Chlor-2-methylphenyl)amino]carbonyl}amino)-1-[4-(trifluormethyl)-benzyl]-1H-imidazol-2-carbonsäure (Beispiel 20A) werden in 6 ml absolutem DMF gelöst und mit 0.58 g (3.6 mmol) N,N-Carbonyldiimidazol versetzt. Die Reaktionsmischung wird 3 h bei RT gerührt. Dann werden 18.4 g (16.5 ml, 297 mmol) 1,2-Ethandiol und 303 mg (418 µl, 3 mmol) Triethylamin zugegeben. Die Reaktionsmischung wird 1.5 h bei 50°C und über Nacht bei RT gerührt. Nach Verdünnen mit Wasser wird zweimal mit Ethylacetat extrahiert. Die vereinten Extrakte werden zweimal mit gesättigter Natriumhydrogencarbonatlösung, einmal mit Wasser und zweimal mit gesättigter Natriumchloridlösung gewaschen, über Magnesiumsulfat getrocknet und im Vakuum eingeengt. Der Eindampfrückstand wird durch Chromatographie an Kieselgel mit Cyclohexan/Ethylacetat als Elutionsmittel gereinigt.
Ausbeute: 0.95 g (64 % d. Th.)
LC-MS (Methode 2): Rₜ = 2.60 min.
MS (ESI⁺): m/z = 497 [M+H]^{+ 1}H-NMR (300MHz, DMSO-d₆): δ = 9. 5 (s, 1H), 8.6 (s breit, 1H), 7.95 (d, 1H), 7.7 (d, 2H), 7.4 (d, 2H), 7.4 (s, 1H), 7.25 (d, 1H), 7.15 (dd, 1H), 5.7 (s, 2H), 4.85 (tr, 1H), 4.25 (tr, 2H), 3.65 (q, 2H), 2.3 (s, 3H) ppm.

### Beispiel 126

2-Hydroxyethyl-4-[({[4-(trifluormethoxy)phenyl]amino}carbonyl)amino]-1-[4-(trifluormethyl)-benzyl]-1H-imidazol-2-carboxylat 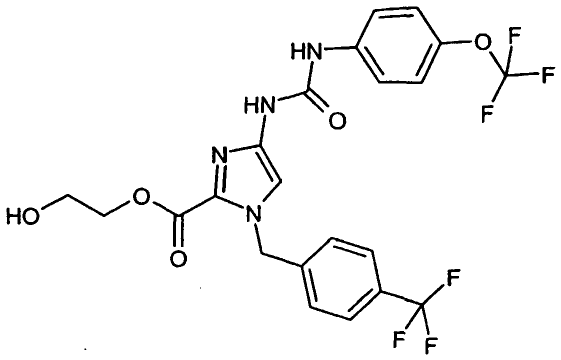 Die Herstellung erfolgt analog zu Beispiel 125 aus 4-[({[4-(Trifluormethoxy)phenyl]-amino}carbonyl)amino]-1-[4-(trifluormethyl)benzyl]-1H-imidazol-2-carbonsäure (Beispiel 19A), der Eindampfrückstand wird durch Chromatographie an Kieselgel mit Dichlormethan/Methanol als Elutionsmittel gereinigt. Ausbeute: 760 mg (48 % d. Th.)
LC-MS (Methode 4): Rₜ = 2.64 min.
MS (ESI⁺): m/z = 533 [M+H]⁺
¹H-NMR (300MHz, DMSO-d₆): δ = 9.05 (s, 1H), 8.95 (s, 1H), 7.75 (d, 2H), 7.55 (d, 2H), 7.5 (s, 1H), 7.4 (s, 2H), 7.25 (d, 2H), 5.7 (s, 2H), 4.85 (tr, 1H), 4.2 (tr, 2H), 3.5 (q, 2H) ppm.

### Beispiel 127

4-({[(4-Chlor-2-methylphenyl)amino]carbonyl}amino)-N-[2-(dimethylamino)ethyl]-1-[4-(trifluormethyl)benzyl]-1H-imidazol-2-carboxamid 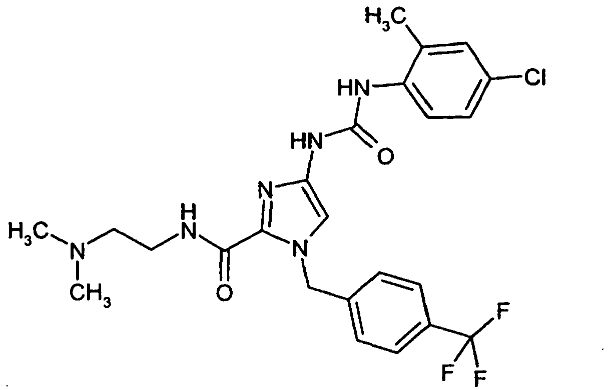 25.7 mg (0.05 mmol) N-(2-Chlorethyl)-4-({[(4-chlor-2-methylphenyl)amino]carbonyl}amino)-1-[4-(trifluormethyl)benzyl]-1H-imidazol-2-carboxamid (Beispiel 21A) werden in 200 µl einer 2 molaren Lösung von Dimethylamin in THF gelöst und mit 7.5 mg (0.05 mmol) Natriumiodid versetzt. Die Reaktionsmischung wird in einem fest verschlossenen Reaktionsgefäß 16 h bei 55°C gerührt. Die Reaktionslösung wird dann im Vakuum eingedampft, der Eindampfrückstand in DMSO gelöst, filtriert und durch präparative HPLC gereinigt (Methode 10).
Ausbeute: 10.4 mg (40 % d. Th.)
LC-MS (Methode 4): Rₜ = 1.90 min.
MS (ESI⁺): m/z = 523 [M+H]⁺
¹H-NMR (300MHz, DMSO-d₆): δ = 9.4 (s, 1H), 8.2 (s breit, 1H), 7.9 (s breit, 1H), 7.9 (d, 1H), 7.7 (d, 2H), 7.45 (d, 2H), 7.3 (s, 1H), 7.25 (s, 1H), 7.15 (d, 1H), 5.75 (s, 2H), 2.4 (tr, 2H), 2.25 (s, 3H), 2.2 (s, 6H) ppm.

### Beispiel 128

4-({[(4-Chlor-2-methylphenyl)amino]carbonyl}amino)-N-[2-(cyclopropylamino)ethyl]-1-[4-(trifluormethyl)benzyl]-1H-imidazol-2-carboxamid 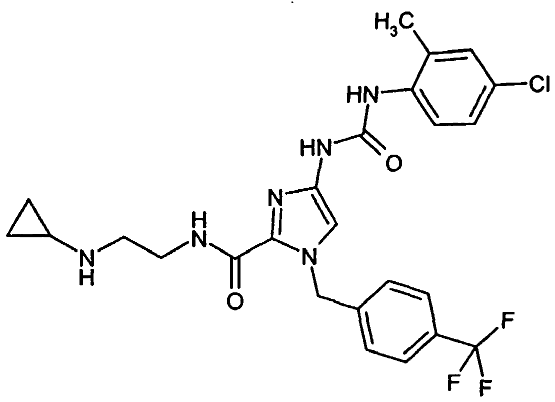 51.4 mg (0.1 mmol) N-(2-Chlorethyl)-4-({[(4-chlor-2-methylphenyl)amino]carbonyl}amino)-1-[4-(trifluormethyl)benzyl]-1H-imidazol-2-carboxamid (Beispiel 21A) werden zusammen mit 57.1 mg Cyclopropylamin in 0.4 ml Dimethoxyethan gelöst und mit 15 mg (0.1 mmol) Natriumiodid versetzt. Die Reaktionsmischung wird 16 h unter Rückfluss erhitzt. Nach Abkühlen wird DMSO zugegeben, filtriert und durch präparative HPLC gereinigt (Methode 10).
Ausbeute: 8.5 mg (16 % d. Th.)
LC-MS (Methode 4): Rₜ = 1.92 min.
MS (ESI⁺): m/z = 535 [M+H]⁺
¹H-NMR (300MHz, DMSO-d₆): δ = 9.3 (s, 1H), 8.35 (s breit, 1H), 8.05 (tr, 1H), 7.9 (d, 1H), 7.75 (d, 2H), 7.45 (d, 2H), 7.3 (s, 1H), 7.25 (s, 1H), 7.15 (d, 1H), 5.75 (s, 2H), 2.7 (tr, 2H), 2.25 (s, 3H), 2.05 (m, 1H), 0.35 (m, 2H), 0.2 (m, 2H) ppm.
Analog zu dem Beispiel 128 werden die in der Tabelle 2 aufgeführten Beispiele 129 bis 132 aus der Verbindung aus Beispiel 22A hergestellt.

**Tabelle 2:**

| Bsp.-Nr. | Struktur | Molmasse | MS (EI) [M+H]⁺ | LC-MS Rₜ [min] (Methode) | Ausbeute [mg] (% d. Th.) |
|---|---|---|---|---|---|
| 129 | 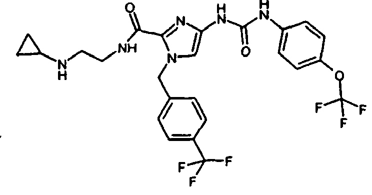 | 570.49 | 571 | 2.24 (2) | 10 (18) |
| | 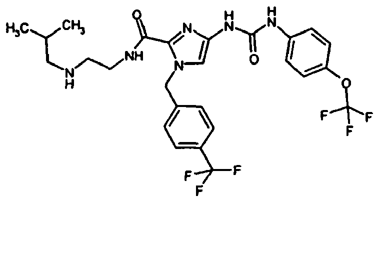 | | | | |
| 130 | | 586.53 | 587 | 2.32 | 26 |
| | | | | (2) | (44) |
| | | | | | |
| | 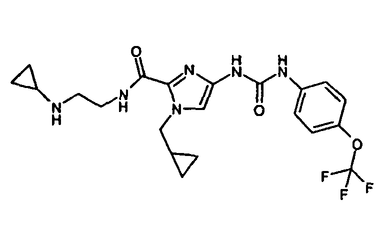 | | | | |
| 131 | | 466.46 | 467 | 1.97 | 4.2 |
| | | | | (2) | (9) |
| | | | | | |
| | 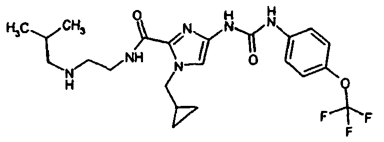 | | | | |
| 132 | | 482.50 | 483 | 2.06 | 15 |
| | | | | (2) | (31) |

### Beispiel 133

2-[({4-({[(4-Chlor-2-methylphenyl)amino]carbonyl}amino)-1-[4-(trifluormethyl)benzyl]-1H-imidazol-2-yl}carbonyl)amino]ethyl-N,N-dimethylglycinat 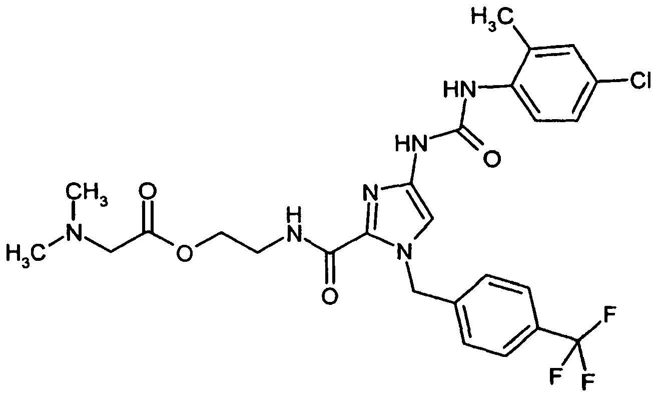 114.5 mg (0.2 mmol) 2-[({4-({[(4-Chlor-2-methylphenyl)amino]carbonyl}amino)-1-[4-(trifluormethyl)benzyl]-1H-imidazol-2-yl}carbonyl)amino]ethylchloracetat (Beispiel 26A) werden in 2 ml einer 2 molaren Lösung von Dimethylamin in THF gelöst. Die Reaktionsmischung wird in einem fest verschlossenen Reaktionsgefäß 16 h bei RT geschüttelt und dann 64 h bei RT stehen gelassen. Die Reaktionslösung wird dann eingedampft, der Eindampfrückstand in DMF gelöst, filtriert und durch präparative HPLC gereinigt (Methode 9).
Ausbeute: 16 mg (14 % d. Th.)
LC-MS (Methode 2): Rₜ = 2.18 min.
MS (ESI⁺): m/z= 581 [M+H]⁺
¹H-NMR (300MHz, DMSO-d₆): δ = 9.2 (s, 1H), 8.4 (s breit, 1H), 8.25 (tr, 1H), 7.9 (d, 1H), 7.7 (d, 2H), 7.4 (d, 2H), 7.3 (s, 1H), 7.25 (s, 1H), 7.15 (d, 1H), 5.75 (s, 2H), 4.15 (tr, 2H), 3.5 (q, 2H), 3.15 (s, 2H), 2.25 (s, 9H) ppm.

### Beispiel 134

2-[({4-[({[4-(Trifluormethoxy)phenyl]amino}carbonyl)amino]-1-[4-(trifluormethyl)benzyl]-1H-imidazol-2-yl}carbonyl)amino]ethyl-N,N-dimethylglycinat 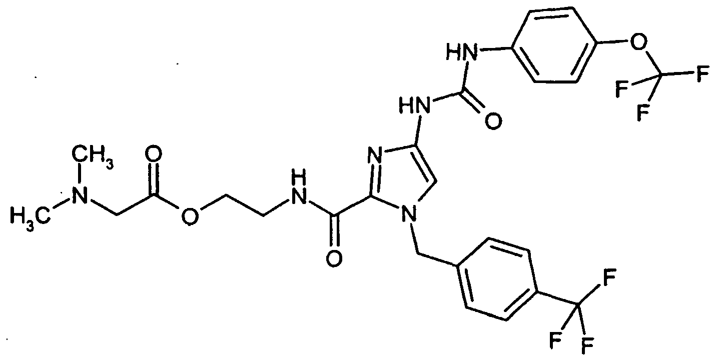 Die Herstellung erfolgt analog zu Beispiel 133 aus 2-[({4-[({[2-Methyl-4-(trifluormethoxy)-phenyl] amino}carbonyl)amino]-1-[4-(trifluormethyl)benzyl]-1H-imidazol-2-yl}carbonyl)amino]-ethylchloracetat (Beispiel 25A). Ausbeute: 14 mg (11 % d. Th.)
LC-MS (Methode 2): Rₜ = 2.27 min.
MS (ESI⁺): m/z = 617 [M+H]^{+ 1}H-NMR (300MHz, DMSO-d₆): δ = 9.1 (s, 1H), 8.7 (s, 1H), 8.3 (tr, 1H), 7.7 (d, 2H), 7.55 (d, 2H), 7.4 (d, 2H), 7.35 (s, 1H), 7.25 (s, 2H), 5.75 (s, 2H), 4.15 (tr, 2H), 3.5 (q, 2H), 3.15 (s, 2H), 2.25 (s, 9H) ppm.

### Beispiel 135

2-[({4-({[(4-Chlor-2-methylphenyl)amino]carbonyl}amino)-1-[4-(trifluormethyl)benzyl]-1H-imidazol-2-yl}carbonyl)amino]ethyl-N,N-diethylglycinat 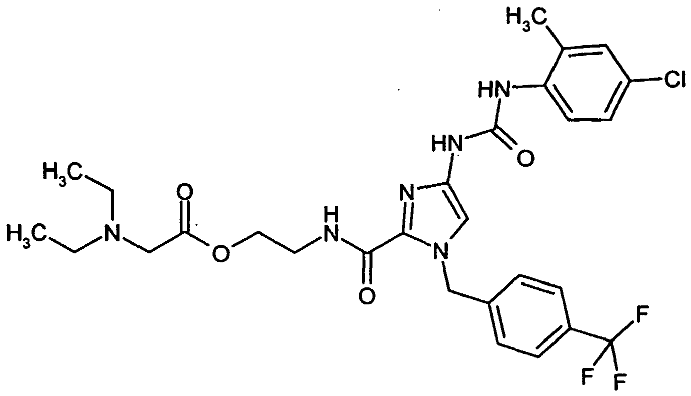 123.4 mg (0.2 mmol) 2-[({4-({[(4-Chlor-2-methylphenyl)amino]carbonyl}aminorl-[4-(trifluormethyl)benzyl]-1H-imidazol-2-yl}carbonyl)amino]ethylchloracetat (Beispiel 26A) werden in 0.8 ml absolutem DMF unter Argon gelöst und mit 29.3 (0.4 mmol) Diethylamin versetzt. Die Reaktionsmischung wird 2.5 h bei RT gerührt, filtriert und durch präparative HPLC gereinigt (Methode 9).
Ausbeute: 34.1 mg (28 % d. Th.)
LC-MS (Methode 4): Rₜ = 2.29 min.
MS (ESI⁺): m/z = 496 [M+H]⁺
¹H-NMR (300MHz, DMSO-d₆): δ = 9.25 (s, 1H), 8.3 (s breit, 1H), 8.15 (s, 1H), 7.9 (d, 1H), 7.7 (d, 2H), 7.4 (d, 2H), 7.3 (s, 1H), 7.25 (d, 1H), 7.15 (dd, 1H), 5.75 (s, 2H), 4.1 (tr, 2H), 3.5 (q, 2H), 2.5 (q, 4H), 2.25 (s, 3H), 0.9 (tr, 6H) ppm.

Analog zu dem Beispiel 135 werden die in der Tabelle 3 aufgeführten Beispiele 136 bis 140 aus der Verbindung aus Beispiel 25A hergestellt.

**Tabelle 3:**

| **Bsp.-Nr.** | **Struktur** | **Molmasse** | **MS (EI) [M+H]⁺** | **LC-MS Rₜ [min] (Methode)** | **Ausbeute [mg] (% d. Th.)** |
|---|---|---|---|---|---|
| | 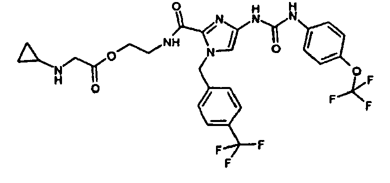 | | | | |
| 136 | | 628.52 | 629 | 2.22 | 40 |
| | | | | (2) | (32) |
| | | | | | |
| | 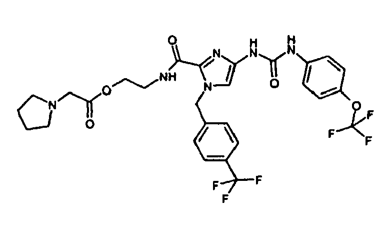 | | | | |
| 137 | | 642.55 | 643 | 2.3 | 46 |
| | | | | (2) | (36) |
| | | | | | |
| | 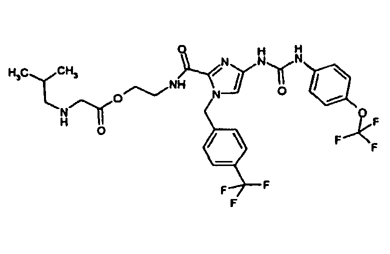 | | | | |
| 138 | | 644.57 | 645 | 2.38 | 42 |
| | | | | (2) | (33) |
| | | | | | |
| | 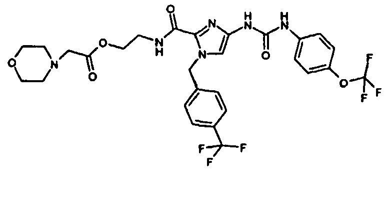 | | | | |
| 139 | | 658.55 | 659 | 2.35 | 15.2 |
| | | | | (2) | (30) |
| | | | | | |
| | 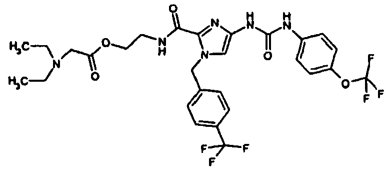 | | | | |
| 140 | | 644.57 | 645 | 2.34 | 26.6 |
| | | | | (2) | (21) |
| | | | | | |

### Beispiel 141

2-[(2-Morpholin-4-ylacetyl)oxy]ethyl-4-({[(4-chlor-2-methylphenyl)amino]carbonyl}amino)-1-[4-(trifluormethyl)benzyl]-1H-imidazol-2-carboxylat 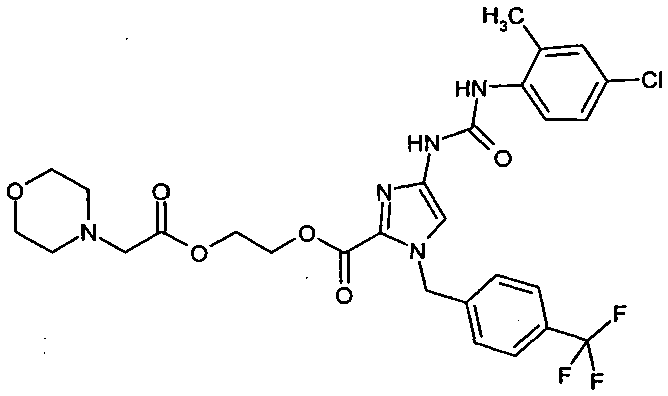 61.8 mg (0.1 mmol) 2-[(2-Bromacetyl)oxy]ethyl-4-({[(4-chlor-2-methylphenyl)amino]carbonyl}-amino)-1-[4-(trifluormethyl)benzyl]-1H-imidazol-2-carboxylat (Beispiel 24A) werden unter Argon in 0.4 ml 1,2-Dimethoxyethan gelöst und mit 15 mg (0.1 mmol) Natriumiodid und 43.6 mg (0.5 mmol) Morpholin versetzt. Die Reaktionsmischung wird 2 h unter Rückfluss erhitzt, dann mit 0.3 ml DMSO versetzt, filtriert und durch präparative HPLC gereinigt (Methode 9).
Ausbeute: 14.5 mg (23 % d. Th.)
LC-MS (Methode 2): Rₜ = 2.38 min.
MS (ESI⁺): m/z = 624 [M+H]⁺
¹H-NMR (300MHz, DMSO-d₆): δ = 9.55 (s, 1H), 8.6 (s breit, 1H), 7.95 (d, 1H), 7.7 (d, 2H), 7.45 (s, 1H), 7.4 (d, 2H), 7.25 (d, 1H), 7.15 (dd, 1H), 5.7 (s, 2H), 4.45 (m, 2H), 4.3 (m, 2H), 3.5 (tr, 4H), 3.2 (s, 2H), 2.45 (tr, 4H), 2.25 (s, 3H) ppm.

Analog zu dem Beispiel 141 werden die in der Tabelle 4 aufgeführten Beispiele 142 bis 145 hergestellt.

**Tabelle 4:**

| **Bsp.-Nr.** | **Struktur** | **Molmasse** | **MS (EI) [M+H]⁺** | **LC-MS Rₜ [min] (Methode)** | **Ausgangsverbindung Ausbeute [mg] (% d. Th.)** |
|---|---|---|---|---|---|
| | 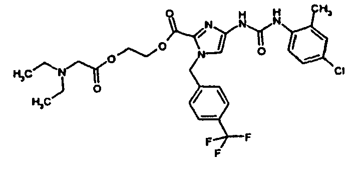 | | | | |
| | | | | | Beispiel 24A |
| 142 | | 610.03 | 611 | 2.27 | 31.9 |
| | | | | (2) | (52) |
| | 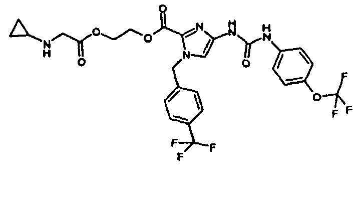 | | | | |
| | | | | | Beispiel 27A |
| 143 | | 629.51 | 630 | 2.35 | 5.7 |
| | | | | (2) | (9) |
| | | | | | |
| | 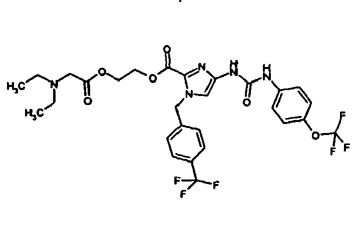 | | | | |
| | | | | | Beispiel 27A |
| 144 | | 645.55 | 646 | 2.35 | 31.9 |
| | | | | (2) | (49) |
| | | | | | |
| | 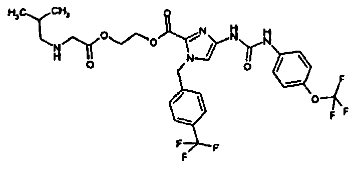 | | | | |
| | | | | | Beispiel 27A |
| 145 | | 645.55 | 646 | 2.43 | 5.4 |
| | | | | (2) | (8) |

### Beispiel 146

(4*R*)-4-Amino-5-{2-[({1-butyl-4-[({[4-(trifluormethoxy)phenyl]amino}carbonyl)amino]-1*H-*imidazol-2-yl}carbonyl)amino]ethoxy}-5-oxopentansäure Hydrochlorid 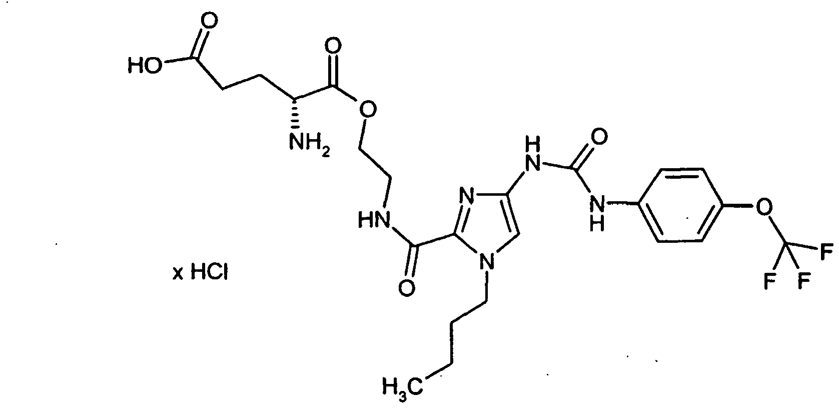 91 mg (0.3 mmol) (2R)-5-tert.-Butoxy-2-[(tert.-butoxycarbonyl)amino]-5-oxopentansäure werden in 1 ml DMF gelöst, mit 58 mg (0.3 mmol) EDCI x HCl und 37 mg (0.30 mmol) DMAP versetzt und 10 Minuten bei Raumtemperatur gerührt. Dann werden 70 mg (0.15 mmol) 1-Butyl-N-(2-hydroxyethyl)-4-[({[4-(trifluormethoxy)phenyl]amino}carbonyl)amino]-1*H*-imidazol-2-carboxamid (Beispiel 81) zugegeben und über Nacht bei Raumtemperatur gerührt. Die Reaktionsmischung wird durch präparative HPLC gereinigt. Die Produkt enthaltenden Fraktionen werden im Vakuum eingedampft.
Ausbeute: 41 mg (46 % d. Th.)
LC-MS (Methode 4): Rₜ = 1.70 min.
MS (ESI⁺): m/z = 559 [M+H]⁺
¹H-NMR (300MHz, DMSO-d₆): δ = 9.86 (s, 1H), 8.84 (s, 1H), 8.46 (d, 2H), 8.35 (t, 1H), 7.58 (d, 2H), 7.26 (m, 3H), 4.20-4.45 (m, 4H), 3.90-4.11 (m, 1H), 3.53 (q, 2H), 2.32-2.55 (m, 2H), 1.91-2.12 (m, 2H), 1.61- 1.76 (quintett, 2H), 1.18-1.34 (m, 2H), 0.89 (t, 3H) ppm.

### Beispiel 147

(4*S*)-4-Amino-5-{2-[({1-butyl-4-[({[4-(trifluormethoxy)phenyl]amino}carbonyl)amino]-1*H-*imidazol-2-yl}carbonyl)amino]ethoxy}-5-oxopentansäure Hydrochlorid 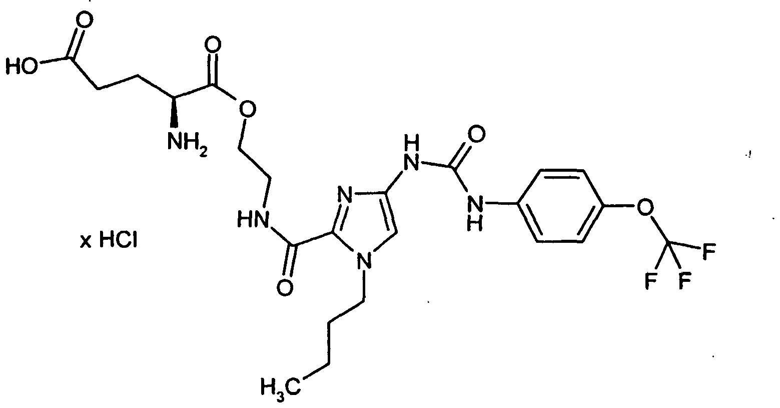 91 mg (0.30 mmol) (2*S*)-5-tert.-Butoxy-2-[(tert.-butoxycarbonyl)amino]-5-oxopentansäure werden in 5 ml DMF gelöst, mit 58 mg (0.3 mmol) EDCl x HCl und 37 mg (0.30 mmol) DMAP versetzt und 10 Minuten bei Raumtemperatur gerührt. Dann werden 70 mg (0.15 mmol) 1-Butyl-N-(2-hydroxyethyl)-4-[({([4-(trifluormethoxy)phenyl]amino}carbonyl)amino]-1*H*-imidazol-2-carboxamid (Beispiel 81) zugegeben und über Nacht bei Raumtemperatur gerührt. Die Reaktionsmischung wird durch präparative HPLC gereinigt. Die Produkt enthaltenden Fraktionen werden im Vakuum eingedampft.
Ausbeute: 35 mg (39 % d. Th.)
LC-MS (Methode 5): Rₜ = 1.97 min.
MS (ESI⁺): m/z = 559 [M+H]⁺
¹H-NMR (400MHz, DMSO-d₆): δ = 9.76 (s, 1H), 8.81 (s, 1H), 8.46 (d, 2H), 8.35 (t, 1H), 7.57 (d, 2H), 7.26 (m, 3H), 4.21-4.44 (m, 4H), 3.99-4.11 (m, 1H), 3.53 (m, 2H), 2.32-2.55 (m, 2H), 1.91-2.13 (m, 2H), 1.61- 1.76 (quintett, 2H), 1.18-1.34 (m, 2H), 0.89 (t, 3H) ppm.

Analog zu dem Beispiel 147 werden die in der Tabelle 5 aufgeführten Beispiele 148 bis 150 aus der Verbindung aus Beispiel 81 hergestellt.

**Tabelle 5:**

| **Bsp.-Nr.** | **Struktur** | **Molmasse** | **MS (EI) [M+H]⁺** | **LC-MS Rₜ [min] (Methode)** | **Ausbeute [mg] (% d. Th.)** |
|---|---|---|---|---|---|
| | 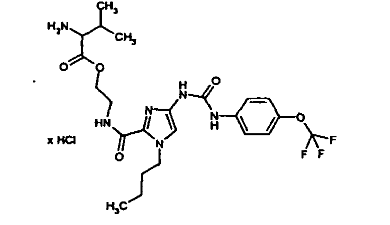 | | | | |
| 148 | | 564.99 | 529 | 1.71 | 69 |
| | | | | (4) | (73) |
| | | | | | |
| | 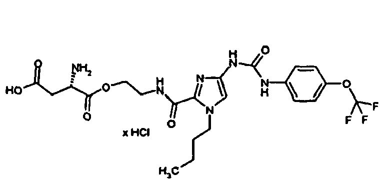 | | | | |
| 149 | | 580.95 | 545 | 1.72 | 34 |
| | | | | (4) | (39) |
| | 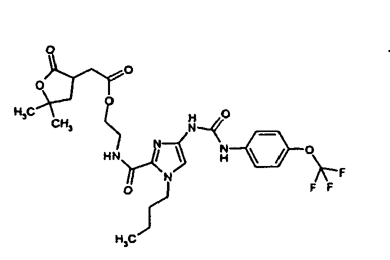 | | | | |
| 150 | | 583.56 | 584 | 2.54 | 27 |
| | | | | (4) | (31) |
| | | | | | |

### Beispiel 151

*N*-(3-Chlorpropyl)-1-methyl-4-[({[4-(trifluormethoxy)phenyl]amino}carbonyl)amino]-1*H-*imidazol-2-carboxamid 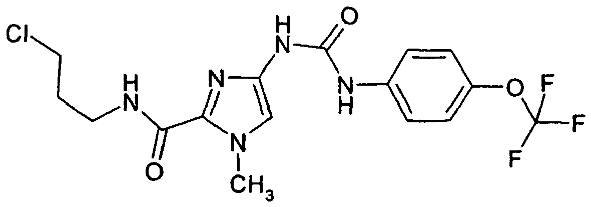 140 mg (0.36 mmol) 1-Methyl-4-[({[4-(trifluormethoxy)phenyl]amino}carbonyl)amino]-1*H-*imidazol-2-carbonsäure (Beispiel 29A) werden in 8 ml DMF gelöst, mit 204 mg (0.54 mmol) HATU und 66 mg (0.54 mmol) DMAP versetzt und 15 Minuten bei Raumtemperatur gerührt. Dann wird eine Lösung von 93 mg (0.72 mmol) 3-Chlorpropan-1-amin-hydrochlorid und 80 mg (0.79 mmol) Triethylamin in 1 ml DMF zugetropft und die Reaktionsmischung über Nacht gerührt. Die Reaktionsmischung wird durch präparative HPLC gereinigt. Die Produkt enthaltenden Fraktionen werden im Vakuum eingedampft.
Ausbeute: 144 mg (96 % d. Th.)
LC-MS (Methode 2): Rₜ = 2.43 min.
MS (ESI⁺): m/z = 420 [M+H]⁺

### Beispiel 152

1-Methyl-N-(4,4,4-trifluorbutyl)-4-[({[4-(trifluormethoxy)phenyl]amino}carbonyl)amino]-1*H-*imidazol-2-carboxamid 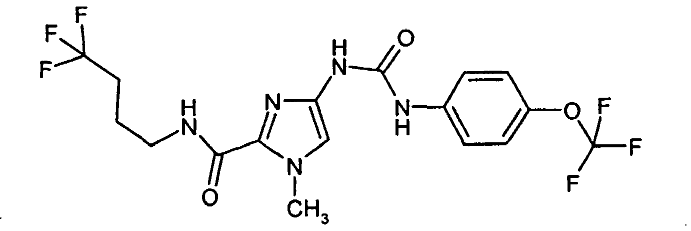 Die Herstellung erfolgt analog zu Beispiel 151.
Ausbeute: 80 mg (99 % d. Th.)
LC-MS (Methode 2): Rₜ = 2.54 min.
MS (ESI⁺): m/z = 454 [M+H]⁺

### Beispiel 153

1-Butyl-*N*-(3-chlorpropyl)-4-[({[4-(trifluormethoxy)phenyl]amino}carbonyl)amino]-1*H*-imidazol-2-carboxamid 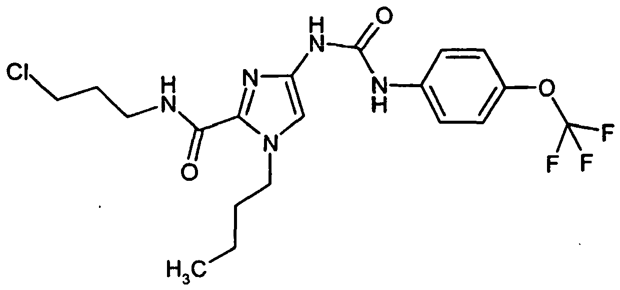 Die Herstellung erfolgt analog zu Beispiel 1.
Ausbeute: 115 mg (83 % d. Th.)
LC-MS (Methode 5): Rₜ = 2.78 min.
MS (ESI⁺): m/z = 462 [M+H]⁺

Analog zu dem Beispiel 1 werden die in der Tabelle 6 aufgeführten Beispiele 154 bis 171 aus der Verbindung aus Beispiel 7 hergestellt.

| **Tabelle 6** | | | | | | |
|---|---|---|---|---|---|---|
| **Beispiel-Nr.** | **Struktur** | **Molmasse** | **MS (EI) [M+H]⁺** | **LC-MS Retentions-zeit [min] (Methode)** | **Synthese der Amin Ausgangsverbindung** | **Ausbeute [mg] (% d. Th.)** |
| **154** | 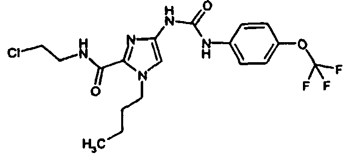 | 447,84 | 448 | 2,72 (5) | | 125 (89) |
| **155** | 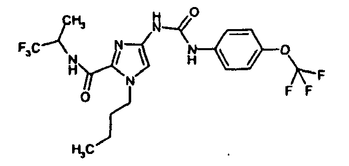 | 481,40 | 482 | 2,73 (4) | gemäß Soloshonok, Vadim A.; Ono, Taizo; J.Org.Chem.; 62; 10; 1997; 3030-3031 | 68 (93) |
| **156** | 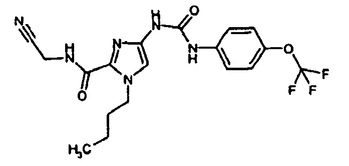 | 424,38 | 425 | 2,38 (4) | | 59 (90) |
| **157** | 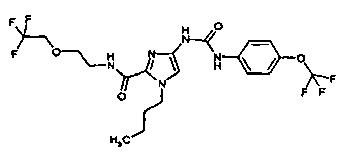 | 511,42 | 512 | 2,63 (4) | | 67 (84) |
| **158** | 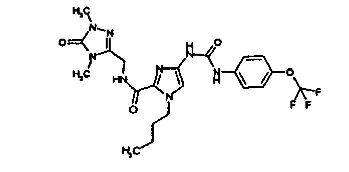 | 510,47 | 511 | 2,13 (4) | gemäß Gehlen; Blankenstein; Justus Liebigs Ann. Chem.; 651; 1962; 137,139. | 76 (99) |
| **159** | 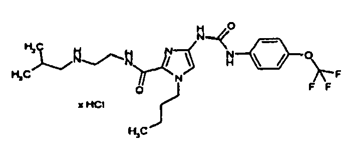 | 520,98 | 485 | 1,95 (2) | gemäß Yung,D.K. et al.; J.Pharm.Sci.; EN; 57; 1968; 2073-2080. | 76 (97) |
| **160** | 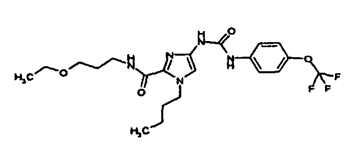 | 471,48 | 472 | 2,73 (2) | | 70 (99) |
| **161** | 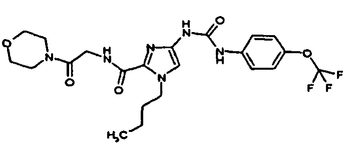 | 512,49 | 513 | 2,47 (2) | gemäß Venuti, Michael C.; Alvarez, Robert; Bruno, John J.; Strosberg. Arthur M.; Gu, Leo; J.Med.Chem.; 31; 11; 1988; 2145-2152. | 80 (quant.) |
| **162** | 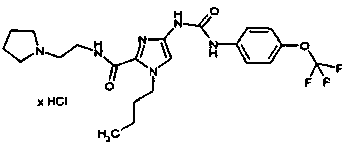 | 518,97 | 483 | 1,56 (4) | | 72 (92) |
| **163** | 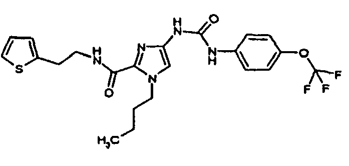 | 495,52 | 496 | 2,75 (4) | | 72 (97) |
| **164** | 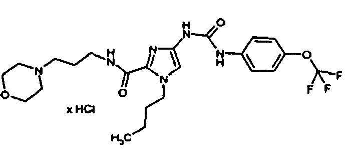 | 548,99 | 513 | 1,57 (4) | | 81 (98) |
| **165** | 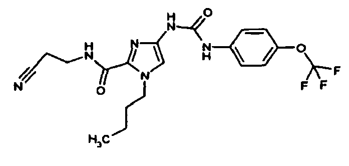 | 480,49 | 481 | 2,67 (5) | | 57 (79) |
| **166** | 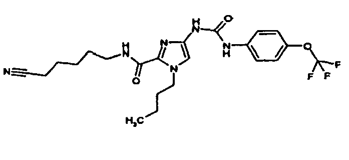 | 438,41 | 439 | 2,47 (4) | | 65 (98) |
| **167** | 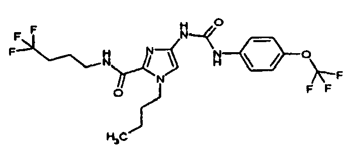 | 495,42 | 496 | 2,86 (5) | gemäß Raasch,M.S.;J.Org.C hem.; EN; 27; 1962; 1406-1409. | 65 (86) |
| **168** | 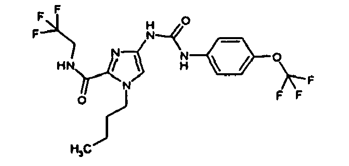 | 467,37 | 468 | 2,79 (5) | | 68 (97) |
| **169** | 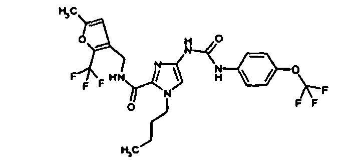 | 547,45 | 548 | 3,05 (2) | | 61 (84) |
| **170** | 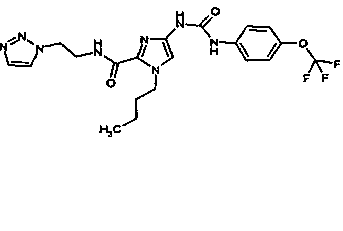 | 480,45 | 481 | 2,39 (5) | gemäß Sun, Li; Liang, Chris; Shirazian, Sheri; Zhou, Yong; Miller, Todd; Cui, Jean; Fukuda, Juri Y.; Chu, Ji-Yu; Nematalla, Asaad; Wang, Xueyan; Chen, Hui; et al. J.Med.Chem.; 46; 7; 2003; 1116 - 1119. | 61 (84) |
| **171** | 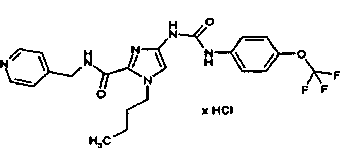 | 512,92 | 477 | 1,97 (5) | | 71 (92) |

### Beispiel 172

1-Butyl-*N*-{3-[(2,2,2-trifluorethyl)amino]propyl}-4-[({[4-(trifluormethoxy)phenyl]amino}-carbonyl)amino]-1*H*-imidazol-2-carboxamid Hydrochlorid 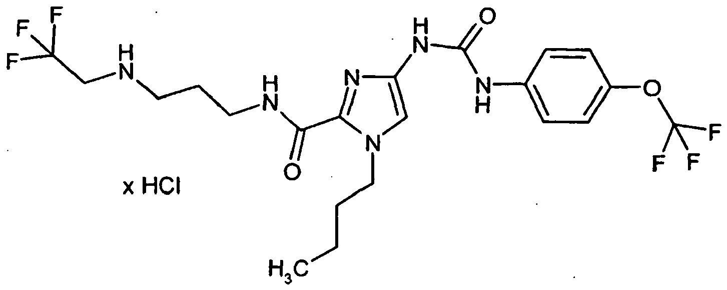 90 mg (0.19 mmol) 1-Butyl-*N*-(3-chlorpropyl)-4-[({[4-(trifluormethoxy)phenyl]amino}carbonyl)-amino]-1*H*-imidazol-2-carboxamid (Beispiel 151) werden in 3 ml 1,2-Dimethoxyethan unter Argon mit 97 mg (0.97 mmol) 2,2,2-Trifluorethylamin und 29 mg (0.19 mmol) Natriumiodid versetzt und über Nacht bei 90°C gerührt. Die Reaktionsmischung wird im Vakuum eingeengt und durch präparative HPLC gereinigt.
Ausbeute: 48 mg (40 % d. Th.)
LC-MS (Methode 2): Rₜ = 2.15 min.
MS (ESI⁺): m/z = 525 [M+H]⁺

### Beispiel 173

1-Butyl-N-[3-(1H-pyrazol-1-yl)propyl]-4-[({[4-(trifluormethyl)phenyl]amino}carbonyl)amino]-1H-imidazol-2-carbonsäureamid 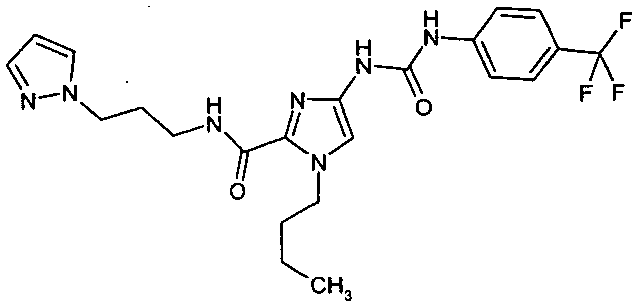 50 mg (0.14 mmol) Beispiel 30A werden in 2 ml DMF gelöst und mit 65 mg (0.2 mmol) O-(Benzotriazol-1-yl)-N,N,N',N'-tetramethyluroniumtetrafluoroborat (TBTU) und 25 mg
(0.2 mmol) 4-Dimethylaminopyridin versetzt. Nach Zugabe von 34 mg (0.27 mmol) 3-(1H-Pyrazol-1-yl)propylamin lässt man 12 h bei RT rühren. Das Reaktionsgemisch wird durch RP-HPLC gereinigt.
Ausbeute: 38 mg (59 % d. Th.)
LC-MS (Methode 4): Rₜ = 2.38 min.
MS (ESI⁺): m/z= 478 [M+H]⁺
¹H-NMR (300MHz, DMSO-d₆): δ = 9.29 (bs, 1H), 8.79 (bs, 1H), 8.28 (t, 1H), 7.75 (d, 1H), 7.67 (d, 2H), 7.62 (d, 2H), 7.44 (d, 1H), 7.26 (s, 1H), 6.23 (t, 1H), 4.39 (t, 2H), 4.15 (t, 2H), 3.21 (q, 2H), 1.99 (quint, 2H), 1.68 (quint, 2H), 1.25 (sext, 2H), 0.88 (t, 3H).

Analog zu dem Beispiel 173 werden die in der Tabelle 7 aufgeführten Beispiele hergestellt.

**Tabelle 7:**

| Bsp.-Nr. | Struktur | Molmasse | MS (ESI) [M+H]⁺ | LC-MS Rₜ [min] (Methode) | Ausgangsverbindung | Ausbeute [mg] (% d. Th.) |
|---|---|---|---|---|---|---|
| 174 | 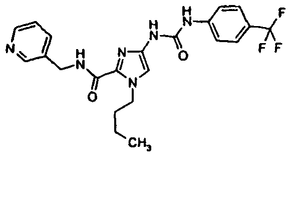 | 460.46 | 461 | 1.98 (4) | Beispiel 30A | 38 (61) |
| 175 | 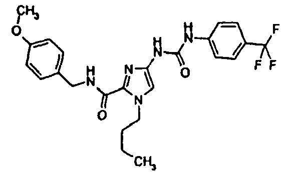 | 489.49 | 490 | 2.67 (4) | Beispiel 30A | 38 (57) |
| 176 | 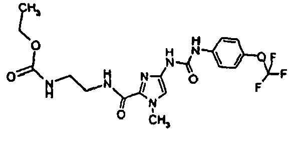 | 458.39 | 459 | 2.21 (2) | Beispiel 29A | 51 (77) |
| 177 | 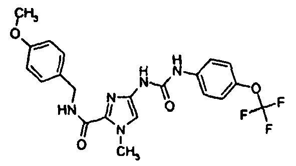 | 463.41 | 464 | 2.59 (2) | Beispiel 29A | 66 (95) |
| 178 | 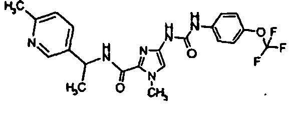 | 462.43 | 463 | 1.61 (4) | Beispiel 29A | 75 (28) |
| 179 | 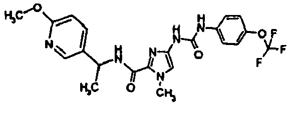 | 478.43 | 479 | 2.42 (2) | Beispiel 29A | 62 (89) |
| 180 | 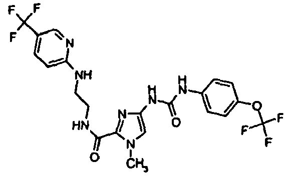 | 531.42 | 532 | 2.50 (2) | Beispiel 29A | 72 (93) |
| 181 | 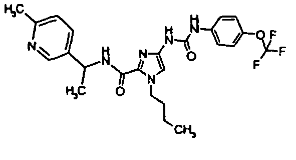 | 504.51 | 505 | 2.15 (5) | Beispiel 7A | 33 (13) |
| 182 | 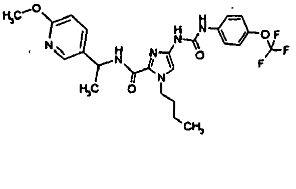 | 520.51 | 521 | 2.77 (2) | Beispiel 7A | 45 (67) |
| 183 | 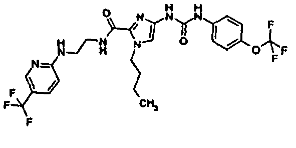 | 573.49 | 574 | 2.82 (2) | Beispiel 7A | 64 (86) |
| 184 | 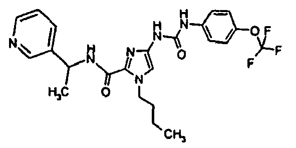 | 490.48 | 491 | 2.32 (5) | Beispiel 7A | 20 (8) |

Die Beispiele der Tabelle 8 werden analog zu Beispiel 1 hergestellt.

**Tabelle 8:**

| Bsp.-Nr. | Struktur | Molmasse | MS (ESI) [M+H]⁺ | HPLC Rₜ [min] (Methode) | Ausgangsverbindung | Ausbeute [mg] (% d. Th.) |
|---|---|---|---|---|---|---|
| 185 | 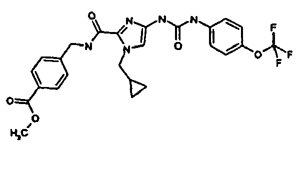 | 531.49 | 532 | 4.96 (11) | Beispiel 5A | 64 (29) |
| 186 | 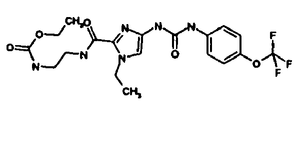 | 472.43 | 473 | 4.39 (11) | Beispiel 31A | 23 (35) |
| 187 | 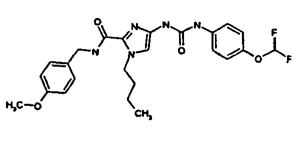 | 487.51 | 488 | 4.83 (11) | Beispiel 33A | 35 (52) |
| 188 | 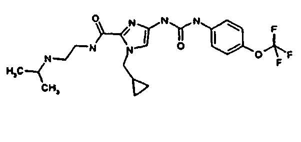 | 468.48 | 469 | 4.40 (11) | Beispiel 5A | 13 (30) |
| 189 | 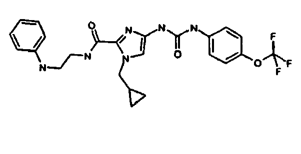 | 502.50 | 503 | 4.54 (11) | Beispiel 5A | 20 (38) |
| 190 | 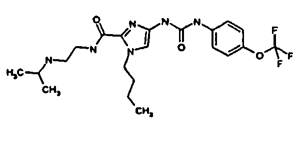 | 470.50 | 471 | 4.51 (11) | Beispiel 7A | 12 (28) |
| 191 | 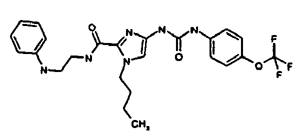 | 504.52 | 505 | 4.71 (11) | Beispiel 7A | 20 (38) |
| 192 | 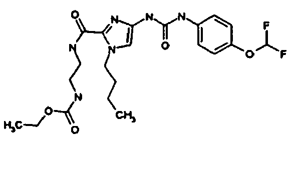 | 482.49 | 483 | 4.46 (11) | Beispiel 33A | 49 (79) |
| 193 | 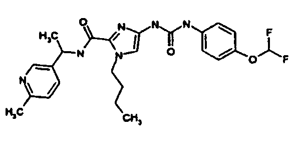 | 486.53 | 487 | 4.23 (11) | Beispiel 33A | 26 (59) |
| 194 | 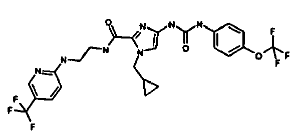 | 571.49 | 572 | 4.62 (11) | Beispiel 5A | 42 (81) |

### B. Bewertung der physiologischen Wirksamkeit

Die *in vitro*-Wirkung der erfindungsgemäßen Verbindungen kann in folgenden Assays gezeigt werden:

### Anti-HCMV- (Anti-Humanes Cvtomegalo-Virus) Zytopathogenitätstests

Die Testverbindungen werden als 50 millimolare (mM) Lösungen in Dimethysulfoxid (DMSO) eingesetzt. Ganciclovir, Foscarnet und Cidofovir dienen als Referenzverbindungen. Nach der Zugabe von jeweils 2 µl der 50, 5, 0,5 und 0,05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x 10⁴ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0,001 - 0,002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0,0005 µM. Die Platten werden 6 Tage bei 37°C / 5 % CO₂ inkubiert, d.h. bis in den Viruskontrollen alle Zellen infiziert sind (100 % cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque multiplier der Firma Technomara) visuell ausgewertet.

Die folgenden Daten können von den Testplatten ermittelt werden:
CC₅₀ (NHDF) = Substanzkonzentration in µM, bei der im Vergleich zur unbehandelten Zellkontrolle keine sichtbaren cytostatischen Effekte auf die Zellen erkennbar sind;
EC₅₀ (HCMV) = Substanzkonzentration in µM, die den CPE (cytopathischen Effekt) um 50 % im Vergleich zur unbehandelten Viruskontrolle hemmt;
SI (Selektivitätsindex) = CC₅₀ (NHDF) / EC₅₀ (HCMV).

Repräsentative in-vitro-Wirkdaten für die erfindungsgemäßen Verbindungen sind in Tabelle A wiedergegeben:

**Tabelle A**

| **Beispiel-Nr.** | **NHDF CC₅₀ [nM]** | **HCMV EC₅₀ [nM]** | **SI HCMV** |
|---|---|---|---|
| **2** | 94000 | 0.5 | 188000 |
| **3** | 25000 | 3.0 | 8333 |
| **11** | 25000 | 1.0 | 25000 |
| **25** | 6250 | 1.0 | 6250 |
| **32** | 3130 | 1.0 | 3130 |
| **124** | 9400 | 1.4 | 6714 |
| **139** | 4700 | 2.8 | 1679 |
| **152** | 31000 | 1.9 | 16316 |

Die Eignung der erfindungsgemäßen Verbindungen zur Behandlung von HCMV-Infektionen kann im folgenden Tiermodell gezeigt werden:

### HCMV Xenograft-Gelfoam^{®}-Modell

### Tiere:

3-4 Wochen alte weibliche immundefiziente Mäuse (16-18 g), Fox Chase SCID oder Fox Chase SCID-NOD oder SCID-beige werden von kommerziellen Züchtern (Bomholtgaard, Jackson) bezogen. Die Tiere werden unter sterilen Bedingungen (einschließlich Streu und Futter) in Isolatoren gehalten.

### Virusanzucht:

Humanes Cytomegalovirus (HCMV), Stamm Davis, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0,01 werden die virusinfizierten Zellen 5-7 Tage später geerntet und in Gegenwart von Minimal Essential Medium (MEM), 10% foetalem Kälberserum (FKS) mit 10 % DMSO bei -40°C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot oder Fixierung und Färbung mit einem Formalin-Giemsa Gemisch (wie oben beschrieben).

### Vorbereitung der Schwämme, Transplantation, Behandlung und Auswertung:

1x1x1 cm große Kollagenschwämme (Gelfoam^{®}; Fa. Peasel & Lorey, Best.-Nr. 407534; K.T. Chong et al., Abstracts of 39th Interscience Conference on Antimicrobial Agents and Chemotherapy, 1999, S. 439; P.M. Kraemer et al., Cancer Research 1983, (43): 4822-4827) werden zunächst mit Phosphat-gepufferter Saline (PBS) benetzt, die eingeschlossenen Luftblasen durch Entgasen entfernt und dann in MEM + 10 % FKS aufbewahrt. 1 x 10⁶ virusinfizierte NHDF-Zellen (Infektion mit HCMV-Davis M.O.I = 0.01) werden 3 Stunden nach Infektion abgelöst und in 20 µl MEM, 10 % FKS auf einen feuchten Schwamm getropft. Optional werden nach 12-13 Stunden auf die infizierten Schwämme 5 ng/µl basic Fibroblast Growth Factor (bFGF) in 25 µl PBS / 0,1% BSA / 1 mM DTT aufgebracht und 1 Stunde inkubiert. Zur Transplantation werden die immundefizienten Mäuse mit Avertin oder einem Gemisch aus Azepromazin-Xylazin und Ketamin narkotisiert, das Rückenfell mit Hilfe eines Trockenrasierers entfernt, die Oberhaut 1-2 cm geöffnet, entlastet und die feuchten Schwämme unter die Rückenhaut transplantiert. Die Operationswunde wird mit Gewebekleber verschlossen. 24 Stunden nach der Transplantation werden die Mäuse über einen Zeitraum von 8 Tagen dreimal täglich (7.00 Uhr und 14.00 Uhr und 19.00 Uhr), zweimal täglich (8.00 Uhr und 17.00 Uhr), oder einmal täglich (14.00 Uhr) peroral mit Substanz behandelt. Die Dosis beträgt 3 oder 10 oder 30 oder 100 mg/kg Körpergewicht, das Applikationsvolumen 10 ml/kg Körpergewicht. Die Formulierung der Substanzen erfolgt in Form einer 0,5%igen Tylosesuspension optional mit 2 % DMSO: 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U / 1,5 ml) aus dem Schwamm freigesetzt und in Gegenwart von MEM, 10 % foetalem Kälberserum, 10 % DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot oder nach Fixierung und Färbung mit einem Formalin-Giemsa Gemisch (wie oben beschrieben). Ermittelt wird die Anzahl infektiöser Viruspartikel nach Substanzbehandlung im Vergleich zur placebobehandelten Kontrollgruppe.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6 h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die erfindungsgemäße Verbindung wird zusammen mit Polyethylenglykol 400 in dem Wasser unter Rühren gelöst. Die Lösung wird sterilfiltriert (Porendurchmesser 0,22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 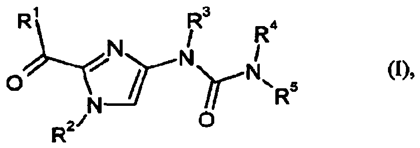 in welcher
R¹ für -OR⁶ oder -NR⁷R⁸ steht,
R² für C₁-C₆-Alkyl oder C₁-C₆-Alkenyl steht,
wobei Alkyl und Alkenyl substituiert sein können mit 1 bis 3 Substituenten, wobei die Substituenten unabhängig voneinander ausgewählt werden aus der Gruppe, bestehend aus Halogen, C₁-C₆-Alkoxy, C₃-C₈-Cycloalkyl, 5- bis 10-gliedriges Heterocyclyl mit bis zu 3 Heteroatomen aus der Reihe S, **O** und/oder N, C₆-C₁₀-Aryl Phenoxy und 5- bis 10-gliedriges Heteroaryl mit bis zu 5 Heteroatomen aus der Reihe S, **O** und/oder N,
worin Cycloalkyl, Heterocyclyl, Aryl, Phenoxy und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl, C₁-C₆-Alkylaminocarbonyl und Phenyl,
R³ und R⁴ unabhängig voneinander für Wasserstoff oder C₁-C₆-Alkyl stehen,
R⁵ für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Difluormethyl, Trifluormethoxy, Difluormethoxy, C₁-C₆-Alkyl und C₁-C₆-Alkoxy,
R⁶ für C₁-C₆-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, C₁-C₆-Alkoxy, Hydroxycarbonyl, C₁-C₆-Alkoxycarbonyl, Amino, C₁-C₆-Alkylamino, Aminocarbonyl, C₁-C₆-Alkylcarbonyloxy, C₁-C₆-Alkylcarbonylamino, C₁-C₆-Alkylaminocarbonyl, C₁-C₆-Alkoxycarbonylamino, C₃-C₈-Cycloalkyl, C₃-C₈-Cycloalkylamino, 5- bis 10-gliedriges Heterocyclyl mit bis zu 3 Heteroatomen aus der Reihe S, **O** und/oder N, 5- bis 7-gliedriges Heterocyclyl-carbonyl mit bis zu 3 Heteroatomen aus der Reihe S, **O** und/oder N, C₆-C₁₀-Aryl und 5- bis 10-gliedriges Heteroaryl mit bis zu 5 Heteroatomen aus der Reihe S, **O** und/oder N,
worin Cycloalkyl, Cycloalkylamino, Heterocyclyl, Heterocyclylcarbonyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Nitro, Cyano, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₈-Cycloalkylamino und 5- bis 7-gliedriges Heterocyclyl mit bis zu 3 Heteroatomen aus der Reihe S, O und/oder N,
worin Heterocyclyl seinerseits substituiert sein kann mit 1 bis 3 Substituenten, wobei die Substituenten unabhängig voneinander ausgewählt werden aus der Gruppe, bestehend aus C₁-C₄-Alkyl und Oxo,
R⁷ für Wasserstoff oder C₁-C₆-Alkyl steht,
und
R⁸ für C₁-C₆-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, Trifluormethyl, C₁-C₆-Alkoxy, Hydroxycarbonyl, C₁-C₆-Alkoxycarbonyl, Amino, C₁-C₆-Alkylamino, Aminocarbonyl, C₁-C₆-Alkylcarbonyloxy, C₁-C₆-Alkylcarbonylamino, C₁-C₆-Alkylaminocarbonyl, C₁-C₆-Alkoxycarbonylamino, C₃-C₈-Cycloalkyl, C₃-C₈-Cycloalkylamino, 5- bis 10-gliedriges Heterocyclyl mit bis zu 3 Heteroatomen aus der Reihe S, O und/oder N, 5- bis 7-gliedriges Heterocyclylcarbonyl mit bis zu 3 Heteroatomen aus der Reihe S, O und/oder N, C₆-C₁₀-Alkyl, C₆-C₁₀-Arylamino, 5- bis 10-gliedrigcs Heteroaryl mit bis zu 5 Heteroatomen aus der Reihe S, O und/oder N und 5- bis 10-gliedriges Heteroarylamino mit bis zu 5 Heteroatomen aus der Reihe S, O und/oder N,
worin Alkoxy und Alkylamino substituiert sein können mit 1 bis 3 Substituenten, wobei die Substituenten unabhängig voneinander ausgewählt werden aus der Gruppe, bestehend aus Halogen, Hydroxy und C₁-C₆-Alkoxy,
und
worin Cycloalkyl, Cycloalkylamino, Heterocyclyl, Heterocyclylcarbonyl, Aryl, Arylamino, Heteroaryl und Heteroarylamino substituiert sein können mit 1 bis 3 Substituenten, wobei die Substituenten unabhängig voneinander ausgewählt werden aus der Gruppe, bestehend aus Halogen, Hydroxy, Oxo, Nitro, Cyano, Trifluormethyl, Difluormethyl, Trifluormethoxy, Difluormethoxy, C₁-C₆-Alkyl, C₁-C₆-Alkoxy, Hydroxycarbonyl, C₁-C₆-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₈-Cycloalkylamino und 5- bis 7-gliedriges Heterocyclyl mit bis zu 3 Heteroatomen aus der Reihe S, O und/oder N,
worin Heterocyclyl seinerseits substituiert sein kann mit 1 bis 3 Substituenten, wobei die Substituenten unabhängig voneinander ausgewählt werden aus der Gruppe, bestehend aus C₁-C₄-Alkyl und Oxo,
oder eines ihrer Salze, ihrer Solvate oder der Solvate ihrer Salze.

2. Verbindung nach Anspruch 1, **dadurch gekennzeichnet, dass**
R¹ für -OR⁶ oder -NR⁷R⁸ steht,
R² für C₁-C₄-Alkyl oder C₁-C₅-Alkenyl steht,
wobei Alkyl und Alkenyl substituiert sein können mit 1 bis 2 Substituenten, wobei die Substituenten unabhängig voneinander ausgewählt werden aus der Gruppe, bestehend aus Halogen, C₁-C₄-Alkoxy, C₃-C₆-Cycloalkyl, Phenyl und Phenoxy,
worin Cycloalkyl, Phenyl und Phenoxy substituiert sein können mit 1 bis 3 Substituenten, wobei die Substituenten unabhängig voneinander ausgewählt werden aus der Gruppe, bestehend aus Halogen, Hydroxy, Cyano, Trifluormethyl, Trifluormethoxy, C₁-C₄-Alkyl, C₁-C₄-Alkoxy, Hydroxycarbonyl, C₁-C₄-Alkoxycarbonyl, Amino, C₁-C₆-Alkylamino, Aminocarbonyl, C₁-C₆-Alkylaminocarbonyl und Phenyl,
R³ und R⁴ für Wasserstoff stehen,
R⁵ für Phenyl steht,
wobei Phenyl substituiert sein kann mit 1 bis 3 Substituenten, wobei die Substituenten unabhängig voneinander ausgewählt werden aus der Gruppe, bestehend aus Halogen, Hydroxy, Trifluormethyl, Trifluormethoxy, C₁-C₄-Alkyl und C₁-C₄-Alkoxy,
R⁶ für C₁-C₅-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, C₁-C₄-Alkoxy, Hydroxycarbonyl, Amino, C₁-C₆-Alkylamino, Aminocarbonyl, C₁-C₄-Alkylcarbonyloxy, C₁-C₄-Alkylcarbonylamino, C₁-C₆-Alkylaminocarbonyl, C₁-C₄-Alkoxycarbonylamino, C₃-C₇-Cycloalkyl, C₃-C₇-Cycloalkylamino, 5-bis 7-gliedriges Heterocyclyl mit bis zu 3 Heteroatomen aus der Reihe S, O und/oder N, 5- bis 7-gliedriges Heterocyclylcarbonyl mit bis zu 3 Heteroatomen aus der Reihe S, O und/oder N, C₆-C₁₀-Aryl und 5- bis 10-gliedriges Heteroaryl mit bis zu 5 Heteroatomen aus der Reihe S, O und/oder N,
worin Cycloalkyl, Cycloalkylamino, Heterocyclyl, Heterocyclylcarbonyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Cyano, C₁-C₄-Alkyl, C₁-C₄-Alkoxy, Hydroxycarbonyl, C₁-C₄-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₇-Cycloalkylamino und 5- bis 7-gliedriges Heterocyclyl mit bis zu 3 Heteroatomen aus der Reihe S, O und/oder N,
R⁷ für Wasserstoff steht,
und
R⁸ für C₁-C₅-Alkyl steht,
wobei Alkyl substituiert sein kann mit 1 bis 3 Substituenten, wobei die Substituenten unabhängig voneinander ausgewählt werden aus der Gruppe, bestehend aus Halogen, Cyano, Hydroxy, Trifluormethyl, C₁-C₆-Alkoxy, Hydroxycarbonyl, Amino, C₁-C₆-Alkylamino, Aminocarbonyl, C₁-C₄-Alkylcarbonyloxy, C₁-C₄-Alkylcarbonylamino, C₁-C₆-Alkylaminocarbonyl, C₁-C₄-Alkoxycarbonylamino, C₃-C₇-Cycloalkyl, C₃-C₇-Cycloalkylamino, 5- bis 7-gliedriges Heterocyclyl mit bis zu 3 Heteroatomen aus der Reihe S, O und/oder N, 5- bis 7-gliedriges Heterocyclyl-carbonyl mit bis zu 3 Heteroatomen aus der Reihe S, O und/oder N, C₆-C₁₀-Aryl und 5- bis 10-gliedriges Heteroaryl mit bis zu 5 Heteroatomen aus der Reihe S, O und/oder N,
worin Alkoxy und Alkylamino substituiert sein können mit 1 bis 3 Substituenten, wobei die Substituenten unabhängig voneinander ausgewählt werden aus der Gruppe, bestehend aus Halogen, Hydroxy und C₁-C₄-Alkoxy,
und
worin Cycloalkyl, Cycloalkylamino, Heterocyclyl, Heterocyclylcarbonyl, Aryl und Heteroaryl substituiert sein können mit 1 bis 3 Substituenten, wobei die Substituenten unabhängig voneinander ausgewählt werden aus der Gruppe, bestehend aus Halogen, Hydroxy, Oxo, Cyano, Trifluormethyl, C₁-C₄-Alkyl, C₁-C₄-Alkoxy, Hydroxycarbonyl, C₁-C₄-Alkoxycarbonyl, Amino, C₁-C₆-Alkylamino, Aminocarbonyl und C₁-C₆-Alkylaminocarbonyl,
und
worin Alkylcarbonyloxy substituiert sein kann mit 1 bis 3 Substituenten, wobei die Substituenten unabhängig voneinander ausgewählt werden aus der Gruppe, bestehend aus Hydroxycarbonyl, Amino, C₁-C₆-Alkylamino, C₃-C₇-Cycloalkylamino und 5- bis 7-gliedriges Heterocyclyl mit bis zu 3 Heteroatomen aus der Reihe S, O und/oder N,
worin Heterocyclyl seinerseits substituiert sein kann mit 1 bis 3 Substituenten, wobei die Substituenten unabhängig voneinander ausgewählt werden aus der Gruppe, bestehend aus C₁-C₄-Alkyl und Oxo.

3. Verbindung nach Anspruch 1 oder 2, **dadurch gekennzeichnet, dass**
R¹ für -OR⁶ oder -NR⁷R⁸ steht,
R² für Methyl, Ethyl, n-Bntyl, Prop-2-en-1-yl oder 3-Methyl-but-2-en-1-yl steht,
wobei Methyl, Ethyl, n-Butyl und Propyl-2-en-1-yl substituiert sein können mit 1 bis 2 Substituenten, wobei die Substituenten unabhängig voneinander ausgewählt werden aus der Gruppe, bestehend aus Chlor, Methoxy, Cyclopropyl, Phenyl und Phenoxy,
worin Phenyl substituiert sein kann mit einem Substituenten Trifluormethyl,
R³ und R⁴ für Wasserstoff stehen,
R⁵ für Phenyl steht,
wobei Phenyl substituiert sein kann mit 1 bis 3 Substituenten, wobei die Substituenten unabhängig voneinander ausgewählt werden aus der Gruppe, bestehend aus Fluor, Chlor, Trifluormethoxy und Methyl,
R⁶ für C₁-C₃-Alkyl steht,
wobei Alkyl substituiert sein kann mit 1 bis 2 Substituenten, wobei die Substituenten unabhängig voneinander ausgewählt werden aus der Gruppe, bestehend aus Halogen, Cyano, Hydroxy und Methylcarbonyloxy,
worin Methylcarbonyloxy substituiert sein kann mit 1 bis 3 Substituenten, wobei die Substituenten unabhängig voneinander ausgewählt werden aus der Gruppe, bestehend aus Isobutylamino, Dimethylamino, Diethylamino, Cyclopropylamino, Pyrrolidinyl und Morpholinyl,
R⁷ für Wasserstoff steht,
und
R⁸ für C₁-C₃-Alkyl steht,
wobei Alkyl substituiert sein kann mit 1 bis 2 Substituenten, wobei die Substituenten unabhängig voneinander ausgewählt werden aus der Gruppe, bestehend aus Halogen, Cyano, Hydroxy, Trifluormethyl, Ethoxy, Isobutylamino, Dimethylamino, Diethylamino, Methylethylamino, Aminocarbonyl, Methylcarbonyloxy, Propylcarbonyloxy, Dimethylaminocarbonyl, Diethylaminocarbonyl, Ethoxycarbonylamino, Cyclopropylamino, Pyrrolidinyl, Piperidinyl, Morpholinyl, Phenyl, Thienyl, Pyrazolyl, Imidazolyl, Triazolyl, Pyridyl und Benzimidazolyl,
worin Ethoxy und Methylethylamino substituiert sein können mit einem Substituenten, wobei der Substituent ausgewählt wird aus der Gruppe, bestehend aus Hydroxy und Methoxy,
und
worin Phenyl, Pyrazolyl, Imidazolyl, Pyridyl und Benzimidazolyl substituiert sein können mit 1 bis 3 Substituenten, wobei die Substituenten unabhängig voneinander ausgewählt werden aus der Gruppe, bestehend aus Methyl und Methoxy,
und
worin Methylcarbonyloxy und Propylcarbonyloxy substituiert sein können mit 1 bis 3 Substituenten, wobei die Substituenten unabhängig voneinander ausgewählt werden aus der Gruppe, bestehend aus Hydroxycarbonyl, Amino, Isobutylamino, Dimethylamino, Diethylamino, Cyclopropylamino, Pyrrolidinyl und Morpholinyl.

4. Verfahren zur Herstellung einer Verbindung der Formel (I) nach Anspruch 1, **dadurch gekennzeichnet, dass**
nach Verfahren [A]
eine Verbindung der Formel 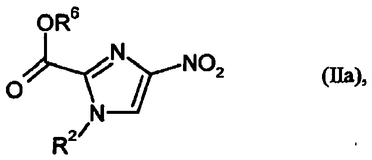 in welcher
R⁶ die in Anspruch 1 angegebene Bedeutung hat,
und
R² die in Anspruch 1 angegebene Bedeutung hat,
in der ersten Stufe mit einem Reduktionsmittel,
in der zweiten Stufe gegebenenfalls mit einer Verbindung der Formel 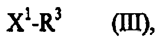 in welcher
R³ die in Anspruch 1 angegebene Bedeutung hat, und
X¹ für Halogen, bevorzugt Brom oder Chlor, steht
und in der dritten Stufe in Gegenwart eines Kohlensäurederivates mit einer Verbindung der Formel 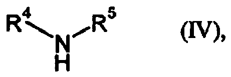 in welcher
R⁴ und R⁵ die in Anspruch 1 angegebene Bedeutung haben, zu einer Verbindung der Formel 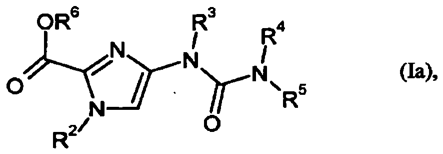 in welcher
R⁶ die gleiche Bedeutung wie in Formel (IIa) hat, und
R², R³, R⁴ und R⁵ die in Anspruch 1 angegebene Bedeutung haben, umgesetzt wird,
oder
nach Verfahren [B]
eine Verbindung der Formel (Ia),
in welcher
R⁸ für Methyl oder Ethyl steht,
in Gegenwart einer Base zu einer Verbindung der Formel 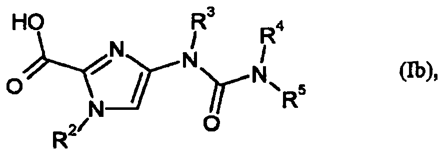 in welcher
R², R³, R⁴ und R⁵ die in Anspruch 1 angegebene Bedeutung haben, umgesetzt wird,
oder
nach Verfahren [C]
eine Verbindung der Formel (Ib) mit einer Verbindung der Formel 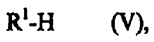 in welcher
R¹ die in Anspruch 1 angegebene Bedeutung hat,
in Gegenwart eines Dehydratisierungsreagenzes zu einer Verbindung der Formel (I) umgesetzt wird,
oder
nach Verfahren [D]
eine Verbindung der Formel 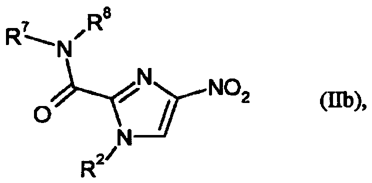 in welcher
R², R⁷ und R⁸ die in Anspruch 1 angegebene Bedeutung haben,
in der ersten Stufe mit einem Reduktionsmittel,
in der zweiten Stufe gegebenenfalls mit einer Verbindung der Formel (III)
und in der dritten Stufe in Gegenwart eines Kohlensäurederivates mit einer Verbindung der Formel (IV)
zu einer Verbindung der Formel 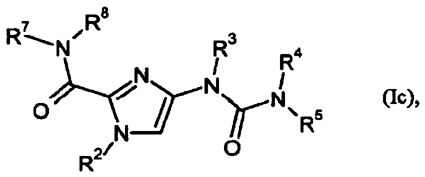 in welcher
R², R³, R⁴, R⁵, R⁷ und R⁸ die in Anspruch 1 angegebene Bedeutung haben, umgesetzt wird,
oder
nach Verfahren [E]
eine Verbindung der Formel (IIa) oder (IIb)
in der ersten Stufe mit einem Reduktionsmittel,
in der zweiten Stufe gegebenenfalls mit einer Verbindung der Formel (III) und in der dritten Stufe mit einer Verbindung der Formel 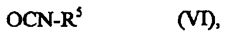 in welcher
R⁵ die in Anspruch 1 angegebene Bedeutung hat, zu einer Verbindung der Formel 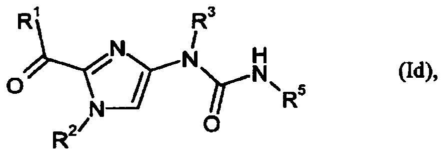 in welcher
R¹, R², R³ und R⁵ die in Anspruch 1 angegebene Bedeutung haben,
umgesetzt wird.

5. Verbindung nach einem der Ansprüche 1 bis 3 zur Behandlung und/oder Prophylaxe von Krankheiten.

6. Arzneimittel enthaltend eine Verbindung nach einem der Ansprüche 1 bis 3 in Kombination mit mindestens einem inerten, nichttoxischen, pharmazeutisch geeigneten Hilfsstoff.

7. Verwendung einer Verbindung nach einem der Ansprüche 1 bis 3 zur Herstellung eines Arzneimittels zur Behandlung und/oder Prophylaxe von Virusinfektionen.

8. Verwendung nach Anspruch 7, **dadurch gekennzeichnet, dass** die Virusinfektion eine Infektion mit dem humanen Cytomegalovirus (HCMV) oder einem anderen Vertreter der Gruppe der Herpes viridae ist.

9. Arzneimittel nach Anspruch 6 zur Behandlung und/oder Prophylaxe von Virusinfektionen.

10. Verbindung nach einem der Ansprüche 1 bis 3, Arzneimittel nach Anspruch 6 oder ein nach Anspruch 7 oder 8 erhaltenes Arzneimittel zur Verwendung in einem Verfahren zur Bekämpfung von Virusinfektionen in Menschen und Tieren.

## Claims

1. Compound of formula 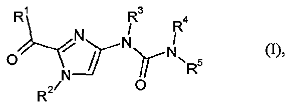 in which
R¹ represents -OR⁶ or -NR⁷R⁸,
R² represents C₁-C₆-alkyl or C₁-C₆-alkenyl,
whereby alkyl and alkenyl may be substituted with 1 to 3 substituents, whereby the substituents are selected independently of one another from the goup consisting of halogen, C₁-C₆-alkoxy, C₃-C₈-cycloalkyl, 5- to 10-membered heterocyclyl, having up to 3 heteroatoms from the series S, O and/or N, C₆-C₁₀-aryl, phenoxy and 5- to 10-membered heteroaryl, having up to 5 heteroatoms from the series S, O and/or N,
wherein cycloalkyl, heterocyclyl, aryl, phenoxy and heteroaryl may be substituted with 1 to 3 substituents, whereby the substituents are selected independently of one another from the group consisting of halogen, hydroxy, oxo, nitro, cyano, trifluoromethyl, difluoromethyl, trifluoromethoxy, difluoromethoxy, C₁-C₆-alkyl, C₁-C₆-alkoxy, hydroxycarbonyl, C₁-C₆-alkoxycarbonyl, amino, C₁-C₆-alkylamino, aminocarbonyl, C₁-C₆-alkylaminocarbonyl and phenyl,
R³ and R⁴ independently of one another represent hydrogen or C₁-C₆-alkyl,
R⁵ represents phenyl,
whereby phenyl may be substituted with 1 to 3 substituents, whereby the substituents are selected independently of one another from the group consisting of halogen, hydroxy, trifluoromethyl, difluoromethyl, trifluoromethoxy, difluoromethoxy, C₁-C₆-alkyl and C₁-C₆-alkoxy,
R⁶ represents C₁-C₆-alkyl,
whereby alkyl may be substituted with 1 to 3 substituents, whereby the substituents are selected independently of one another from the group consisting of halogen, cyano, hydroxy, C₁-C₆-alkoxy, hydroxycarbonyl, C₁-C₆-alkoxycarbonyl, amino, C₁C₆-alkylamino, aminocarbonyl, C₁-C₆-alkylcarbonyloxy, C₁-C₆-alkylcarbonylamino, C₁-C₆-alkylaminocarbonyl, C₁-C₆-alkoxycarbonylamino, C₃-C₈-cycloalkyl, C₃-C₈-cycloalkylamino, 5- to 10-membered heterocyclyl, having up to 3 heteroatoms from the series S, O and/or N, 5- to 7-membered heterocyclylcarbonyl, having up to 3 heteroatoms from the series S, O and/or N, C₆-C₁₀-aryl and 5- to 10-membered heteroaryl, having up to 5 heteroatoms from the series S, O and/or N,
wherein cycloalkyl, cycloalkylamino, heterocyclyl, heterocyclylcarbonyl, aryl and heteroaryl may be substituted with 1 to 3 substituents, whereby the substituents are selected independently of one another from the group consisting of halogen, hydroxy, nitro, cyano, C₁-C₆-alkyl, C₁-C₆-alkoxy, hydroxycarbonyl, C₁-C₆-alkoxycarbonyl, amino, C₁-C₆-alkylamino, aminocarbonyl and C₁-C₆-alkylaminocarbonyl,
and
wherein alkylcarbonyloxy may be substituted with 1 to 3 substituents, whereby the substituents are selected independently of one another from the group consisting of hydroxycarbonyl, amino, C₁-C₆-alkylamino, C₃-C₈-cycloalkylamino and 5- to 7-membered heterocyclyl, having up to 3 heteroatoms from the series S, O and/or N
wherein heterocyclyl in turn may be substituted with 1 to 3 substituents, whereby the substituents are selected independently of one another from the group consisting of C₁-C₄-alkyl and oxo,
R⁷ represents hydrogen or C₁-C₆-alkyl,
and
R⁸ represents C₁-C₆-alkyl,
whereby alkyl may be substituted with 1 to 3 substituents, whereby the substituents are selected independently of one another from the group consisting of halogen, cyano, hydroxy, trifluoromethyl, C₁-C₆-alkoxy, hydroxycarbonyl, C₁-C₆-alkoxycarbonyl, amino, C₁-C₆-alkylamino, aminocarbonyl, C₁-C₆-alkylcarbonyloxy, C₁-C₆-alkylcarbonylamino, C₁-C₆-alkylaminocarbonyl, C₁-C₆-alkoxycarbonylamino, C₃-C₈-cycloalkyl, C₃-C₈-cycloalkylamino, 5- to 10-membered heterocyclyl, having up to 3 heteroatoms from the series S, O and/or N, 5- to 7 membered heterocyclylcarbonyl, having up to 3 heteroatoms from the series S, O and/or N, C₆-C₁₀-aryl, C₆-C₁₀-arylamino, 5- to 10-membered heteroaryl, having up to 5 heteroatoms from the series S, O and/or N, and 5- to 10-membered heteroarylamino, having up to 5 heteroatoms from the series S, O and/or N,
wherein alkoxy and alkylamino may be substituted with 1 to 3 substituents, whereby the substituents are selected independently of one another from the group consisting of halogen, hydroxy and C₁-C₆-alkoxy,
and
wherein cycloalkyl, cycloalkylamino, heterocyclyl, heterocyclylcarbonyl, army, arylamino, heteroaryl and heteroarylamino may be substituted with 1 to 3 substituents, whereby the substituents are selected independently of one another from the group consisting of halogen, hydroxy, oxo, nitro, cyano, trifluoromethyl, difluoromethyl, trifluoromethoxy, difluoromethoxy, C₁-C₆-alkyl, C₁-C₆-alkoxy, hydroxycarbonyl, C₁-C₆-alkoxycarbonyl, amino, C₁-C₆-alkylamino, aminocarbonyl and C₁-C₆-alkylaminocarbonyl,
and
wherein alkylcarbonyloxy may be substituted with 1 to 3 substituents, whereby the substituents are selected independently of one another from the group consisting of hydroxycarbonyl, amino, C₁-C₆-alkylamino C₃-C₈-cycloalkylamino and 5- to 7-membered heterocyclyl, having up to 3 heteroatoms from the series S, O and/or N,
wherein heterocyclyl in turn may be substituted with 1 to 3 substituents, whereby the substituents are selected independently of one another from the group consisting of C₁-C₄-alkyl and oxo,
or one of the salts thereof, the solvates thereof or the solvates of the salts thereof.

2. Compound according to Claim 1, **characterized in that**
R¹ represents -OR⁶ or -NR⁷R⁸,
R² represents C₁-C₄-alkyl or C₁-C₅-alkenyl,
whereby alkyl and alkenyl may be substituted with 1 to 2 substituents, whereby the substituents are selected independently of one another from the group consisting of halogen, C₁-C₄-alkoxy, C₃-C₆-cycloalkyl, phenyl and phenoxy,
wherein cycloalkyl, phenyl and phenoxy may be substituted with 1 to 3 substituents, whereby the substituents are selected independently of one another from the group consisting of halogen, hydroxy, cyano, trifluoromethyl, trifluoromethoxy, C₁-C₄-alkyl, C₁-C₄-alkoxy, hydroxycarbonyl, C₁-C₄-alkoxycarbonyl, amino, C₁-C₆-alkylamino, aminocarbonyl, C₁-C₆-alkylaminocarbonyl and phenyl,
R³ and R⁴ represent hydrogen,
R⁵ represents phenyl,
whereby phenyl may be substituted with 1 to 3 substituents, whereby the substituents are selected independently of one another from the group consisting of halogen, hydroxy, trifluoromethyl, trifluoromethoxy, C₁-C₄-alkyl and C₁-C₄-alkoxy,
R⁶ represents C₁-C₅-alkyl,
whereby alkyl may be substituted with 1 to 3 substituents, whereby the substituents are selected independently of one another from the group consisting of halogen, cyano, hydroxy, C₁-C₄-alkoxy, hydroxycarbonyl, amino, C₁-C₆-alkylamino, aminocarbonyl, C₁-C₄-alkylcarbonyloxy, C₁-C₄-alkylcarbonylamino, C₁-C₆-alkylaminocarbonyl, C₁-C₄-alkoxycarbonylamino, C₃-C₇-cycloalkyl, C₃-C₇-cycloalkylamino, 5- to 7-membered heterocyclyl, having up to 3 heteroatoms from the series S, O and/or N, 5- to 7-membered heterocyclylcarbonyl, having up to 3 heteroatoms from the series S, O and/or N, C₆-C₁₀-aryl and 5- to 10-membered heteroaryl, having up to 5 heteroatoms from the series S, O and/or N,
wherein cycloalkyl, cycloalkylamino, heterocyclyl, heterocyclylcarbonyl, aryl and heteroaryl may be substituted with 1 to 3 substituents, whereby the substituents are selected independently of one another from the group consisting of halogen, hydroxy, cyano, C₁-C₄-alkyl, C₁-C₄-alkoxy, hydroxycarbonyl, C₁-C₄-alkoxycarbonyl, amino, C₁-C₆-alkylamino, aminocarbonyl and C₁-C₆-alkylaminocarbonyl,
and
wherein alkylcarbonyloxy may be substituted with 1 to 3 substituents, whereby the substituents are selected independently of one another from the group consisting of hydroxycarbonyl, amino, C₁-C₆-alkylamino, C₃-C₇-cycloalkylamino and 5- to 7-membered heterocyclyl, having up to 3 heteroatoms from the series S, O and/or N,
I
R⁷ represents hydrogen,
and
R⁸ represents C₁-C₅-alkyl,
whereby alkyl may be substituted with 1 to 3 substituents, whereby the substituents are selected independently of one another from the group consisting of halogen, cyano, hydroxy, trifluoromethyl, C₁-C₆-alkoxy, hydroxycarbonyl, amino, C₁-C₆-alkylamino, aminocarbonyl, C₁-C₄-alkylcarbonlyoxy, C₁-C₄-alkylcarbonylamino, C₁-C₆-alkylaminocarbonyl, C₁-C₄-alkoxycarbonylamino, C₃-C₇-cycloalkyl, C₃-C₇-cycloalkylamino, 5- to 7-membered heterocyclyl, having up to 3 heteroatoms from the series S, O and/or N, 5- to 7-membered heterocyclylcarbonyl, having up to 3 heteroatoms from the series S, O and/or N, C₆-C₁₀-aryl and 5- to 10-membered heteroaryl, having up to 5 heteroatoms from the series S, O and/or N,
wherein alkoxy and alkylamino may be substituted with 1 to 3 substituents, whereby the substituents are selected independently of one another from group consisting of halogen, hydroxy and C₁-C₄-alkoxy,
and
wherein cycloalkyl, cycloalkylamino, heterocyclyl, heterocyclylcarbonyl, aryl and heteroaryl may be substituted with 1 to 3 substituents, whereby the substituents are selected independently of one another from group consisting of halogen, hydroxy, oxo, cyano, trifluoromethyl, C₁-C₄-alkyl, C₁-C₄-alkoxy, hydroxycarbonyl, C₁-C₄-alkoxycarbonyl, amino, C₁-C₆-alkylamino, aminocarbonyl and C₁-C₆-alkylaminocarbonyl,
and
wherein alkylcarbonyloxy may be substituted with 1 to 3 substituents, whereby the substituents are selected independently of one another from the group consisting of hydroxycarbonyl, amino, C₁-C₆-alkylamino, and 5- to 7-membered heterocyclyl, having up to 3 heteroatoms from the series S, O and/or N,
wherein heterocyclyl in turn may be substituted with 1 to 3 substituents, whereby the substituents are selected independently of another from the group consisting of C₁-C₄-alkyl and oxo.

3. Compound according to Claim 1 or 2, **characterized in that**
R¹ represents -OR⁶ or -NR⁷R⁸,
R² represents methyl, ethyl, n-butyl, prop-2-en-1-yl or 3-methylbut-2-en-1-yl,
whereby methyl, ethyl, n-butyl and prop-2-en-1-yl may be substituted with 1 to 2 substituents, whereby the substituents are selected independently of one another from the group consisting of chlorine, methoxy, cyclopropyl, phenyl and phenoxy,
wherein phenyl may be substituted with a substituent trifluoromethyl,
R³ and R⁴ represent hydrogen,
R⁵ represents phenyl,
whereby phenyl may be substituted with 1 to 3 substituents, whereby the substituents are selected independently of one another from the group consisting of fluorine, chlorine, trifluoromethoxy and methyl,
R⁶ represents C₁-C₃-alkyl,
whereby alkyl may be substituted with 1 to 2 substituents, whereby the substituents are selected independently of one another from the group consisting of halogen, cyano, hydroxy and methylcarbonyloxy,
wherein methylcarbonyloxy may be substituted with 1 to 3 substituents, whereby the substituents are selected independently of one another from the group consisting of isobutylamino, dimethylamino, diethylamino, cyclopropylamino, pyrrolidinyl and morpholinyl,
R⁷ represents hydrogen,
and
R⁸ represents C₁-C₃-alkyl,
whereby alkyl may be substituted with 1 to 2 substituents, whereby the substituents are selected independently of one another from the group consisting of halogen, cyano, hydroxy, trifluoromethyl, ethoxy, isobutylamino, dimethylamino, diethylamino, methylethylamino, aminocarbonyl, methylcarbonyloxy, propylcarbonyloxy, dimethylaminocarbonyl, diethylaminocarbonyl, ethoxycarbonylamino, cyclopropylamino, pyrrolidinyl, piperidinyl, morpholinyl, phenyl, thienyl, pyrazolyl, imidazolyl, triazolyl, pyridyl and benzimidazolyl,
wherein ethoxy and methylethylamino may be substituted with a substituent, whereby the substituent is selected from the group consisting of hydroxy and methoxy,
and
wherein phenyl, pyrazolyl, imidazolyl, pyridyl and benzimidazolyl may be substituted with 1 to 3 substituents, whereby the substituents are selected independently of one another from the group consisting of methyl and methoxy,
and
wherein methylcarbonyloxy and propylcarbonyloxy may be substituted with 1 to 3 substituents, whereby the substituents are selected independently of one another from the group consisting of hydroxycarbonyl, amino, isobutylamino, dimethylamino, diethylamino, cyclopropylamino, pyrrolidinyl and morpholinyl.

4. Process for preparing a compound of formula (I) according to Claim 1, **characterized in that**
according to process [A]
a compound of formula 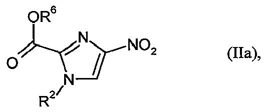 in which
R⁶ has the meaning indicated in Claim 1, and
R² has the meaning indicated in Claim 1,
is reacted in the first stage with a reducing agent,
in the second stage where appropriate with a compound of formula 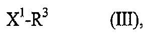 in which
R³ has the meaning indicated in Claim 1, and
X¹ represents halogen, preferably bromine or chlorine,
and in the third stage in the presence of a carbonic acid derivative with a compound of formula 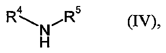 in which
R⁴ and R⁵ have the meaning indicated in Claim 1, to give a compound of formula 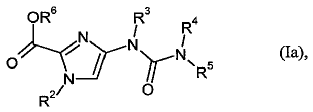 in which
R⁶ has the same meaning as in formula (IIa), and
R², R³, R⁴ and R⁵ have the meaning indicated in Claim 1,
or
according to process [B]
a compound of formula (Ia),
in which
R⁸ represents methyl or ethyl,
is reacted in the presence of a base to give a compound of formula 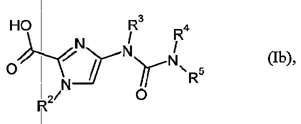 in which
R², R³, R⁴ and R⁵ have the meaning indicated in Claim 1,
or
according to process [C]
a compound of formula (Ib) is reacted with a compound of formula 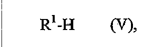 in which
R¹ has the meaning indicated in Claim 1, in the presence of a dehydrating reagent to give a compound of the formula (I),
or
according to process [D]
a compound of formula 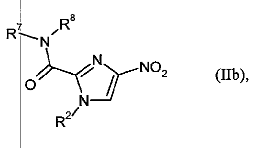 in which
R², R⁷ and R⁸ have the meaning indicated in Claim 1,
is reacted in the first stage with a reducing agent,
in the second stage where appropriate with a compound of formula (III) and in the third stage in the presence of a carbonic acid derivative with a compound of formula (IV)
to give a compound of formula 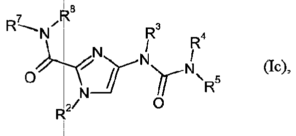 in which
R², R³, R⁴, R⁵, R⁷ and R⁸ have the meaning indicated in Claim 1, or
according to process [E]
a compound of formula (IIa) or (IIb) is reacted in the first stage with a reducing agent,
in the second stage where appropriate with a compound of formula (III) and in the third stage with a compound of formula 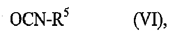 in which R⁵ has the meaning indicated in Claim 1,
to give a compound of formula 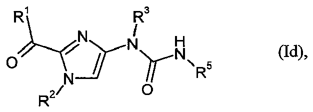 in which
R¹, R², R³ and R⁵ have the meaning indicated in Claim 1.

5. Compound according to any one of Claims 1 to 3 for the treatment and/or prophylaxis of diseases.

6. Medicament comprising a compound according to any one of Claims 1 to 3 in combination with at least one inert, non-toxic, pharmaceutically suitable excipient.

7. Use of a compound according to any one of Claims I to 3 for producing a medicament for the treatment and/or prophylaxis of viral infections.

8. Use according to Claim 7, **characterized in that** the viral infection is an infection with the human cytomegalovirus (HCMV) or another representative of the group of Herpes viridae.

9. Medicament according to Claim 6 for the treatment and/or prophylaxis of viral infections.

10. Compound according to any one of Claims 1 to 3, medicament according to Claim 6 and/or a medicament obtained according to Claim 7 or 8 for use in a method for controlling viral infections in humans and animals.

## Revendications

1. Composé de formule 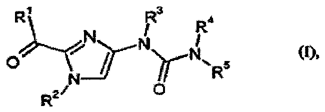 dans laquelle
R¹ désigne -OR⁶ ou -NR⁷R⁸,
R² désigne un groupe alkyle en C₁ à C₆ ou alcényle en C₁ à C₆,
les groupes alcyle et alcényle pouvant être substitués par 1 à 3 substituants, les substituants étant choisis indépendamment les uns des autres dans le groupe comprenant un atome d'halogène, un groupe alcoxy en C₁ à C₆, cycloalkyle en C₃ à C₈, hétérocyclyle de 5 à 10 chaînons comportant jusqu'à 3 hétéro-atomes de la série S, O et/ou N, un groupe aryle en C₆ à C₁₀, phénoxy et hétéroaryle de 5 à 10 chaînons comportant jusqu'à 5 hétéroatomes de la série S, O et/ou N,
dans lequel les groupes cycloalkyle, hétérocyclyle, aryle, phénoxy et hétéroaryle peuvent être substitués par 1 à 3 substituants, les substituants pouvant être choisis indépendamment les uns des autres dans le groupe comprenant un atome d'halogène, un groupe hydroxy, oxo, nitro, cyano, trifluorométhyle, difluorométhyle, trifluorométhoxy, difluorométhoxy, alkyle en C₁ à C₆, alcoxy en C₁ à C₆, hydroxycarbonyle, alcoxy en C₁ à C₆, carbonyle, amino, alkylamino en C₁ à C₆, aminocarbonyle, alkylaminocarbonyle en C₁ à C₆ et phényle,
R³ et R⁴ désignent indépendamment l'un de l'autre, un atome d'hydrogène ou un groupe alkyle en C₁ à C₆,
R⁵ désigne un groupe phényle,
le groupe phényle pouvant être substitué par 1 à 3 substituants, les substituants étant choisis indépendamment les uns des autres dans le groupe comprenant un atome d'halogène, un groupe hydroxy, trifluorométhyle, difluorométhyle, trifluorométhoxy, difluorométhoxy, alkyle en C₁ à C₆ et alcoxy en C₁ à C₆,
R⁶ désigne un groupe alkyle en C₁ à C₆,
le groupe alkyle pouvant être substitué par 1 à 3 substituants, les substituants étant choisis indépendamment les uns des autres dans le groupe comprenant un atome d'halogène, un groupe cyano, hydroxy, alcoxy en C₁ à C₆, hydroxycarbonyle, alcoxycarbonyle en C₁ à C₆, amino, alkylamino en C₁ à C₆, aminocarbonyle, alkylcarbonyloxy en C₁ à C₆, alkylcarbonylamino en C₁ à C₆, alkylaminocarbonyle en C₁ à C₆, alcoxycarbonylamino en C₁ en C₆, cycloalkyle en C₃ à C₈, cycloalkylamino en C₃ à C₈, hétérocyclyle de 5 à 10 chaînons comportant jusqu'à 3 hétéroatomes de la série S, O et/ou N, hétérocyclyl-carbonyle de 5 à 7 chaînons comportant jusqu'à 3 hétéroatomes de la série S, O et/ou N, aryle en C₆ à C₁₀ et hétéroaryle de 5 à 10 chaînons comportant jusqu'à 5 hétéroatomes de la série S, O et/ou N,
dans lequel les groupes cycloalkyle, cycloalkylamino, hétérocyclyle, hétérocyclyl-carbonyle, aryle et hétéroaryle peuvent être substitués par 1 à 3 substituants, les substituants pouvant être choisis indépendamment les uns des autres dans le groupe comprenant un atome d'halogène, un groupe hydroxy, nitro, cyano, alkyle en C₁ à C₆, alcoxy en C₁ à C₆, hydroxycarbonyle, alcoxycarbonyle en C₁ à C₆, amino, alkylamino en C₁ à C₆, aminocarbonyle et alkylaminocarbonyle en C₁ à C₆,
et
dans lequel le groupe alkylcarbonyloxy peut être substitué par 1 à 3 substituants, les substituants étant choisis indépendamment les uns des autres dans le groupe comprenant les groupes hydroxycarbonyle, amino, alkylamino en C₁ à C₆, cycloalkylamino en C₃ à C₈ et hétérocyclyle de 5 à 7 chaînons comportant jusqu'à 3 hétéroatomes de la série S, O et/ou N,
dans lequel le groupe hétérocyclyle peut être substitué de son côté par 1 à 3 substituants, les substituants étant choisis indépendamment les uns des autres dans le groupe consistant en un groupe alkyle en C₁ à C₄ et oxo,
R⁷ désigne un atome d'hydrogène ou un groupe alkyle en C₁ à C₆,
et
R⁸ désigne un groupe alkyle en C₁ à C₆,
le groupe alkyle pouvant être substitué par 1 à 3 substituants, les substituants étant choisis indépendamment les uns des autres dans le groupe comprenant un atome d'halogène, un groupe cyano, hydroxy, trifluorométhyle, alcoxy en C₁ à C₆, hydroxycarbonyle, alcoxycarbonyle en C₁ à C₆, amino, alkylamino en C₁ à C₆, aminocarbonyle, alkylcarbonyloxy en C₁ à C₆, alkylcarbonylamino en C₁ à C₆, alkylaminocarbonyle en C₁ à C₆, alcoxycarbonylamino en C₁ en C₆, cycloalkyle en C₃ à C₈, cycloalkylamino en C₃ à C₈, hétérocyclyle de 5 à 10 chaînons comportant jusqu'à 3 hétéroatomes de la série S, O et/ou N, hétérocyclyl-carbonyle de 5 à 7 chaînons comportant jusqu'à 3 hétéroatomes de la série S, O et/ou N, aryle en C₆ à C₁₀, arylamino en C₆ à C₁₀, hétéroaryl de 5 à 10 chaînons comportant jusqu'à 5 hétéroatomes de la série S, O et/ou N et hétéroarylamino de 5 à 10 chaînons comportant jusqu'à 5 hétéroatomes de la série S, O et/ou N,
dans lequel les groupes alcoxy et alkylamino peuvent être substitués par 1 à 3 substituants, les substituants pouvant être choisis indépendamment les uns des autres dans le groupe comprenant un atome d'halogène, un groupe hydroxy et alcoxy en C₁ en C₆,
et
dans lequel les groupes cycloalkyle, cycloalkylamino, hétérocyclyle, hétérocyclyl-carbonyle, aryle, arylamino, hétéroaryle et hétéroarylamino peuvent être substitués par 1 à 3 substituants, les substituants pouvant être choisis indépendamment les uns des autres dans le groupe comprenant un atome d'halogène, un groupe hydroxy, oxo, nitro, cyano, trifluorométhyle, difluorométhyle, trifluorométhoxy, difluorométhoxy, alkyle en C₁ à C₆, alcoxy en C₁ à C₆, hydroxycarbonyle, alcoxycarbonyle en C₁ à C₆, amino, alkylamino en C₁ à C₆, aminocarbonyle et alkylaminocarbonyle en C₁ à C₆,
et
dans lequel le groupe alkylcarbonyloxy peut être substitué par 1 à 3 substituants, les substituants étant choisis indépendamment les uns des autres dans le groupe comprenant les groupes hydrocarbonyle, amino, alkylamino en C₁ à C₆, cycloalkylamino en C₃ à C₈ et hétérocyclyle de 5 à 7 chaînons comportant jusqu'à 3 hétéroatomes de la série S, O et/ou N,
dans lequel le groupe hétérocyclyle peut être substitué de son côté par 1 à 3 substituants, les substituants étant choisis indépendamment les uns des autres dans le groupe consistant en un groupe alkyle en C₁ à C₄ et oxo,
ou l'un de leurs sels, leurs solvates ou les solvates de leurs sels.

2. Composé selon la revendication 1, **caractérisé en ce que**
R¹ désigne -OR⁶ ou -NR⁷R⁸,
R² désigne un groupe alkyle en C₁ à C₄ ou alcényle en C₁ à C₅,
les groupes alkyle et alcényle pouvant être substitués par 1 à 2 substituants, les substituants étant choisis indépendamment les uns des autres dans le groupe comprenant un atome d'halogène, un groupe alcoxy en C₁ à C₄, cycloalkyle en C₃ à C₆, phényle et phénoxy,
dans lequel les groupes cycloalkyle, phényle et phénoxy peuvent être substitués par 1 à 3 substituants, les substituants pouvant être choisis indépendamment les uns des autres dans le groupe comprenant un atome d'halogène, un groupe hydroxy, cyano, trifluorométhyle, trifluorométhoxy, alkyle en C₁ à C₄, alcoxy en C₁ à C₄, hydroxycarbonyle, alcoxycarbonyle en C₁ à C₄, amino, alkylamino en C₁ à C₆, aminocarbonyle, alkylaminocarbonyle en C₁ à C₆ et phényle,
R³ et R⁴ désignent un atome d'hydrogène,
R⁵ désigne un groupe phényle,
le groupe phényle pouvant être substitué par 1 à 3 substituants, les substituants étant choisis indépendamment les uns des autres dans le groupe comprenant un atome d'halogène, un groupe hydroxy, trifluorométhyle, trifluorométhoxy, alkyle en C₁ à C₄ et alcoxy en C₁ à C₄,
R⁶ désigne un groupe alkyle en C₁ à C₅,
le groupe alkyle pouvant être substitué par 1 à 3 substituants, les substituants étant choisis indépendamment les uns des autres dans le groupe comprenant un atome d'halogène, un groupe cyano, hydroxy, alcoxy en C₁ à C₄, hydroxycarbonyle, amino, alkylamino en C₁ à C₆, aminocarbonyle, alkylcarbonyloxy en C₁ à C₄, alkylcarbonylamino en C₁ à C₄, alkylaminocarbonyle en C₁ à C₆, alcoxycarbonylamino en C₁ en C₄, cycloalkyle en C₃ à C₇, cycloalkylamino en C₃ à C₇, hétérocyclyle de 5 à 7 chaînons comportant jusqu'à 3 hétéroatomes de la série S, O et/ou N, hétérocyclyl-carbonyle de 5 à 7 chaînons comportant jusqu'à 3 hétéroatomes de la série S, O et/ou N, aryle en C₆ à C₁₀ et hétéroaryle de 5 à 10 chaînons comportant jusqu'à 5 hétéroatomes de la série S, O et/ou N,
dans lequel les groupes cycloalkyle, cycloalkylamino, hétérocyclyle, hétérocyclyl-carbonyle, aryle et hétéroaryle peuvent être substitués par 1 à 3 substituants, les substituants pouvant être choisis indépendamment les uns des autres dans le groupe comprenant un atome d'halogène, un groupe hydroxy, cyano, alkyle en C₁ à C₄, alcoxy en C₁ à C₄, hydroxycarbonyle, alcoxycarbonyle en C₁ à C₄, amino, alkylamino en C₁ à C₆, aminocarbonyle et alkylaminocarbonyle en C₁ à C₆,
et dans lequel le groupe alkylcarbonyloxy peut être substitué par 1 à 3 substituants, les substituants étant choisis indépendamment les uns des autres dans le groupe comprenant les groupes hydrocarbonyle, amino, alkylamino en C₁ à C₆, cycloalkylamino en C₃ à C₇ et hétérocyclyle de 5 à 7 chaînons comportant jusqu'à 3 hétéroatomes de la série S, O et/ou N,
R⁷ désigne un atome d'hydrogène,
et
R⁸ désigne un groupe alkyle en C₁ à C₅,
le groupe alkyle pouvant être substitué par 1 à 3 substituants, les substituants étant choisis indépendamment les uns des autres dans le groupe comprenant un atome d'halogène, un groupe cyano, hydroxy, trifluorométhyle, alcoxy en C₁ à C₆, hydroxycarbonyle, amino, alkylamino en C₁ à C₆, aminocarbonyle, alkylcarbonyloxy en C₁ à C₄, alkylcarbonylamino en C₁ à C₄, alkylaminocarbonyle en C₁ à C₆, alcoxycarbonylamino en C₁ en C₄, cycloalkyle en C₃ à C₇, cycloalkylamino en C₃ à C₇, hétérocyclyle de 5 à 7 chaînons comportant jusqu'à 3 hétéroatomes de la série S, O et/ou N, hétérocyclyl-carbonyle de 5 à 7 chaînons comportant jusqu'à 3 hétéroatomes de la série S, O et/ou N, aryle en C₆ à C₁₀ et hétéroaryle de 5 à 10 chaînons comportant jusqu'à 5 hétéroatomes de la série S, O et/ou N,
dans lequel les groupes alcoxy et alkylamino peuvent être substitués par 1 à 3 substituants, les substituants pouvant être choisis indépendamment les uns des autres dans le groupe comprenant un atome d'halogène, un groupe hydroxy et alcoxy en C₁ à C₄,
et
dans lequel les groupes cycloalkyle, cycloalkylamino, hétérocyclyle, hétérocyclyl-carbonyle, aryle et hétéroaryle peuvent être substitués par 1 à 3 substituants, les substituants pouvant être choisis indépendamment les uns des autres dans le groupe comprenant un atome d'halogène, un groupe hydroxy, oxo, cyano, trifluorométhyle, alkyle en C₁ à C₄, alcoxy en C₁ à C₄, hydroxycarbonyle, alcoxycarbonyle en C₁ à C₄, amino, alkylamino en C₁ à C₆, aminocarbonyle et alkylaminocarbonyle en C₁ à C₆,
et
dans lequel le groupe alkylcarbonyloxy peut être substitué par 1 à 3 substituants, les substituants étant choisis indépendamment les uns des autres dans le groupe comprenant les groupes hydroxycarbonyle, amino, alkylamino en C₁ à C₆, cycloalkylamino en C₃ à C₇ et hétérocyclyle de 5 à 7 chaînons comportant jusqu'à 3 hétéroatomes de la série S, O et/ou N,
dans lequel le groupe hétérocyclyle peut être substitué de son côté par 1 à 3 substituants, les substituants étant choisis indépendamment les uns des autres dans le groupe consistant en un groupe alkyle en C₁ à C₄ et oxo,

3. Composé selon la revendication 1 ou 2, **caractérisé en ce que**
R¹ désigne -OR⁶ ou -NR⁷R⁸,
R² désigne un groupe méthyle, éthyle, n-butyle, prop-2-én-1-yle ou 3-méthyl-but-2-én-1-yle,
les groupes méthyle, éthyle, n-butyle et propyl-2-én-1-yle pouvant être substitués par 1 à 2 substituants, les substituants étant choisis indépendamment dans le groupe comprenant un atome de chlore, un groupe méthoxy, cyclopropyle, phényle et phénoxy,
dans lequel le groupe phényle peut être substitué par un substituant trifluorométhyle,
R³ et R⁴ désignent un atome d'hydrogène,
R⁵ désigne un groupe phényle,
le groupe phényle pouvant être substitué par 1 à 3 substituants, les substituants étant choisis indépendamment les uns des autres dans le groupe comprenant un atome de fluor, de chlore, un groupe trifluorométhoxy et méthyle,
R⁶ désigne un groupe alkyle en C₁ à C₃,
le groupe alkyle pouvant être substitué par 1 à 2 substituants, les substituants étant choisis indépendamment les uns des autres dans le groupe comprenant un atome d'halogène, un groupe cyano, hydroxy et méthylcarbonyloxy,
dans lequel le groupe méthylcarbonyloxy peut être substitué par 1 à 3 substituants, les substituants pouvant être choisis indépendamment les uns des autres dans le groupe comprenant les groupes isobutylamino, diméthylamino, diéthylamino, cyclopropylamino, pyrrolidinyle et morpholinyle,
R⁷ désigne un atome d'hydrogène,
et
R⁸ désigne un groupe alkyle en C₁ à C₃,
le groupe alkyle pouvant être substitué par 1 à 2 substituants, les substituants étant choisis indépendamment les uns des autres dans le groupe comprenant un atome d'halogène, un groupe cyano, hydroxy, trifluorométhyle, éthoxy, isobutylamino, diméthylamino, diéthylamino, méthyléthylamino, aminocarbonyle, méthylcarbonyloxy, propylcarbonyloxy, diméthylaminocarbonyle, diéthylaminocarbonyle, éthoxycarbonylamino, cyclopropylamino, pyrrolidinyle, pipéridinyle, morpholinyle, phényle, thiényle, pyrazolyle, imidazolyle, triazolyle, pyridyle et benzimidazolyle,
dans lequel les groupes éthoxy et méthyléthylamino peuvent être substitués par un substituant, le substituant étant choisi dans le groupe constitué des groupes hydroxy et méthoxy,
et
dans lequel les groupes phényle, pyrazolyle, imidazolyle, pyridyle et benzimidazolyle pouvant être substitués par 1 à 3 substituants, les substituants étant choisis indépendamment les uns des autres dans le groupe comprenant les groupes méthyle et méthoxy,
et
dans lequel les groupes méthylcarbonyloxy et propylcarbonyloxy peuvent être substitués par 1 à 3 substituants, les substituants étant choisis indépendamment les uns des autres dans le groupe comprenant les groupes hydroxycarbonyle, amino, isobutylamino, diméthylamino, cyclopropylamino, pyrrolidinyle et morpholinyle.

4. Procédé de production d'un composé de formule (I) selon la revendication 1, **caractérisé en ce que**
selon le procédé [A]
on convertit un composé de formule 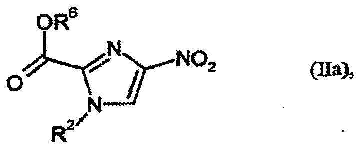 dans lequel
R⁶ a la signification indiquée dans la revendication 1,
et
R² a la signification indiquée dans la revendication 1,
dans la première étape avec un agent de réduction,
dans la deuxième étape éventuellement avec un composé de formule 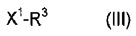 dans laquelle
R³ a la signification indiquée dans la revendication 1, et
X¹ désigne un atome d'halogène, de préférence de brome ou de chlore, et dans la troisième étape, en présence d'un dérivé d'acide carbonique avec un composé de formule 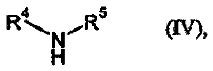 dans laquelle
R⁴ et R⁵ ont la signification indiquée dans la revendication 1 en un composé de formule 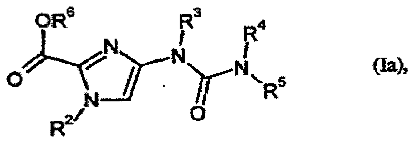 dans laquelle
R⁶ a la même signification que dans la formule (IIa), et
R², R³, R⁴ et R⁵ ont la signification indiquée dans la revendication 1, ou
selon le procédé [B],
on convertit un composé de formule (Ia),
dans laquelle
R⁸ désigne un groupe méthyle ou éthyle,
en présence d'une base en un composé de formule 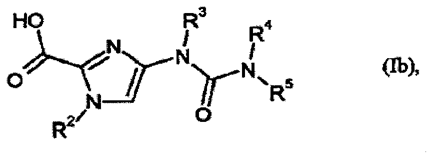 dans laquelle
R², R³, R⁴ et R⁵ ont la signification indiquée dans la revendication 1, ou
selon le procédé [C]
on convertit un composé de formule (Ib) avec un composé de formule 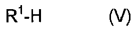 dans laquelle
R¹ a la signification indiquée dans la revendication 1 en présence d'un réactif de déshydratation en un composé de formule (I), ou
selon le procédé [D]
on convertit un composé de formule 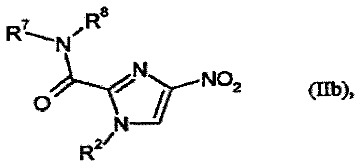 dans lequel
R², R⁷ et R⁸ ont la signification indiquée dans la revendication 1,
à la première étape avec un agent de réduction,
à la deuxième étape éventuellement avec un composé de formule (III) et à la troisième étape en présence d'un dérivé d'acide carbonique avec un composé de formule (IV)
en un composé de formule 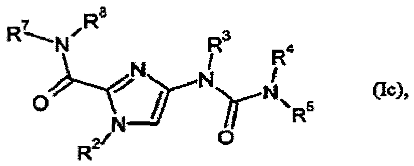 dans laquelle
R², R³, R⁴, R⁵, R⁷ et R⁸ ont la signification indiquée dans la revendication 1,
ou
selon le procédé [E]
on convertit un composé de formule (IIa) ou (IIb) à la première étape avec un agent de réduction,
à la deuxième étape éventuellement avec un composé de formule (III) et à la troisième étape avec un composé de formule 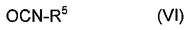 dans laquelle
R⁵ a la signification indiquée à la revendication 1, en un composé de formule 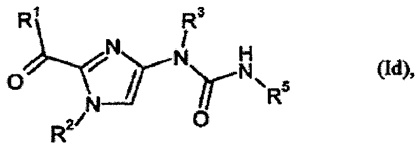 dans laquelle
R¹, R², R³ et R⁵ ont la signification indiquée dans la revendication 1.

5. Composé selon l'une des revendications 1 à 3, pour le traitement et/ou la prophylaxie de maladies.

6. Médicament contenant un composé selon l'une des revendications 1 à 3, en combinaison avec au moins un adjuvant inerte, non toxique, pharmaceutiquement approprié.

7. Utilisation d'un composé selon l'une des revendications 1 à 3, pour la production d'un médicament pour le traitement et/ou la prophylaxie d'infections virales.

8. Utilisation selon la revendication 7, **caractérisée en ce que** l'infection virale est une infection par le cytomégalovirus humain (HCMV) ou un autre représentant du groupe des herpès-virus.

9. Médicament selon la revendication 6, pour le traitement et/ou la prophylaxie d'infections virales.

10. Composé selon l'une des revendications 1 à 3, médicament selon la revendication 6 ou médicament obtenu selon la revendication 7 ou 8, pour l'utilisation dans un procédé de lutte contre des infections virales chez l'être humain et l'animal.
